(19) Europäisches Patentamt
European Patent Office
Office européen des brevets

(11) Veröffentlichungsnummer: **0 304 025 B1**

(12) **EUROPÄISCHE PATENTSCHRIFT**

(45) Veröffentlichungstag der Patentschrift: **22.07.92**

(21) Anmeldenummer: **88113332.6**

(22) Anmeldetag: **17.08.88**

(51) Int. Cl.[5]: **C07C 333/08**, C07C 333/24, C07C 267/00, C07C 331/28, C07C 211/45, A01N 47/30, A01N 47/42, A01N 47/40

(54) **Phenylthioharnstoffe, -isothioharnstoffe und -carbodiimide, ihre Herstellung und Verwendung bei der Kontrolle von Schädlingen.**

(30) Priorität: **20.08.87 CH 3197/87**
**03.03.88 CH 790/88**

(43) Veröffentlichungstag der Anmeldung:
**22.02.89 Patentblatt 89/08**

(45) Bekanntmachung des Hinweises auf die Patenterteilung:
**22.07.92 Patentblatt 92/30**

(84) Benannte Vertragsstaaten:
**BE CH DE FR GB IT LI NL**

(56) Entgegenhaltungen:
**EP-A- 0 282 452**
**EP-A- 0 296 120**
**EP-A- 0 304 402**
**BE-A- 888 179**
**DE-A- 3 801 395**

**JOURNAL OF THE CHEMICAL SOCIETY (C), 1967, Seiten 1780-1782; M. ELLIOTT et al.: "Synthesis of 4-alkyl- and 4-benzyl-2,6-dimethylbromobenzene from 2,6-xylidine"**

(73) Patentinhaber: **CIBA-GEIGY AG**
**Klybeckstrasse 141**
**CH-4002 Basel(CH)**

(72) Erfinder: **Ehrenfreund, Josef, Dr.**
**Amselstrasse 11**
**CH-4123 Allschwil(CH)**

(74) Vertreter: **Zumstein, Fritz, Dr. et al**
**Bräuhausstrasse 4**
**W-8000 München 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die vorliegende Erfindung betrifft neue substituierte Phenylthioharnstoffe, -isothioharnstoffe und -carbodiimide, ihre Salze mit organischen und anorganischen Säuren, Verfahren und Zwischenprodukte zu ihrer Herstellung, Schädlingsbekämpfungsmittel, die diese Verbindungen enthalten, sowie ihre Verwendung bei der Kontrolle von Schädlingen.

Die erfindungsgemässen Verbindungen entsprechen der Formel I

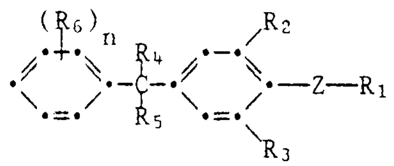

(I),

worin

$R_1$ für $C_1$-$C_8$-Alkyl, ein- oder mehrfach durch Halogen und/oder $C_1$-$C_6$-Alkoxy substituiertes $C_1$-$C_8$-Alkyl, $C_3$-$C_8$-Cycloalkyl, ein- oder mehrfach durch $C_1$-$C_3$-Alkyl substituiertes $C_3$-$C_8$-Cycloalkyl oder $C_3$-$C_8$-Cycloalkyl-$C_1$-$C_4$-alkyl;

$R_2$ und $R_3$ je für $C_1$-$C_6$-Alkyl, $C_5$-$C_6$-Cycloalkyl oder $C_5$-$C_6$-Cycloalkenyl;

$R_4$ und $R_5$ je für Wasserstoff oder $C_1$-$C_4$-Alkyl;

$R_6$ je für Halogen, $C_1$-$C_4$-Alkyl, $C_1$-$C_4$-Alkoxy, $C_1$-$C_4$-Halogenalkyl, $C_1$-$C_4$-Halogenalkoxy oder für eine

$$-(-CH{=}CH-)_2-,\quad -(-CH_2-)_3- \text{ oder}$$

$$-(-CH_2-)_4-$$

Brücke in 2,3- oder 3,4-Stellung;

n für 0, 1 oder 2;

Z für -NH-CS-NH-, -N = C($SR_7$)-NH- oder -N = C = N- und

$R_7$ für $C_1$-$C_{10}$-Alkyl oder $C_3$-$C_5$-Alkenyl

stehen.

Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden Alkyle können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl usw. und ihre Isomeren genannt.

Die als Substituenten in Betracht kommenden Alkenyle können geradkettig oder verzweigt sein und eine oder mehrere Doppelbindungen aufweisen. Beispiele solcher Alkenyle sind u.a. Allyl, 1-Propenyl, Isopropenyl, Allenyl, Butenyle oder Pentenyle.

Die als Substituenten in Betracht kommenden ein- oder mehrfach durch Halogen und/oder $C_1$-$C_6$-Alkoxy substituierten $C_1$-$C_8$-Alkoxy substituierten $C_1$-$C_8$-Alkyle können geradkettig oder verzweigt und nur teilweise oder auch perhalogeniert und/oder ein- bis fünffach durch $C_1$-$C_6$-Alkoxy substituiert sein, wobei für die Halogene und die Alkyle die oben gegebenen Definitionen gelten. Geeignete Beispiele solcher Substituenten sind u.a. das ein- bis dreifach durch Fluor, Chlor und/oder Brom substituierte Methyl wie z.B. $CHF_2$ oder $CF_3$; das ein- bis fünffach durch Fluor, Chlor und/oder Brom substituierte Aethyl wie z.B. $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder CClFCHClF; das ein- bis siebenfach durch Fluor, Chlor und/oder Brom substituierte Propyl oder Isopropyl wie z.B. $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren wie z.B. $CF(CF_3)CHFCF_3$ oder $CH_2$-$(CF_2)_2CF_3$; Methoxymethyl, Methoxyäthyl, Aethoxyäthyl, Methoxypropyl, Aethoxypropyl, Propoxypropyl, Methoxybutyl, Aethoxybutyl, Propoxybutyl oder Butoxybutyl, 1,2-Dimethoxyäthyl, 1,3-Dimethoxypropyl oder 2,4-Dimethoxybutyl. Diese Ausführungen gelten sinngemäss auch für die gegebenenfalls ein- oder mehrfach halogenierten $C_1$-$C_4$-Alkyl- und -alkoxyreste sowie die unsubstituierten $C_1$-$C_4$-Alkoxyreste.

Bei den als Substituenten in Betracht kommenden Cycloalkylen und Cycloalkenylen handelt es sich beispielsweise um Cyclopropyl, Cyclobutyl, Cyclopentyl, Cyclopentenyl, Cyclohexyl oder Cyclohexenyl. Die Cycloalkyle können ein- oder mehrfach durch einen $C_1$-$C_4$-Alkylrest substituiert und/oder über eine $C_1$-$C_4$-Alkylenbrücke mit dem Rest des Moleküls verbunden sein.

Die Verbindungen der Formel I, in denen Z für -N=C($SR_7$)-NH- steht, können auch in Form von Säureadditionssalzen vorliegen. Zur Bildung solcher Salze eignen sich sowohl organische als auch anorganische Säuren. Beispiele solcher Säuren sind u.a. Chlorwasserstoffsäure, Bromwasserstoffsäure, Jodwasserstoffsäure, Salpetersäure, verschiedene Phosphorsäuren, Schwefelsäure, Essigsäure, Propionsäure, Buttersäure, Valeriansäure, Oxalsäure, Malonsäure, Bernsteinsäure, Aepfelsäure, Maleinsäure, Fumarsäure, Milchsäure, Weinsäure, Zitronensäure, Benzoesäure, Phthalsäure, Zimtsäure, Phenylsulfonsäuren oder Salicylsäure.

Verbindungen der Formel I, in denen Z für -N=C($SR_7$)-NH- steht, können in den tautomeren Formen

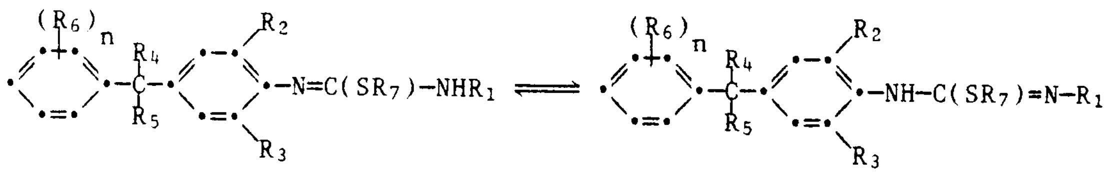

vorliegen. Die Erfindung umfasst sowohl die einzelnen Tautomeren, als auch Tautomerengemische.

Der Benzylrest kann, je nach der Grösse von n, mehrfach durch $R_6$ substituiert sein. Ist n grösser als 1, so können die verschiedenen $R_6$ gleiche oder verschiedene Bedeutung haben.

Unter den Verbindungen der Formel I stehen diejenigen im Vordergrund, in denen $R_1$ für $C_1$-$C_6$-Alkyl, ein- oder mehrfach durch Halogen und/oder $C_1$-$C_3$-Alkoxy substituiertes $C_1$-$C_6$-Alkyl oder $C_5$-$C_6$-Cycloalkyl; $R_2$ und $R_3$ je für $C_1$-$C_5$-Alkyl oder $C_5$-$C_6$-Cycloalkyl; $R_4$ und $R_5$ je für Wasserstoff oder $C_1$-$C_3$-Alkyl, $R_6$ für Halogen, Methyl, Methoxy, Trifluormethyl oder Trifluormethoxy; n für 0 oder 1; Z für -NH-CS-NH-, -N=C-($SR_7$)-NH-oder -N=C=N-; und $R_7$ für $C_1$-$C_6$-Alkyl oder $C_3$-$C_4$-Alkenyl stehen.

Dabei sind diejenigen Verbindungen der Formel I bevorzugt, in denen $R_1$ für $C_1$-$C_5$-Alkyl oder Cyclopentyl; $R_2$ und $R_3$ je für $C_3$-$C_5$-Alkyl oder $C_5$-$C_6$-Cycloalkyl; $R_4$ und $R_5$ je für Wasserstoff oder Methyl; $R_6$ für Fluor, Chlor oder Methyl; n für 0 oder 1; Z für -NH-CS-NH-, -N=C($SR_7$)-NH- oder -N=C=N-; und $R_7$ für $C_1$-$C_3$-Alkyl oder Allyl stehen.

Von besonderer Bedeutung sind diejenigen Verbindungen der Formel I, in denen

a) $R_1$ für $C_3$-$C_5$-Alkyl oder Cyclopentyl; $R_2$ und $R_3$ je für $C_3$-$C_5$-Alkyl; $R_4$ und $R_5$ je für Wasserstoff oder Methyl; n für 0; und Z für -NH-CS-NH-stehen; oder

b) $R_1$ für $C_3$-$C_5$-Alkyl oder Cyclopentyl, $R_2$ und $R_3$ je für $C_3$-$C_5$-Alkyl; $R_4$ und $R_5$ je für Wasserstoff oder Methyl; n für 0; Z für -N=C($SR_7$)-NH-; und $R_7$ für $C_1$-$C_2$-Alkyl stehen; oder

c) $R_1$ für $C_3$-$C_5$-Alkyl oder Cyclopentyl, $R_2$ und $R_3$ je für $C_3$-$C_5$-Alkyl; $R_4$ und $R_5$ je für Wasserstoff oder Methyl; n für 0; und Z für -N=C=N-stehen.

Die erfindungsgemässen Verbindungen der Formel I können nach im Prinzip bekannten Verfahren hergestellt werden, indem man z.B.

A) ein Isothiocyanat der Formel II

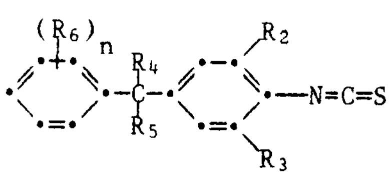

(II)

mit einem Amin der Formel III

$H_2N—R_1$ (III)

zum Thioharnstoff umsetzt und gegebenenfalls

B) den erhaltenen Thioharnstoff mit einer Verbindung der Formel IV

$X—R_7$ (IV)

zum Isothioharnstoff umsetzt oder

C) den erhaltenen Thioharnstoff durch Abspalten von Schwefelwasserstoff in das Carbodiimid überführt. $R_1$, $R_2$, $R_3$, $R_4$, $R_5$, $R_6$, $R_7$ und n haben dabei die angegebene Bedeutung und X steht für eine geeignete Abgangsgruppe wie z.B. ein Halogenatom, insbesondere Chlor, Brom oder Jod, oder einen gegebenenfalls halogenierten oder alkylierten Sulfonsäureester, wie z.B. ein Tosylat, Brosylat oder Mono- oder Dialkylsulfat (Mesylat, Dimethylsulfat).

Das Verfahren A wird üblicherweise unter normalem Druck, und in Gegenwart eines organischen Lösungs- oder Verdünnungsmittels durchgeführt. Die Temperatur beträgt dabei zwischen 0 und +150°C, vorzugsweise +10 bis 70°C. Als Lösungs- oder Verdünnungsmittel eignen sich z.B. Aether und ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon, Methylisobutylketon oder Cyclohexanon.

Das Verfahren B wird zweckmässigerweise in einem inerten organischen Lösungsmittel und unter leicht erhöhtem oder normalem Druck durchgeführt. Die Temperatur beträgt dabei zwischen +10 und 250°C, vorzugsweise Siedetemperatur des verwendeten Lösungsmittels oder +50 bis 150°C. Geeignete Lösungs- oder Verdünnungsmittel sind z.B. Aether oder ätherartige Verbindungen, wie Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon, Alkohole oder Dimethylformamid. Die Reaktion wird entweder in Gegenwart einer Base durchgeführt oder aber das gebildete Salz anschiessend einer basischen Nachbehandlung unterworfen. (vgl. J.B. Hendricksen et al., "Organic Chemistry", Mc Graw Hill Book Co., 1970, p. 378-382).

Das Verfahren C wird zweckmässigerweise in einem aprotischen organischen Lösungs- oder Verdünnungsmittel unter normalem Druck durchgeführt. Die Temperatur beträgt dabei zwischen 0 und +150°C, vorzugsweise +10 bis 50°C. Geeignete Lösungs- oder Verdünnungsmittel sind z.B. Aether oder ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon oder Cyclohexanon. Die Abspaltung von Schwefelwasserstoff erfolgt nach in der Literatur beschriebenen Arbeitsweisen (T. Shibanuma, Chemistry Letters 1977, p. 575-76; S. Kim, Tetrahedron Letters 1985, p. 1661-64; W. Weith, B. 6, 1873, p. 1398; G. Amiard, Bull. Soc. chim. 1956, p. 1360). Als Abspaltungs-Reagenzien werden dabei u.a. HgO, bestimmte Pyridinium Salze, Chloressigsäureester, Cyanursäurechlorid, p-Toluolsulfochlorid oder bestimmte Phosphorsäureester-Derivate verwendet.

Die Isothiocyanate der Formel II können nach im Prinzip bekannten Methoden hergestellt werden, z.B. indem man ein Anilin der Formel V

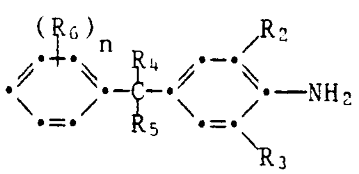

(V),

mit Thiophosgen umsetzt, wobei $R_2$, $R_3$, $R_4$, $R_5$, $R_6$ und n die für Formel I angegebene Bedeutung haben.

Das Verfahren zur Herstellung der Verbindungen der Formel II wird zweckmässigerweise in Gegenwart einer organischen oder anorganischen Base und einem gegenüber den Reaktionsteilnehmern inerten Lösungs- oder Verdünnungsmittels, bei einer Temperatur zwischen 0 und +100°C und bei normalem Druck durchgeführt. Geeignete Lösungs- und Verdünnungsmittel sind u.a. Aether oder ätherartige Verbindungen wie z.B. Diäthyläther, Di-isopropyläther, Dioxan oder Tetrahydrofuran; aromatische Kohlenwasserstoffe wie Benzol, Toluol oder Xylole; Ketone wie Aceton, Methyläthylketon oder Cyclohexanon; oder chlorierte Kohlenwasserstoffe, wie Dichlormethan oder Tetrachlormethan.

Geeignete Basen können organischen oder anorganischen Ursprungs sein wie z.B. Natriumhydrid,

Natrium- oder Calciumcarbonat, tertiäre Amine wie Triäthylamin, Triäthylendiamin oder 4-Dimethylaminopyridin oder Pyridin.

Eine andere Möglichkeit zur Herstellung der Isothiocyanate der Formel II besteht im Umweg über den entsprechenden, an einem N-Atom unsubstituierten Thioharnstoff. Dabei wird ein Anilin der Formel V mit Ammoniumrhodanid in saurem, vorzugsweise mineralsaurem Medium zum entsprechenden Thioharnstoff umgesetzt, der seinerseits beim Erhitzen auf +130 bis 200°C Ammoniak abspaltet und in ein Isothiocyanat der Formel II übergeht (vgl. Saul Patai "The chemistry of cyanates and their thio derivatives", John Wiley and Sons, 1977, p. 1032 ff; Chemistry and Industry, July 3, 1954, p. 735; J.N. Baxter et al., "New method of preparation of aryl isothiocyanates").

Die Aniline der Formel V ihrerseits können nach im Prinzip bekannten Methoden hergestellt werden, z.B. indem man ein Anilin der Formel VI

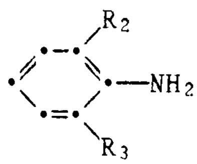

(VI)

bei erhöhter Temperatur und gegebenenfalls unter Druck in Gegenwart einer Säure als Katalysator, vorzugsweise einem Metallsalz wie z.B. Zinkchlorid in wässrigem saurem Medium mit einer Verbindung der Formel VII

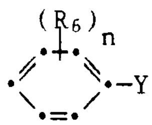

(VII)

umsetzt (vgl. EP-OS 069 065). Dabei steht Y für

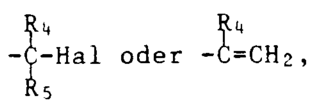

oder ,

wobei Hal Halogen, insbesondere Chlor bedeutet und $R_4$, $R_5$, $R_6$ und n die für Formel I angegebene Bedeutung haben. Eine andere Herstellungsmöglichkeit besteht in der Umsetzung eines Anilins der Formel Va-Vc

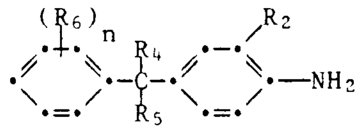

(Va),

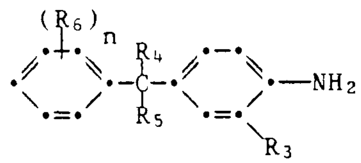

(Vb) oder

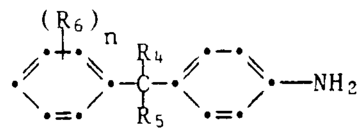

(Vc)

mit einem $R_2$ bzw. $R_3$ entsprechenden Alken oder Cycloalken in Gegenwart von Aluminium und Aluminiumchlorid unter Druck und bei erhöhter Temperatur (vgl. DE-OS 27 27 529).

Aniline der Formel V, in denen $R_2$ und/oder $R_3$ $C_5$-$C_6$-Cycloalkenyl bedeuten, können nach der Aza-Claisen-Umlagerung hergestellt werden (vgl. I.B. Abdrakhmanov et al., Zh. Org. Khim. 15, 2601, 1979; Izv. Akad. Nauk. SSSR, Ser. Khim. 1982, 2160). Die katalytische Hydrierung dieser Aniline liefert Aniline, in denen $R_2$ und/oder $R_3$ für $C_5$-$C_6$-Cycloalkyle stehen.

Eine andere in manchen Fällen gangbare Methode ist die Umlagerung entsprechend substituierter Benzylaniline analog zu M. Elliott et al., J. Chem. Soc. (C) 1967, 1780.

Die Verbindungen der Formeln II und V sind zum Teil bekannt oder können nach im Prinzip bekannten Methoden hergestellt werden. Die neuen Verbindungen bilden ebenfalls einen Gegenstand der vorliegenden Erfindung und entsprechen den Formeln II′ und V′

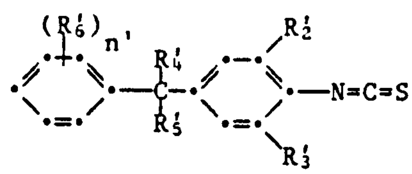

(II') und

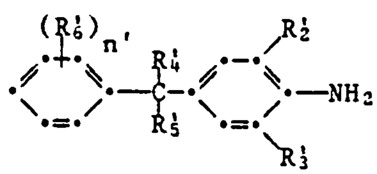

(V') .

Dabei stehen $R_2^1$ und $R_3^1$ je für $C_3$-$C_5$-Alkyl oder $C_5$-$C_6$-Cycloalkyl, insbesondere für $C_3$-$C_5$-Alkyl; $R_4^1$ und $R_5^1$ je für Wasserstoff oder Methyl; $R_6^1$ für Fluor, Chlor oder Methyl; und n' für 0 oder 1, insbesondere für 0.

Die Verbindungen der Formeln III, IV, VI und VII sind bekannt oder können nach im Prinzip bekannten Methoden hergestellt werden.

Es sind bereits aus der EP-Patentanmeldung 175.649 Phenoxyphenylcarbodiimide und aus der DE-OS 3 034 905 sowie der EP-Patentanmeldung 025.010 Phenoxyphenylthiound -isothioharnstoffe mit insektizider und akarizider Wirkung bekannt. Auch in der BE-PS 888.179 werden substituierte N-Phenoxyphenyl-N'-alkyl-thioharnstoffe und -isothioharnstoffe mit insektizider und akarizider Wirkung beschrieben. Demgegenüber unterscheiden sich die erfindungsgemässen Thioharnstoff- und und Isothioharnstoff-Verbindungen der Formel I strukturell im wesentlichen durch das Vorliegen einer N-Benzylphenylgruppierung.

Ueberraschenderweise wurde gefunden, dass auch die erfindungsgemässen Verbindungen der Formel I bei günstiger Warmblüter- und Pflanzenverträglichkeit wertvolle Wirkstoffe bei der Kontrolle von Schädlingen sind. So eignen sich die Verbindungen der Formel I z.B. zur Kontrolle von Schädlingen an Tieren und Pflanzen. Solche Schädlinge gehören hauptsächlich dem Stamm der Arthropoden an, wie insbesondere

Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen wirkungsvoll bekämpft werden. Werden Verbindungen der Formel I von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Kontrolle von tierparasitären Schädlingen, insbesondere an Haus- und Nutztieren, kommen vor allem Ektoparasiten, wie z.B. Milben und Zecken und Dipteren, wie z.B. Lucilia sericata in Betracht.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit in der Formulierungstechnik üblichen, inerten und pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z. B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit anderen Insektiziden oder Akariziden, 1 bis 99,9 % eines festen oder flüssigen Zusatzstoffes und 0 bis 25 %, insbesondere 0,1 bis 20 %, eines Tensides, Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung

1.1. Zwischenprodukte

1.1.1. Benzylaniline

1.1.1.1. 2,6-Diäthyl-4-benzyl-anilin

74,6 g Diäthylanilin werden bei Raumtemperatur mit 63,3 g Benzylchlorid vermischt und 17 Stunden lang auf +230 °C erhitzt (Badtemperatur). Nach dem Erkalten wird das Reaktionsgemisch mit 10%igem wässrigem Natriumhydroxid und Aether verrührt. Die Aetherphase wird zweimal mit Wasser und einmal mit gesättigter wässriger Natriumchloridlösung gewaschen, über Natriumsulfat getrocknet und am Vakuum eingedampft. Der Rückstand wird am Vakuum fraktioniert, worauf die Titelverbindung der Formel

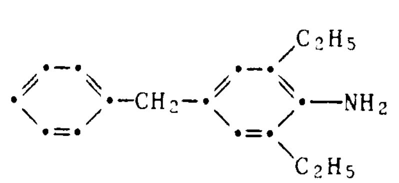

(Verb. Nr. 1.1.1.1.)

in Form eines farblosen Oels vorliegt; Sdp. 113-120 ° C/0,03 Torr; Brechungsindex $n_D^{22}$ : 1,5858.

1.1.1.2. 2,6-Di-sec.butyl-4-benzyl-anilin

81 g 4-Benzylanilin, 1,2 g Aluminiumgries, 3,6 g wasserfreies Aluminiumchlorid und 100 g Buten-1 werden im Autoklaven 24 Stunden lang auf +260 ° C erhitzt. Nach dem Erkalten wird die Reaktionsmasse in eisgekühlte verdünnte Natronlauge gegossen und in Aether aufgenommen. Die Aetherphase wird abgetrennt, über Natriumsulfat getrocknet und fraktioniert destilliert; Sdp. 130-134 ° C/0,03 Torr. Nach chromatographischer Reinigung an Kieselgel mit Hexan/Essigsäureäthylester als Laufmittel liegt die Titelverbindung der Formel

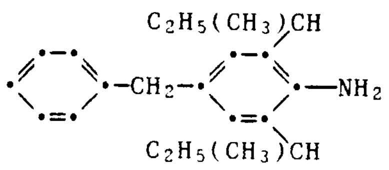

(Verb. Nr. 1.1.1.2.)

in Form eines farblosen Oels vor; Brechungsindex $n_D^{24}$ : 1,5579.

Auf analoge Weise wie unter 1.1.1.1. und 1.1.1.2. beschrieben werden die folgenden Verbindungen hergestellt:

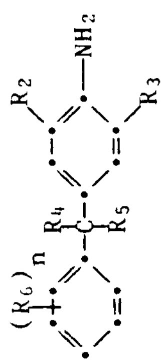

| Verb.Nr. | $R_2$ | $R_3$ | $R_4$ | $R_5$ | $R_6$ | n | phys. Daten |
|---|---|---|---|---|---|---|---|
| 1.1.1.3. | $C_2H_5$ | $C_2H_5$ | $CH_3$ | $CH_3$ | - | 0 | $n_D^{24}$: 1,5750 |
| 1.1.1.4. | $CH(CH_3)_2$ | $CH(CH_3)_2$ | H | H | - | 0 | $n_D^{24}$: 1,5686 |
| 1.1.1.5. | $CH(CH_3)_2$ | $CH(CH_3)_2$ | $CH_3$ | $CH_3$ | - | 0 | $n_D^{22}$: 1,5600 |
| 1.1.1.6. | $CH(CH_3)_2$ | $CH(CH_3)_2$ | $CH_3$ | H | - | 0 | $n_D^{23}$: 1,5636 |
| 1.1.1.7. | $C_2H_5$ | $CH(CH_3)_2$ | H | H | - | 0 | $n_D^{24}$: 1,5762 |
| 1.1.1.8. | $C_2H_5$ | $CH(CH_3)_2$ | H | H | 4-F | 1 | $n_D^{22}$: 1,5636 |
| 1.1.1.9. | $CH(CH_3)_2$ | $CH(CH_3)_2$ | H | H | 4-F | 1 | $n_D^{25}$: 1,5562 |
| 1.1.1.10. | $CH(CH_3)_2$ | $CH(CH_3)_2$ | H | H | 3-F | 1 | $n_D^{24}$: 1,5565 |
| 1.1.1.11. | $CH(CH_3)_2$ | $CH(CH_3)_2$ | H | H | 2-F | 1 | Smp. 38-39°C |
| | $CH(CH_3)_2$ | $CH(CH_3)_2$ | H | H | 2,4-$F_2$ | 2 | |
| | $CH(CH_3)_2$ | $CH(CH_3)_2$ | H | H | 4-Cl | 1 | |
| | $CH(CH_3)_2$ | $CH(CH_3)_2$ | H | H | 2,4-$Cl_2$ | 2 | |
| | $CH(CH_3)_2$ | Cyclo-pentyl | H | H | H | 0 | |
| | Cyclo-pentyl | Cyclo-pentyl | H | H | H | 0 | |

1.1.2. Isothiocyanate

1.1.2.1. 2,6-Diäthyl-4-benzyl-phenylisothiocyanat

34,0 g Thiophosgen, 54,8 g Calciumcarbonat und 460 ml Dichlormethan werden mit 230 ml Wasser verrührt. Zu diesem Gemisch wird bei Raumtemperatur tropfenweise eine Lösung von 59,3 g 2,6-Diäthyl-4-benzylanilin in 200 ml Dichloräthan eingerührt. Das Reaktionsgemisch wird 2 Stunden lang unter Rückfluss gerührt und nach dem Erkalten über Kieselgur filtriert. Aus dem Filtrat wird die organische Phase abgetrennt, über Natriumsulfat getrocknet und vom Lösungsmittel befreit. Die Titelverbindung der Formel

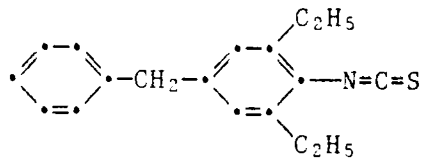

(Verb. Nr. 1.1.2.1.)

liegt in Form eines gelben Oels vor, das ohne weitere Reinigung für die nächste Reaktion verwendet wird.

1.1.2.2. 2,6-Di-sec.butyl-4-benzyl-phenylisothiocyanat

14,3 g Thiophosgen, 23,0 g Calciumcarbonat und 460 ml Dichlormethan werden mit 230 ml Wasser verrührt. Zu diesem Gemisch wird bei Raumtemperatur tropfenweise eine Lösung von 30,8 g 2,6-Di-sec.butyl-4-benzylanilin in 200 ml Dichloräthan eingerührt. Das Reaktionsgemisch wird 2 Stunden lang unter Rückfluss gerührt und nach dem Erkalten über Kieselgur filtriert. Aus dem Filtrat wird die organische Phase abgetrennt, über Natriumsulfat getrocknet und vom Lösungsmittel befreit. Die Titelverbindung der Formel

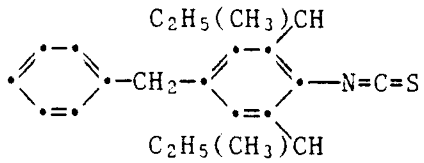

(Verb. Nr. 1.1.2.2.)

liegt in Form eines gelben Oels vor, das ohne weitere Reinigung für die nächste Reaktion verwendet wird.

Auf analoge Weise werden die folgenden Verbindungen hergestellt.

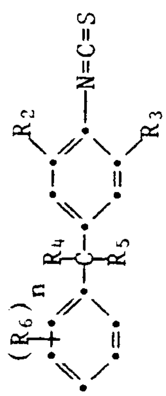

| Verb.Nr. | $R_2$ | $R_3$ | $R_4$ | $R_5$ | $R_6$ | n | phys. Daten |
|---|---|---|---|---|---|---|---|
| 1.1.2.3. | $C_2H_5$ | $C_2H_5$ | $CH_3$ | $CH_3$ | - | 0 | $n_D^{24}$: 1,6101 |
| 1.1.2.4. | $CH(CH_3)_2$ | $CH(CH_3)_2$ | H | H | - | 0 | Smp. 40-43°C |
| 1.1.2.5. | $CH(CH_3)_2$ | $CH(CH_3)_2$ | $CH_3$ | $CH_3$ | - | 0 | $n_D^{27}$: 1,5923 |
| 1.1.2.6. | $CH(CH_3)_2$ | $CH(CH_3)_2$ | $CH_3$ | H | - | 0 | $n_D^{24}$: 1,5998 |
| 1.1.2.7. | $C_2H_5$ | $CH(CH_3)_2$ | H | H | - | 0 | $n_D^{23}$: 1,6143 |
| 1.1.1.8. | $C_2H_5$ | $CH(CH_3)_2$ | H | H | 4-F | 1 | $n_D^{25}$: 1,6023 |
| 1.1.1.9. | $CH(CH_3)_2$ | $CH(CH_3)_2$ | H | H | 4-F | 1 | $n_D^{24}$: 1,5948 |
| 1.1.1.10. | $CH(CH_3)_2$ | $CH(CH_3)_2$ | H | H | 3-F | 1 | $n_D^{24}$: 1,5943 |
| 1.1.1.11. | $CH(CH_3)_2$ | $CH(CH_3)_2$ | H | H | 2-F | 1 | $n_D^{24,5}$: 1,5944 |
| | $CH(CH_3)_2$ | $CH(CH_3)_2$ | H | H | 2,4-$F_2$ | 2 | |
| | $CH(CH_3)_2$ | $CH(CH_3)_2$ | H | H | 4-Cl | 1 | |
| | $CH(CH_3)_2$ | $CH(CH_3)_2$ | H | H | 2,4-$Cl_2$ | 2 | |
| | $CH(CH_3)_2$ | Cyclo-pentyl | H | H | H | 0 | |
| | Cyclo-pentyl | Cyclo-pentyl | H | H | H | 0 | |

### 1.2. Endprodukte

#### 1.2.1. Phenylthioharnstoffe

##### 1.2.1.1. N-[(2,6-Diäthyl-4-benzyl)-phenyl]-N'-tert.butyl-thioharnstoff

22,9 g 2,6-Diäthyl-4-benzyl-phenylisothiocyanat und 15 g tert.Butylamin werden mit 100 ml Tetrahydrofuran verdünnt und 24 Stunden bei Raumtemperatur stehen gelassen. Das Reaktionsgemisch wird auf Eiswasser gegossen, der entstandene Niederschlag abfiltriert, mit Wasser gewaschen und nach dem Trocknen aus Aethanol umkristallisiert. Die Titelverbindung der Formel

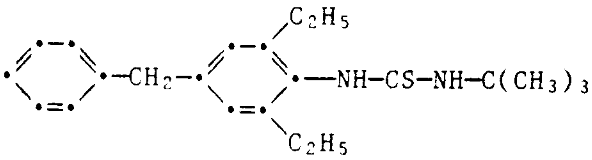

(Verb. Nr. 1.2.1.1.)

liegt als farbloser Feststoff vor; Smp. 136-138 ° C.

Auf analoge Weise werden die folgenden Verbindungen hergestellt:

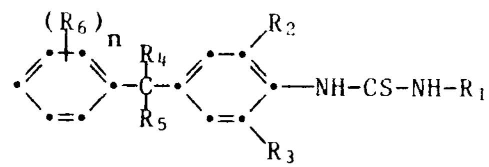

| Verb.Nr. | $R_1$ | $R_2$ | $R_3$ | $R_4$ | $R_5$ | $R_6$ | n | phys. Daten |
|---|---|---|---|---|---|---|---|---|
| 1.2.1.2. | $CH(CH_3)_2$ | $C_2H_5$ | $C_2H_5$ | H | H | – | 0 | Smp. 135-137°C |
| 1.2.1.3. | Cyclopentyl | $C_2H_5$ | $C_2H_5$ | H | H | – | 0 | Smp. 127-129°C |
| 1.2.1.4. | $C(CH_3)_3$ | $CH(CH_3)_2$ | $CH(CH_3)_2$ | H | H | – | 0 | Smp. 153-154°C |
| 1.2.1.5. | $C(CH_3)_3$ | $C_2H_5$ | $C_2H_5$ | $CH_3$ | $CH_3$ | – | 0 | Smp. 142-144°C |
| 1.2.1.6. | $CH(CH_3)_2$ | $C_2H_5$ | $C_2H_5$ | $CH_3$ | $CH_3$ | – | 0 | Smp. 156-158°C |
| 1.2.1.7. | $C(CH_3)_3$ | $CH(CH_3)C_2H_5$ | $CH(CH_3)C_2H_5$ | H | H | – | 0 | Smp. 134-137°C |
| 1.2.1.8. | $CH(CH_3)_2$ | $CH(CH_3)C_2H_5$ | $CH(CH_3)C_2H_5$ | H | H | – | 0 | Smp. 144-146°C |
| 1.2.1.9. | Cyclopentyl | $C_2H_5$ | $C_2H_5$ | $CH_3$ | $CH_3$ | – | 0 | Smp. 171-173°C |
| 1.2.1.10. | $C(CH_3)_3$ | $CH(CH_3)_2$ | $CH(CH_3)_2$ | $CH_3$ | $CH_3$ | – | 0 | Smp. 144-146°C |
| 1.2.1.11. | $C(CH_3)_3$ | $C_2H_5$ | $CH(CH_3)_2$ | H | H | – | 0 | Smp. 140-141,5°C |
| 1.2.1.12. | $CH(CH_3)_2$ | $CH(CH_3)_2$ | $CH(CH_3)_2$ | H | H | – | 0 | Smp. 188-191°C |
| 1.2.1.13. | Cyclopentyl | $CH(CH_3)_2$ | $CH(CH_3)_2$ | H | H | – | 0 | Smp. 178-180,5°C |
| 1.2.1.14. | $CH(CH_3)_2$ | $CH(CH_3)_2$ | $CH(CH_3)_2$ | $CH_3$ | H | – | 0 | Smp. 171-173°C |
| 1.2.1.15. | $C(CH_3)_3$ | $CH(CH_3)_2$ | $CH(CH_3)_2$ | $CH_3$ | H | – | 0 | Smp. 154-155,5°C |
| 1.2.1.16. | $CH(CH_3)C_2H_5$ | $CH(CH_3)_2$ | $CH(CH_3)_2$ | H | H | – | 0 | Smp. 157-158,5°C |
| 1.2.1.17. | $CH(CH_3)CH(CH_3)_2$ | $CH(CH_3)_2$ | $CH(CH_3)_2$ | H | H | – | 0 | Smp. 157-159°C |
| 1.2.1.18. | $CH(CH_3)_2$ | $C_2H_5$ | $CH(CH_3)_2$ | H | H | 4-F | 1 | Smp. 142-144°C |
| 1.2.1.19. | $C(CH_3)_3$ | $C_2H_5$ | $CH(CH_3)_2$ | H | H | 4-F | 1 | Smp. 145-146°C |
| 1.2.1.20. | $C(CH_3)_3$ | $CH(CH_3)_2$ | $CH(CH_3)_2$ | H | H | 2-F | 1 | Smp. 153-154°C |
| 1.2.1.21. | $CH(CH_3)_2$ | $CH(CH_3)_2$ | $CH(CH_3)_2$ | H | H | 2-F | 1 | Smp. 192-194°C |
| 1.2.1.22. | $C(CH_3)_3$ | $CH(CH_3)_2$ | $CH(CH_3)_2$ | H | H | 4-F | 1 | Smp. 154,5-156°C |
| 1.2.1.23. | $CH(CH_3)_2$ | $CH(CH_3)_2$ | $CH(CH_3)_2$ | H | H | 3-F | 1 | Smp. 184-186,5°C |
| 1.2.1.24 | $C(CH_3)_3$ | $CH(CH_3)_2$ | $CH(CH_3)_2$ | H | H | 3-F | 1 | Smp. 138-139,5°C |

| Verb.Nr. | $R_1$ | $R_2$ | $R_3$ | $R_4$ | $R_5$ | $R_6$ | n | phys. Daten |
|---|---|---|---|---|---|---|---|---|
| 1.2.1.25. | $CH(CH_3)_2$ | $CH(CH_3)_2$ | $CH(CH_3)_2$ | H | H | 2-F | 1 | Smp. 192-194°C |
| 1.2.1.26. | $C(CH_3)_3$ | $CH(CH_3)_2$ | $CH(CH_3)_2$ | H | H | 2-F | 1 | Smp. 153-154°C |
| | $C(CH_3)_3$ | $CH(CH_3)_2$ | $CH(CH_3)_2$ | H | H | 2,4-$F_2$ | 2 | |
| | $CH(CH_3)_2$ | $CH(CH_3)_2$ | $CH(CH_3)_2$ | H | H | 2,4-$F_2$ | 2 | |
| | $C(CH_3)_3$ | $CH(CH_3)_2$ | $CH(CH_3)_2$ | H | H | 4-Cl | 1 | |
| | $CH(CH_3)_2$ | $CH(CH_3)_2$ | $CH(CH_3)_2$ | H | H | 4-Cl | 1 | |
| | $C(CH_3)_3$ | $CH(CH_3)_2$ | $CH(CH_3)_2$ | H | H | 2,4-$Cl_2$ | 2 | |
| | $CH(CH_3)_2$ | $CH(CH_3)_2$ | $CH(CH_3)_2$ | H | H | 2,4-$Cl_2$ | 2 | |
| | $C(CH_3)_3$ | $CH(CH_3)_2$ | Cyclopentyl | H | H | - | 0 | |
| | $CH(CH_3)_2$ | $CH(CH_3)_2$ | Cyclopentyl | H | H | - | 0 | |
| | $C(CH_3)_3$ | Cyclopentyl | Cyclopentyl | H | H | - | 0 | |
| | $CH(CH_3)_2$ | Cyclopentyl | Cyclopentyl | H | H | - | 0 | |
| | 1-$(CH_3)$-Cyclopropyl | $CH(CH_3)_2$ | $CH(CH_3)_2$ | H | H | - | 0 | |
| | 1-$(CH_3)$-Cyclopropyl | $CH(CH_3)_2$ | $CH(CH_3)_2$ | $CH_3$ | H | - | 0 | |

1.2.2. Phenylisothioharnstoffe

1.2.2.1. N-[(2,6-Diäthyl-4-benzyl)-phenyl]-N′-(tert.butyl-S-methyl-isothioharnstoff

8 g N-[(2,6-Diäthyl-4-benzyl)-phenyl]-N′-tert.butyl-thioharnstoff in 30 ml Aethanol werden bei Raumtemperatur mit 4,8 g Methyljodid versetzt und 4 Stunden lang unter Rühren auf +50°C erwärmt. Nach dem Erkalten wird die Reaktionslösung auf Wasser gegossen und vom entstandenen Niederschlag, dem Hydrojodid des Isothioharnstoffs, abfiltriert (Smp. 140-144°C). Dieses Isothiuronium-hydrojodid wird in Dichlormethan gelöst, zweimal mit 5%iger wässriger Natriumcarbonatlösung ausgeschüttelt und schliesslich mit Wasser neutral gewaschen. Die organische Phase wird über Natriumsulfat getrocknet und das Lösungsmittel abgedampft. Die Titelverbindung der Formel

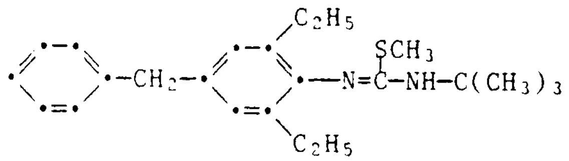

(Verb. Nr. 1.2.2.1.)

liegt in Form eines farblosen Kristallpulvers vor; Smp. 62,5-64,5°C.

Auf analoge Weise werden die folgenden Verbindungen hergestellt:

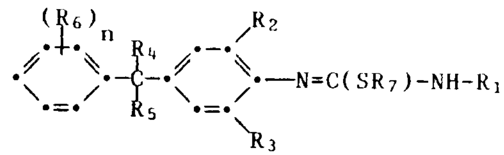

| Verb.Nr. | $R_1$ | $R_2$ | $R_3$ | $R_4$ | $R_5$ | $R_6$ | $R_7$ | n | phys. Daten |
|---|---|---|---|---|---|---|---|---|---|
| 1.2.2.2. | $CH(CH_3)_2$ | $C_2H_5$ | $C_2H_5$ | H | H | - | $CH_3$ | 0 | Smp. 69- 71°C |
| 1.2.2.3. | Cyclopentyl | $C_2H_5$ | $C_2H_5$ | H | H | - | $CH_3$ | 0 | Smp. 47- 48°C |
| 1.2.2.4. | $C(CH_3)_3$ | $CH(CH_3)_2$ | $CH(CH_3)_2$ | H | H | - | $CH_3$ | 0 | Smp. 104-105°C |
| 1.2.2.5. | $C(CH_3)_3$ | $C_2H_5$ | $C_2H_5$ | $CH_3$ | $CH_3$ | - | $CH_3$ | 0 | $n_D^{22}$: 1,5700 |
| 1.2.2.6. | $CH(CH_3)_2$ | $C_2H_5$ | $C_2H_5$ | $CH_3$ | $CH_3$ | - | $CH_3$ | 0 | $n_D^{22}$: 1,5720 |
| 1.2.2.7. | $C(CH_3)_3$ | $CH(CH_3)C_2H_5$ | $CH(CH_3)C_2H_5$ | H | H | - | $CH_3$ | 0 | Smp. 82-85,5°C |
| 1.2.2.8. | $CH(CH_3)_2$ | $CH(CH_3)C_2H_5$ | $CH(CH_3)C_2H_5$ | H | H | - | $CH_3$ | 0 | Smp. 34- 37°C |
| 1.2.2.9. | Cyclopentyl | $C_2H_5$ | $C_2H_5$ | $CH_3$ | $CH_3$ | - | $CH_3$ | 0 | Smp. 47-48,5°C |
| 1.2.2.10. | $C(CH_3)_3$ | $CH(CH_3)_2$ | $CH(CH_3)_2$ | $CH_3$ | $CH_3$ | - | $CH_3$ | 0 | Smp. 85- 87°C |
| 1.2.2.11. | $CH(CH_3)_2$ | $CH(CH_3)_2$ | $CH(CH_3)_2$ | H | H | - | $C_2H_5$ | 0 | $n_D^{26}$: 1,5592 |
| 1.2.2.12. | $CH(CH_3)_2$ | $CH(CH_3)_2$ | $CH(CH_3)_2$ | H | H | - | $(CH_2)_2CH_3$ | 0 | $n_D^{26}$: 1,5552 |
| 1.2.2.13. | $CH(CH_3)_2$ | $CH(CH_3)_2$ | $CH(CH_3)_2$ | H | H | - | $CH_3$ | 0 | Smp. 73,5-75,5°C |
| 1.2.2.14. | Cyclopentyl | $CH(CH_3)_2$ | $CH(CH_3)_2$ | H | H | - | $CH_3$ | 0 | Smp. 81,5-83,5°C |
| 1.2.2.15. | $C(CH_3)_3$ | $C_2H_5$ | $CH(CH_3)_2$ | H | H | - | $CH_3$ | 0 | Smp. 76- 78,5°C |
| 1.2.2.16. | $C(CH_3)_3$ | $C_2H_5$ | $CH(CH_3)_2$ | H | H | - | $C_2H_5$ | 0 | $n_D^{25}$: 1,5639 |
| 1.2.2.17. | $C(CH_3)_3$ | $C_2H_5$ | $CH(CH_3)_2$ | H | H | - | $(CH_2)_2CH_3$ | 0 | $n_D^{25}$: 1,5598 |
| 1.2.2.18. | $C(CH_3)_3$ | $CH(CH_3)_2$ | $CH(CH_3)_2$ | $CH_3$ | H | - | $CH_3$ | 0 | Smp. 106,5-110°C |
| 1.2.2.19. | $CH(CH_3)_2$ | $CH(CH_3)_2$ | $CH(CH_3)_2$ | $CH_3$ | H | - | $CH_3$ | 0 | Smp. 66- 69°C |
| 1.2.2.20. | $CH(CH_3)C_2H_5$ | $CH(CH_3)_2$ | $CH(CH_3)_2$ | H | H | - | $CH_3$ | 0 | $n_D^{24}$: 1,5616 |

| Verb.Nr. | $R_1$ | $R_2$ | $R_3$ | $R_4$ | $R_5$ | $R_6$ | $R_7$ | n | phys. Daten |
|---|---|---|---|---|---|---|---|---|---|
| 1.2.2.21. | $CH(CH_3)C_2H_5$ | $CH(CH_3)_2$ | $CH(CH_3)_2$ | H | H | - | $C_2H_5$ | 0 | $n_D^{24}$: 1,5575 |
| 1.2.2.22. | $C(CH_3)_3$ | $C_2H_5$ | $CH(CH_3)_2$ | H | H | 4-F | $CH_3$ | 1 | Smp. 65-67°C |
| 1.2.2.23. | $CH(CH_3)C_2H_5$ | $CH(CH_3)_2$ | $CH(CH_3)_2$ | H | H | - | $(CH_2)_2CH_3$ | 0 | $n_D^{24,5}$: 1,5525 |
| 1.2.2.24. | $CH(CH_3)CH(CH_3)_2$ | $CH(CH_3)_2$ | $CH(CH_3)_2$ | H | H | - | $CH_3$ | 0 | Smp. 57,5-60°C |
| 1.2.2.25. | $CH(CH_3)_2$ | $C_2H_5$ | $CH(CH_3)_2$ | H | H | 4-F | $CH_3$ | 1 | $n_D^{24}$: 1,5620 |
| 1.2.2.26. | $C(CH_3)_3$ | $CH(CH_3)_2$ | $CH(CH_3)_2$ | H | H | 2-F | $CH_3$ | 1 | Smp. 93-96°C |
| 1.2.2.27. | $C(CH_3)_3$ | $CH(CH_3)_2$ | $CH(CH_3)_2$ | H | H | 3-F | $CH_3$ | | Smp. 72-75°C |
| 1.2.2.28. | $C(CH_3)_3$ | $CH(CH_3)_2$ | $CH(CH_3)_2$ | H | H | 4-F | $CH_3$ | 1 | Smp. 83-85,5°C |
| 1.2.2.29. | $CH(CH_3)_2$ | $CH(CH_3)_2$ | $CH(CH_3)_2$ | H | H | 3-F | $CH_3$ | 1 | Smp. 61-62,5°C |
| 1.2.2.30. | $CH(CH_3)CH(CH_3)_2$ | $CH(CH_3)_2$ | $CH(CH_3)_2$ | H | H | - | $(CH_2)_2CH_3$ | 0 | $n_D^{25}$: 1,5485 |
| 1.2.2.31. | $CH(CH_3)CH(CH_3)_2$ | $CH(CH_3)_2$ | $CH(CH_3)_2$ | H | H | - | $C_2H_5$ | 0 | $n_D^{25}$: 1,5515 |
| 1.2.2.32. | $CH(CH_3)CH(CH_3)_2$ | $CH(CH_3)_2$ | $CH(CH_3)_2$ | H | H | - | $CH_3$ | 0 | HJ-Salz Smp. 190-194°C |
| 1.2.2.33. | $CH(CH_3)CH(CH_3)_2$ | $CH(CH_3)_2$ | $CH(CH_3)_2$ | H | H | - | $C_2H_5$ | 0 | HJ-Salz Smp. 163-168°C |
| 1.2.2.34. | $CH(CH_3)CH(CH_3)_2$ | $CH(CH_3)_2$ | $CH(CH_3)_2$ | H | H | - | $(CH_2)_2CH_3$ | 0 | HJ-Salz Smp. 156-159°C |
| 1.2.2.35. | $C(CH_3)_3$ | $CH(CH_3)_2$ | $CH(CH_3)_2$ | H | H | 3-F | $CH_3$ | 1 | HJ-Salz Smp. 160-163,5°C |
| | $C(CH_3)_3$ | $CH(CH_3)_2$ | $CH(CH_3)_2$ | H | H | 2,4-$F_2$ | $CH_3$ | 2 | |
| | $CH(CH_3)_2$ | $CH(CH_3)_2$ | $CH(CH_3)_2$ | H | H | 2,4-$F_2$ | $CH_3$ | 2 | |
| | $C(CH_3)_3$ | $CH(CH_3)_2$ | $CH(CH_3)_2$ | H | H | 4-Cl | $CH_3$ | 1 | |
| | $CH(CH_3)_2$ | $CH(CH_3)_2$ | $CH(CH_3)_2$ | H | H | 4-Cl | $CH_3$ | 1 | |
| | $C(CH_3)_3$ | $CH(CH_3)_2$ | $CH(CH_3)_2$ | H | H | 2,4-$Cl_2$ | $CH_3$ | 2 | |
| | $CH(CH_3)_2$ | $CH(CH_3)_2$ | $CH(CH_3)_2$ | H | H | 2,4-$Cl_2$ | $CH_3$ | 2 | |
| | $C(CH_3)_3$ | $CH(CH_3)_2$ | Cyclopentyl | H | H | - | $CH_3$ | 0 | |

| Verb.Nr. | $R_1$ | $R_2$ | $R_3$ | $R_4$ | $R_5$ | $R_6$ | $R_7$ | n | phys. Daten |
|---|---|---|---|---|---|---|---|---|---|
| | $CH(CH_3)_2$ | $CH(CH_3)_2$ | Cyclopentyl | H | H | - | $CH_3$ | 0 | |
| | $C(CH_3)_3$ | Cyclopentyl | Cyclopentyl | H | H | - | $CH_3$ | 0 | |
| | $CH(CH_3)_2$ | Cyclopentyl | Cyclopentyl | H | H | - | $CH_3$ | 0 | |
| | 1-($CH_3$)-Cyclopropyl | $CH(CH_3)_2$ | $CH(CH_3)_2$ | H | H | - | $CH_3$ | 0 | |
| | 1-($CH_3$)-Cyclopropyl | $CH(CH_3)_2$ | $CH(CH_3)_2$ | $CH_3$ | H | - | $CH_3$ | 0 | |
| | $C(CH_3)_3$ | $CH(CH_3)_2$ | $CH(CH_3)_2$ | H | H | - | $C_2H_5$ | 0 | |
| | $C(CH_3)_3$ | $CH(CH_3)_2$ | $CH(CH_3)_2$ | H | H | - | $(CH_2)_2CH_3$ | 0 | |
| | $C(CH_3)_3$ | $CH(CH_3)_2$ | $CH(CH_3)_2$ | H | H | - | $(CH_2)_3CH_3$ | 0 | |
| | $C(CH_3)_3$ | $CH(CH_3)_2$ | $CH(CH_3)_2$ | H | H | - | $CH_2CH=CH_2$ | 0 | |
| | $CH(CH_3)_2$ | $CH(CH_3)_2$ | $CH(CH_3)_2$ | H | H | - | $(CH_2)_3CH_3$ | 0 | |
| | $CH(CH_3)_2$ | $CH(CH_3)_2$ | $CH(CH_3)_2$ | H | H | - | $CH_2CH=CH_2$ | 0 | |
| | $CH(CH_3)_2$ | $C_2H_5$ | $CH(CH_3)_2$ | H | H | - | $CH_3$ | 0 | |
| | $CH(CH_3)_2$ | $CH(CH_3)_2$ | Cyclopentyl | H | H | - | $CH_3$ | 0 | |

1.2.3. Phenylcarbodiimide

1.2.3.1. N-[(2,6-Diäthyl-4-benzyl)-phenyl]-N′-tert.butyl-carbodiimid

8 g N-[(2,6-Diäthyl-4-benzyl)-phenyl]-N′-tert.butyl-thioharnstoff und 6,9 g 2-Chlor-1-methylpyridiniumjo-

did werden in 30 ml trockenem Acetonitril vorgelegt, bei Raumtemperatur tropfenweise mit 5,5 g Triäthylamin in 20 ml Acetonitril versetzt und während 3 Stunden unter Rückfluss gerührt. Anschliessend wird das Lösungsmittel am Vakuum entfernt, der Rückstand in Hexan aufgenommen und filtriert. Das Filtrat wird dreimal mit Wasser gewaschen, über Natriumsulfat getrocknet, mit Kieselgel entfärbt und am Vakuum vom Lösungsmittel befreit. Die Titelverbindung der Formel

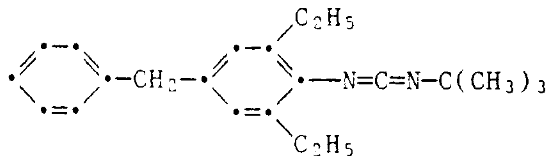

(Verb. Nr. 1.2.3.1.)

liegt als farbloses Oel vor; Brechungsindex $n_D^{22}$ : 1,5658.

Auf analoge Weise werden die folgenden Verbindungen hergestellt:

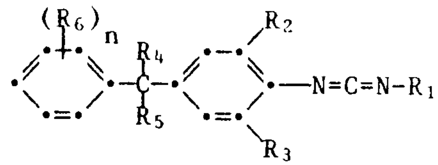

| Verb.Nr. | $R_1$ | $R_2$ | $R_3$ | $R_4$ | $R_5$ | $R_6$ | n | phys. Daten |
|---|---|---|---|---|---|---|---|---|
| 1.2.3.2. | $CH(CH_3)_2$ | $C_2H_5$ | $C_2H_5$ | H | H | – | 0 | $n_D^{22}$: 1,5731 |
| 1.2.3.3. | Cyclopentyl | $C_2H_5$ | $C_2H_5$ | H | H | – | 0 | $n_D^{22}$: 1,5839 |
| 1.2.3.4. | $C(CH_3)_3$ | $CH(CH_3)_2$ | $CH(CH_3)_2$ | H | H | – | 0 | $n_D^{22}$: 1,5552 |
| 1.2.3.5. | $C(CH_3)_3$ | $C_2H_5$ | $C_2H_5$ | $CH_3$ | $CH_3$ | – | 0 | $n_D^{23}$: 1,5609 |
| 1.2.3.6. | $CH(CH_3)_2$ | $C_2H_5$ | $C_2H_5$ | $CH_3$ | $CH_3$ | – | 0 | $n_D^{24}$: 1,5657 |
| 1.2.3.7. | $C(CH_3)_3$ | $CH(CH_3)C_2H_5$ | $CH(CH_3)C_2H_5$ | H | H | – | 0 | $n_D^{26}$: 1,5460 |
| 1.2.3.8. | $CH(CH_3)_2$ | $CH(CH_3)C_2H_5$ | $CH(CH_3)C_2H_5$ | H | H | – | 0 | $n_D^{26}$: 1,5495 |
| 1.2.3.9. | Cyclopentyl | $C_2H_5$ | $C_2H_5$ | $CH_3$ | $CH_3$ | – | 0 | $n_D^{24}$: 1,5772 |
| 1.2.3.10. | $C(CH_3)_3$ | $CH(CH_3)_2$ | $CH(CH_3)_2$ | $CH_3$ | $CH_3$ | – | 0 | $n_D^{25}$: 1,5472 |
| 1.2.3.11. | $C(CH_3)_3$ | $C_2H_5$ | $CH(CH_3)_2$ | H | H | – | 0 | $n_D^{25}$: 1,5588 |
| 1.2.3.12. | $CH(CH_3)_2$ | $CH(CH_3)_2$ | $CH(CH_3)_2$ | H | H | – | 0 | $n_D^{26}$: 1,5593 |
| 1.2.3.13. | Cyclopentyl | $CH(CH_3)_2$ | $CH(CH_3)_2$ | H | H | – | 0 | $n_D^{26}$: 1,5704 |
| 1.2.3.14. | $CH(CH_3)_2$ | $CH(CH_3)_2$ | $CH(CH_3)_2$ | $CH_3$ | H | – | 0 | $n_D^{23}$: 1,5562 |
| 1.2.3.15. | $C(CH_3)_3$ | $CH(CH_3)_2$ | $CH(CH_3)_2$ | $CH_3$ | H | – | 0 | $n_D^{23}$: 1,5516 |
| 1.2.3.16. | $CH(CH_3)C_2H_5$ | $CH(CH_3)_2$ | $CH(CH_3)_2$ | H | H | – | 0 | $n_D^{25,5}$: 1,5559 |

| Verb.Nr. | $R_1$ | $R_2$ | $R_3$ | $R_4$ | $R_5$ | $R_6$ | n | phys. Daten |
|---|---|---|---|---|---|---|---|---|
| 1.2.3.17. | $CH(CH_3)CH(CH_3)_2$ | $CH(CH_3)_2$ | $CH(CH_3)_2$ | H | H | - | 0 | $n_D^{25,5}$: 1,5538 |
| 1.2.3.18. | $CH(CH_3)_2$ | $C_2H_5$ | $CH(CH_3)_2$ | H | H | 4-F | 1 | $n_D^{24}$: 1,5555 |
| 1.2.3.19. | $C(CH_3)_3$ | $CH(CH_3)_2$ | $CH(CH_3)_2$ | H | H | 2-F | 1 | $n_D^{25}$: 1,5458 |
| 1.2.3.20. | $C(CH_3)_3$ | $C_2H_5$ | $CH(CH_3)_2$ | H | H | 4-F | 1 | $n_D^{23}$: 1,5505 |
| 1.2.3.21. | $C(CH_3)_3$ | $CH(CH_3)_2$ | $CH(CH_3)_2$ | H | H | 4-F | 1 | $n_D^{24}$: 1,5445 |
| 1.2.3.22. | $CH(CH_3)_2$ | $CH(CH_3)_2$ | $CH(CH_3)_2$ | H | H | 3-F | 1 | $n_D^{23}$: 1,5483 |
| 1.2.3.23. | $C(CH_3)_3$ | $CH(CH_3)_2$ | $CH(CH_3)_2$ | H | H | 3-F | 1 | $n_D^{22}$: 1,5458 |
| | $CH(CH_3)_2$ | $CH(CH_3)_2$ | $CH(CH_3)_2$ | H | H | 2-F | 1 | |
| | $C(CH_3)_3$ | $CH(CH_3)_2$ | $CH(CH_3)_2$ | H | H | 2,4-$F_2$ | 2 | |
| | $CH(CH_3)_2$ | $CH(CH_3)_2$ | $CH(CH_3)_2$ | H | H | 2,4-$F_2$ | 2 | |
| | $C(CH_3)_3$ | $CH(CH_3)_2$ | $CH(CH_3)_2$ | H | H | 4-Cl | 1 | |
| | $CH(CH_3)_2$ | $CH(CH_3)_2$ | $CH(CH_3)_2$ | H | H | 4-Cl | 1 | |
| | $C(CH_3)_3$ | $CH(CH_3)_2$ | $CH(CH_3)_2$ | H | H | 2,4-$Cl_2$ | 2 | |
| | $CH(CH_3)_2$ | $CH(CH_3)_2$ | $CH(CH_3)_2$ | H | H | 2,4-$Cl_2$ | 2 | |
| | $C(CH_3)_3$ | $CH(CH_3)_2$ | Cyclopentyl | H | H | - | 0 | |
| | $CH(CH_3)_2$ | $CH(CH_3)_2$ | Cyclopentyl | H | H | - | 0 | |
| | $C(CH_3)_3$ | Cyclopentyl | Cyclopentyl | H | H | - | 0 | |
| | $CH(CH_3)_2$ | Cyclopentyl | Cyclopentyl | H | H | - | 0 | |
| | 1-($CH_3$)-Cyclopropyl | $CH(CH_3)_2$ | $CH(CH_3)_2$ | H | H | - | 0 | |
| | 1-($CH_3$)-Cyclopropyl | $CH(CH_3)_2$ | $CH(CH_3)_2$ | $CH_3$ | H | - | 0 | |

Beispiel 2: Formulierungen von Wirkstoffen der Formel I gemäss den Herstellungsbeispielen 1.2.

(% = Gewichtsprozent)

| 2.1. Emulsions-Konzentrate | a) | b) |
|---|---|---|
| Wirkstoff gemäss den Herstellungsbeispielen 1.2. | 10 % | 25 % |
| Ca-Dodecylbenzolsulfonat | - | 5 % |
| Ricinusöl-polyäthylenglykoläther (36 Mol AeO) | 25 % | 5 % |
| Cyclohexanon | - | 40 % |
| Butanol | 15 % | - |
| Xylolgemisch | - | 25 % |
| Essigester | 50 % | - |

Aus solchen Konzentraten können durch Verdünnen mit Wasser Emulsionen jeder gewünschten Konzentration hergestellt werden.

| 2.2. Lösungen | a) | b) |
|---|---|---|
| Wirkstoff gemäss den Herstellungsbeispielen 1.2. | 10 % | 5 % |
| Polyäthylenglykol (MG 400) | 70 % | - |
| N-Methyl-2-pyrrolidon | 20 % | 20 % |
| Epoxidiertes Kokosnussöl | - | 1 % |
| Benzin (Siedegrenzen 160-190 ° C) | - | 74 % |

Die Lösungen sind zur Anwendung in Form kleinster Tropfen geeignet.

| 2.3. Granulate | a) | b) |
|---|---|---|
| Wirkstoff gemäss den Herstellungsbeispielen 1.2. | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 2.4. Extruder Granulat | |
|---|---|
| Wirkstoff gemäss den Herstellungsbeispielen 1.2. | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| 2.5. Umhüllungs-Granulat | |
|---|---|
| Wirkstoff gemäss den Herstellungsbeispielen 1.2. | 3 % |
| Polyäthylenglykol (MG 200) | 3 % |
| Kaolin | 94 % |

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 2.6. Stäubemittel | a) | b) | c) | d) |
|---|---|---|---|---|
| Wirkstoff gemäss den Herstellungsbeispielen 1.2. | 2 % | 5 % | 5 % | 8 % |
| Hochdisperse Kieselsäure | 1 % | 5 % | - | - |
| Talkum | 97 % | - | 95 % | - |
| Kaolin | - | 90 % | - | 92 % |

Durch inniges Vermischen der Trägerstoffe mit dem Wirkstoff und gegebenenfalls Vermahlen auf einer geeigneten Mühle erhält man gebrauchsfertige Stäubemittel.

| 2.7. Spritzpulver | a) | b) | c) |
|---|---|---|---|
| Wirkstoff gemäss den Herstellungsbeispielen 1.2. | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |

Der Wirkstoff wird mit den Zusatzstoffen gut vermischt und in einer geeigneten Mühle vermahlen. Man erhält Spritzpulver, die sich mit Wasser zu Suspensionen jeder gewünschten Konzentration verdünnen lassen.

| 2.8. Suspensions-Konzentrat | |
|---|---|
| Wirkstoff gemäss den Herstellungsbeispielen 1.2. | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Biologische Prüfungen

3.1. Wirkung gegen Musca domestica

Ein Zuckerwürfel wird derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25 ° C und 50 % Luftfeuchtigkeit belassen. Nach 24 Stunden wird die insektizide Wirkung durch Ermittlung der Abtötungsrate festgestellt.

Verbindungen gemäss den Beispielen 1.2. zeigen in diesem Test gute Wirkung.

3.2. Wirkung gegen Lucilia sericata

Zu 9 ml eines Zuchtmediums wird bei 50 ° C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss den Beispielen 1.2. zeigen in diesem Test gute Wirkung gegen Lucilia sericata.

3.3. Wirkung gegen Aedes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 400 ppm erhalten wird. Nach Verdunsten des Acetons wird der Behälter mit 30-40 2tägigen Aedes-Larven beschickt. Nach 2 und 7 Tagen wird die Mortalität geprüft.

Verbindungen gemäss den Beispielen 1.2. zeigen gute Wirkung in diesem Test.

3.4. Wirkung gegen Zecken in verschiedenen Entwicklungsstadien

10 frische, vollgesogene Boophilus microplus Weibchen werden in einer Reihe dorsal auf einer PVC Platte aufgeklebt und mit einem Wattebausch überdeckt. Danach werden die Testtiere mit 10 ml der wässrigen Testlösung übergossen. Eine Stunde später wird der Wattebausch entfernt und die Zecken über Nacht bei 24 °C getrocknet. Nach dem Trocknen werden die Zecken 4 Woche lang bis zum Ende der Eiablage und Beginn des Larvenschlupfes bei 28 °C und 80 % Luftfeuchtigkeit gehalten.

Jede Testsubstanz wird in einer Konzentration von 500 ppm getestet. Die akarizide Wirkung zeigt sich entweder beim Weibchen als Mortalität oder Sterilität oder im Eigelege durch Blockierung der Embryogenese oder des Schlüpfaktes. Alle Substanzen werden gegen zwei verschiedene Zeckenstämme, den OPresistenten Stamm BIARRA und den Amidin-resistenten Stamm ULAM geprüft.

Verbindungen gemäss den Beispielen 1.2. zeigen gute Wirkung im obigen Test.

3.5. Frassgift-Wirkung auf Spodoptera littoralis-Larven ($L_1$)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvalen Stadium besetzt. Der Versuch wird bei 26 °C und ca. 50 % relativer Luftfeuchtigkeit durchgeführt. Nach 2 und 3 Tagen wird die Mortalität und nach 5 Tagen werden Entwicklungs- und Häutungsstörungen der angesetzten Larven bestimmt.

Verbindungen gemäss den Beispielen 1.2. zeigen gute Wirkung in diesem Test.

3.6. Frassgift-Wirkung auf Spodoptera littoralis- und Heliothis virescens-Larven ($L_3$)

Eingetopfte Sojapflanzen (Topfgrösse 10 cm Durchmesser) im 4-Blatt-Stadium werden mit wässrigen Wirkstoffemulsionen besprüht, die den Wirkstoff in einer Konzentration von 400 ppm enthalten.

Nach zwei Tagen wird jede behandelte Sojapflanze mit 10 Larven von Spodoptera littoralis oder Heliothis virescens im dritten larvalen Stadium besiedelt. Der Versuch wird bei 26 °C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2. zeigen 80-100%ige Wirkung (Mortalität).

3.7. Frassgift-Wirkung auf Plutella xylostella- und Crocidolomia binotalis-Larven ($L_2$)

Eingetopfte Chinakohlpflanzen (Topfgrösse 10 cm Durchmesser) im 4-Blatt-Stadium werden mit wässrigen Wirkstoffemulsionen besprüht, die den Wirkstoff in einer Konzentration von 400 ppm enthalten.

Nach zwei Tagen wird jede behandelte Chinakohlpflanze mit 10 Plutella xylostella- oder Crocidolomia binotalis-Larven im zweiten larvalen Stadium besiedelt. Der Versuch wird bei 26 °C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2. zeigen 80-100%ige Wirkung (Mortalität).

3.8. Kontaktwirkung gegen Nilaparvata lugens (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 40 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 6 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Beleuchtungsperiode von 16 Stunden durchgeführt.

Verbindungen gemäss den Beispielen 1.2. zeigen in diesem Test gute Wirkung.

3.9. Systemische Wirkung auf Nilaparvata lugens

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wird mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im $N_2$- bis $N_3$-Stadium besiedelt und mit einem Plastikzylinder abgedeckt. Der Versuch wird bei 26°C und ca. 60 % relativer Luftfeuchtigkeit mit einer Beleuchtungsperiode von 16 Stunden durchgeführt. Nach zwei und fünf Tagen wird auf die Anzahl der abgetöteten Testtiere, im Vergleich zu unbehandelten Kontrollen, bonitiert. Damit wird festgestellt, ob der über die Wurzel aufgenommen Wirkstoff, die Testtiere an den oberen Pflanzenteilen abtötet.

Verbindungen gemäss den Beispielen 1.2. zeigen im obigen Test 80 - 100%ige Wirkung (Mortalität) gegen Nilaparvata lugens.

3.10. Wirkung gegen Pflanzenschädigende Akarinen: Tetranychus cinnabarinus (OP-resistant)

Die Primärblätter von Phaseolus vulgaris-Pflanzen werden 24 Stunden vor dem Versuch auf akarizide Wirkung mit einem infestierten Blattstück aus einer Massenzucht von Tetranychus cinnabarinus belegt (die Resistenz bezieht sich auf die Verträglichkeit gegenüber Diazinon).

Die so behandelten infestierten Pflanzen werden mit einer Versuchslösung enthaltend 200 ppm der jeweils zu prüfenden Verbindung bis zur Tropfnässe besprüht. Nach 48 Stunden und wiederum nach 7 Tagen werden Imagines und Larven (alle beweglichen Stadien) unter dem Binokular auf lebende und tote Individuen ausgewertet. Man verwendet pro Konzentration und pro Testspezies eine Pflanze. Während des Versuchsverlaufs stehen die Pflanzen in Gewächshauskabinen bei 25°C.

Verbindungen gemäss den Beispielen 1.2. zeigen in diesem Versuch gute Wirkung gegen Tetranychus cinnabarinus.

3.11. Ovizide Wirkung auf Tetranychus cinnabarinus (OP-resistent)

Eingetopfte Phaseolus vulgaris-Pflanzen im Primärblatt-Stadium werden jeweils zweimal mit 30 Weibchen von Tetranychus cinnabarinus besiedelt. Nach 24stündiger Eiablage werden die Weibchen mit einer Saugpumpe (Wasserstrahlpumpe) von den Pflanzen entfernt, so dass auf diesen nur noch die abgelegten Milbeneier verbleiben.

Die mit den Milbeneiern infestierten Pflanzen werden dann mit einer wässrigen Emulsion enthaltend 200 ppm der zu prüfenden Verbindung bis zur Tropfnässe besprüht und 5 Tage bei 25°C und etwa 50 % relativer Luftfeuchtigkeit gehalten. Nach dieser Zeit wird die prozentuale Mortalität der Eier und geschlüpften Larven durch Auszählen bestimmt.

Verbindungen gemäss den Beispielen 1.2. zeigen gute Wirkung in obigem Test.

3.12. Pflanzenmitizide Blattpenetrations-Wirkung auf Tetranychus cinnabarinus

Für den Versuch werden eingetopfte Buschbohnenpflanzen im Primärblatt-Stadium, die mit Tetranychus cinnabarinus infestiert sind, verwendet. Die Besiedlung mit den Milben erfolgt 1 Tag vor der Wirkstoffapplikation.

Die Blattoberseiten der mit diesen Milben infestierten Versuchspflanzen werden mit einer Emulsionszubereitung enthaltend 400 ppm des zu prüfenden Wirkstoffes besprüht. Nach dem Antrocknen des Sprühbelages wird jeweils der Rand der Blattoberseite einer Anzahl befallener Blätter mit einem Wulst aus

zähflüssigem Leim (Raupenleim) abgegrenzt, um ein Ueberlaufen der Milben von der Blattunterseite auf die Blattoberseite zu verhindern.

Die behandelten Pflanzen werden dann im Gewächshaus bei einer Temperatur von 25°C bis 27°C und einer relativen Luftfeuchtigkeit von ca. 50 % gehalten. Sechs Tage nach der Wirkstoffapplikation wird festgestellt, ob eine translaminare Wirkung, d.h. Blattpenetration des Wirkstoffs von der Blattoberseite zur Blattunterseite, eingetreten war, und zwar durch Bestimmung der prozentualen Mortalität der Eier und larvalen sowie adulten Stadien.

Verbindungen gemäss den Beispielen 1.2. zeigen gute Wirkung in obigem Test.

3.13. Wirkung gegen Panonychus ulmi (OP und Carb. resistent)

Eingetopfte Apfelsämlinge mit etwa 20-30 Blättern werden mit jeweils 60 adulten Weibchen von Panonychus ulmi besiedelt. Nach sieben Tagen werden die infestierten Pflanzen mit einer wässrigen Emulsion enthaltend 400 ppm der zu prüfenden Verbindung bis zur Tropfnässe besprüht. Die behandelten Pflanzen werden dann während weiterer 14 Tage bei 25°C und etwa 50 % relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Verbindungen gemäss den Beispielen 1.2. zeigen gute Wirkung in obigem Test.

3.14. Kontaktwirkung auf Myzus persicae

Etwa 4 bis 5 Tage alte, in Wasser angezogene Erbsenkeimlinge (Pisum sativum) werden vor Versuchsbeginn jeweils mit ca. 200 Individuen der Spezies Myzus persicae besiedelt. Die so behandelten Pflanzen werden 24 Stunden später mit einer wässrigen Suspension enthaltend 100 ppm der zu prüfenden Verbindung bis zur Tropfnässe direkt besprüht. Man verwendet pro Testsubstanz zwei Pflanzen. Eine Auswertung der erzielten Abtötungsrate erfolgt 3 und 5 Tage nach Applikation. Der Versuch wird bei ca. 21°C und etwa 60 % relativer Luftfeuchtigkeit durchgeführt.

Verbindungen gemäss den Beispielen 1.2. zeigen gute Wirkung in diesem Test.

3.15. Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen ($N_2$ bis $N_3$) der Testtiere. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens einmal nachgegossen werden muss, gehalten. Der Versuch wird bei einer Temperatur von ca. 23°C, bei 55 % relativer Luftfeuchtigkeit und mit einer Belichtungsperiode von 16 Stunden durchgeführt.

Verbindungen gemäss den Beispielen 1.2. zeigen in diesem Test gute Wirkung.

3.16. Wirkung gegen Bodeninsekten (Diabrotica balteata)

Es werden 350 ml Erde (bestehend aus 95 Vol-% Sand und 5 Vol-% Torf) mit 150 ml einer wässrigen Emulsionszubereitung vermischt, die den zu prüfenden Wirkstoff in einer Konzentration von 400 ppm enthält. Dann werden Plastikbecher, die einen oberen Durchmesser von ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 24°C und einer relativen Luftfeuchtigkeit von ca. 50 % gehalten. Sechs Tage nach dem Ansatz wird die in den Bechern enthaltene Erde abgesiebt und die zurückbleibenden Larven werden auf ihre Mortalität geprüft.

Verbindungen gemäss den Beispielen 1.2. zeigen gute Wirkung im obigen Test.

3.17 Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit Testtieren besiedelten Pflanzen gestülpt, um ein Abwandern der Käfer zu verhindern. Die behandelten Pflanzen werden bei 25 °C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss den Beispielen 1.2. zeigen gute Wirkung in diesem Test.

3.18. Wirkung gegen Dermanyssus gallinae

In einem nach oben offenen Glasbehälter werden 2 bis 3 ml einer 100 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben durch Auszählen der toten Individuen ermittelt und in Prozenten angegeben.

Verbindungen gemäss den Beispielen 1.2. zeigen 80-100%ige Wirkung (Mortalität).

3.19. Wirkung gegen Blattella germanica

In eine Petri-Schale von 10 cm Durchmesser wird so viel einer 0,1%igen acetonischen Lösung des Wirkstoffes gegeben, dass die Menge einer Aufwandmenge von 2 $g/m^2$ entspricht. Wenn das Lösungsmittel verdunstet ist, werden 20 Blattella germanica Nymphen (letztes Nymphenstadium) in die so vorbereitete Schale gegeben und 2 Stunden lang der Wirkung der Testsubstanz ausgesetzt. Dann werden die Nymphen mit $CO_2$ narkotisiert, in eine frische Petri-Schale gebracht und im Dunkeln bei 25 °C und 50 bis 70 % Luftfeuchtigkeit gehalten. Nach 48 Stunden wird die insektizide Wirkung durch Ermittlung der Abtötungsrate bestimmt.

Verbindungen gemäss den Beispielen 1.2. zeigen gute Wirkung im obigen Test.

## Patentansprüche

**1.** Verbindungen der Formel I

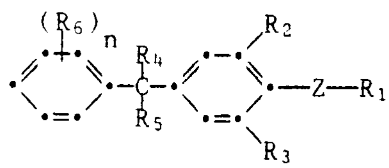

(I),

worin

$R_1$ für $C_1$-$C_8$-Alkyl, ein- oder mehrfach durch Halogen und/oder $C_1$-$C_6$-Alkoxy substituiertes $C_1$-$C_8$-Alkyl, $C_3$-$C_8$-Cycloalkyl, ein- oder mehrfach durch $C_1$-$C_3$-Alkyl substituiertes $C_3$-$C_8$-Cycloalkyl oder $C_3$-$C_8$-Cycloalkyl-$C_1$-$C_4$-alkyl;

$R_2$ und $R_3$ je für $C_1$-$C_6$-Alkyl, $C_5$-$C_6$-Cycloalkyl oder $C_5$-$C_6$-Cycloalkenyl;

$R_4$ und $R_5$ je für Wasserstoff oder $C_1$-$C_4$-Alkyl;

$R_6$ je für Halogen, $C_1$-$C_4$-Alkyl, $C_1$-$C_4$-Alkoxy, $C_1$-$C_4$-Halogenalkyl, $C_1$-$C_4$-Halogenalkoxy oder für eine

$-(-CH=CH-)_2-$, $-(-CH_2-)_3-$

oder $-(-CH_2-)_4-$

Brücke in 2,3- oder 3,4-Stellung;
n für 0, 1 oder 2;
Z für -NH-CS-NH-, -N=C($SR_7$)-NH- oder -N=C=N- und
$R_7$ für $C_1$-$C_{10}$-Alkyl oder $C_3$-$C_5$-Alkenyl
stehen, sowie ihre Salze mit organischen oder anorganischen Säuren.

**2.** Verbindungen der Formel I gemäss Anspruch 1, worin $R_1$ für $C_1$-$C_6$-Alkyl, ein- oder mehrfach durch Halogen und/oder $C_1$-$C_3$-Alkoxy substituiertes $C_1$-$C_6$-Alkyl oder $C_5$-$C_6$-Cycloalkyl; $R_2$ und $R_3$ je für $C_1$-$C_5$-Alkyl oder $C_5$-$C_6$-Cycloalkyl; $R_4$ und $R_5$ je für Wasserstoff oder $C_1$-$C_3$-Alkyl, $R_6$ für Halogen, Methyl, Methoxy, Trifluormethyl oder Trifluormethoxy; n für 0 oder 1; Z für -NH-CS-NH-, -N=C($SR_7$)-NH-oder -N=C=N-; und $R_7$ für $C_1$-$C_6$-Alkyl oder $C_3$-$C_4$-Alkenyl stehen.

**3.** Verbindungen der Formel I gemäss Anspruch 2, worin $R_1$ für $C_1$-$C_5$-Alkyl oder Cyclopentyl; $R_2$ und $R_3$ je für $C_3$-$C_5$-Alkyl oder $C_5$-$C_6$-Cycloalkyl; $R_4$ und $R_5$ je für Wasserstoff oder Methyl; $R_6$ für Fluor, Chlor oder Methyl; n für 0 oder 1; Z für -NH-CS-NH-, -N=C($SR_7$)-NH- oder -N=C=N-; und $R_7$ für $C_1$-$C_3$-Alkyl oder Allyl stehen.

**4.** Verbindungen der Formel I gemäss Anspruch 3, worin $R_1$ für $C_3$-$C_5$-Alkyl oder Cyclopentyl; $R_2$ und $R_3$ je für $C_3$-$C_5$-Alkyl; $R_4$ und $R_5$ je für Wasserstoff oder Methyl; n für 0; und Z für -NH-CS-NH-stehen.

**5.** Verbindungen der Formel I gemäss Anspruch 3, worin $R_1$ für $C_3$-$C_5$-Alkyl oder Cyclopentyl, $R_2$ und $R_3$ je für $C_3$-$C_5$-Alkyl; $R_4$ und $R_5$ je für Wasserstoff oder Methyl; n für 0; Z für -N=C($SR_7$)-NH; und $R_7$ für $C_1$-$C_2$-Alkyl stehen.

**6.** Verbindungen der Formel I gemäss Anspruch 3, worin $R_1$ für $C_3$-$C_5$-Alkyl oder Cyclopentyl, $R_2$ und $R_3$ je für $C_3$-$C_5$-Alkyl; $R_4$ und $R_5$ je für Wasserstoff oder Methyl; n für 0; und Z für -N=C=N-stehen.

**7.** Die Verbindungen gemäss Anspruch 1 oder 4 oder Formeln

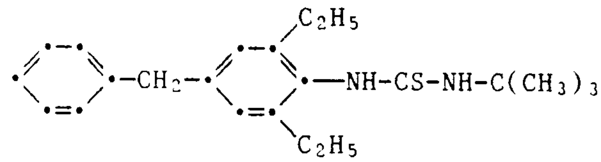,

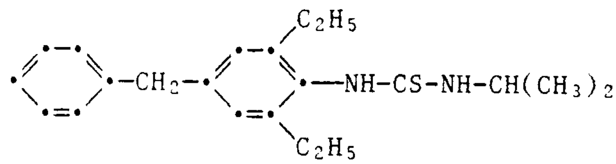,

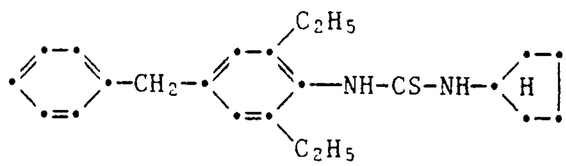,

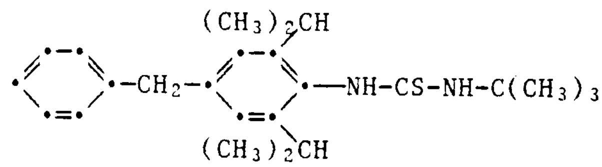,

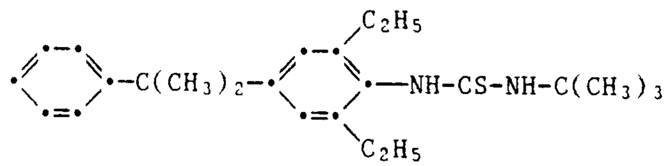,

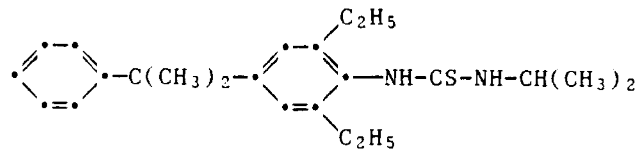
,

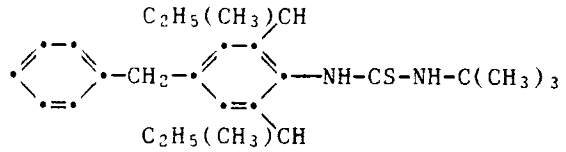
,

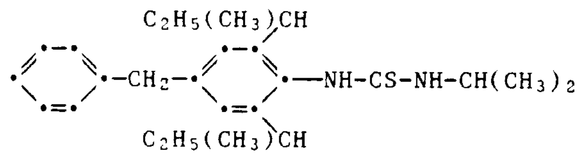
,

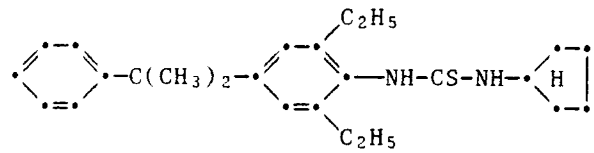
,

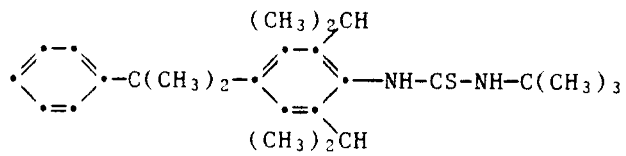
,

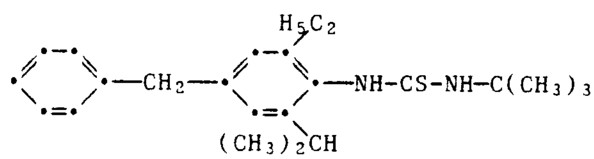
,

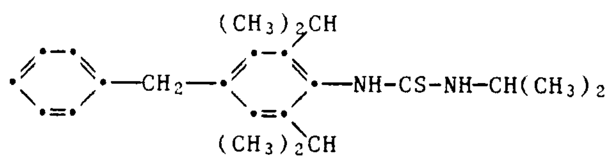
,

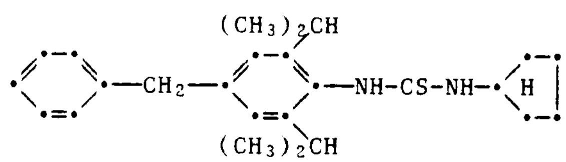
,

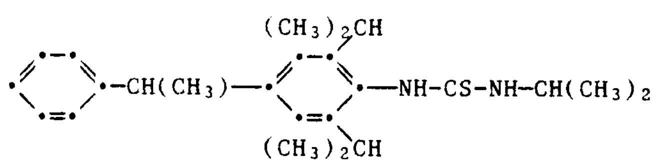
,

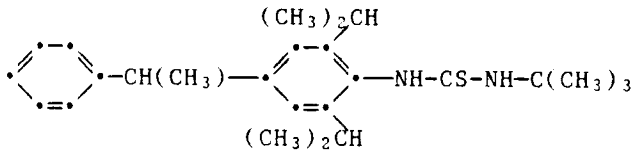
,

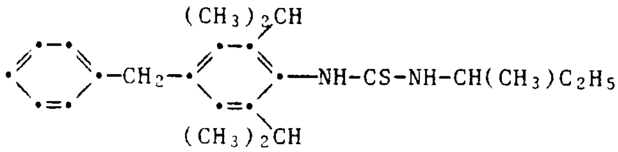
,

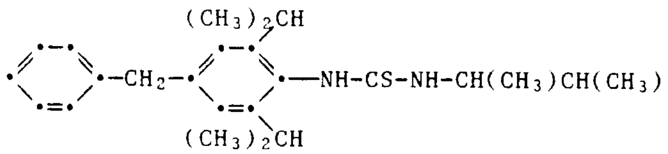
,

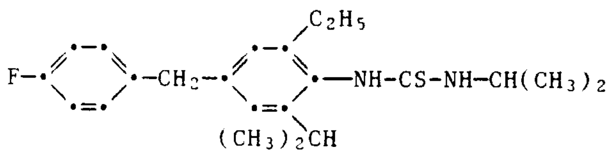
,

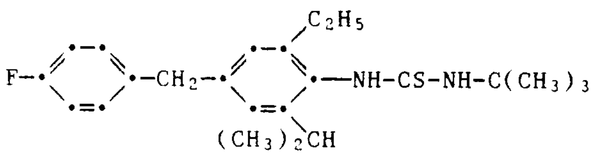
,

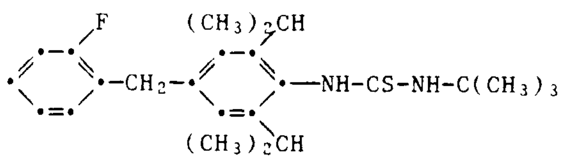
,

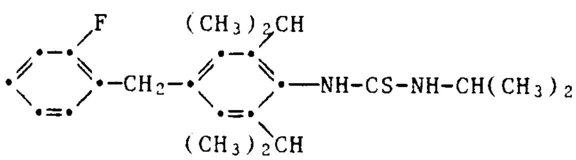
,

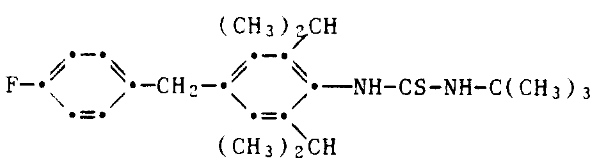
,

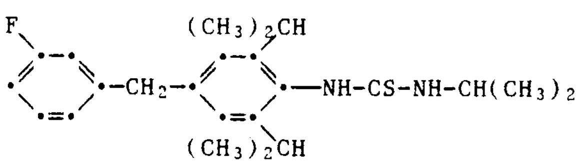
,

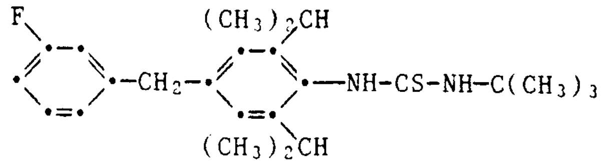 ,

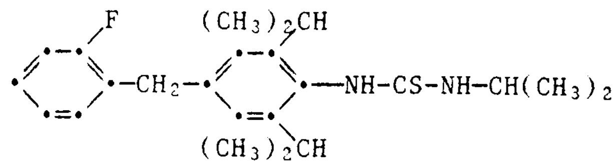 und

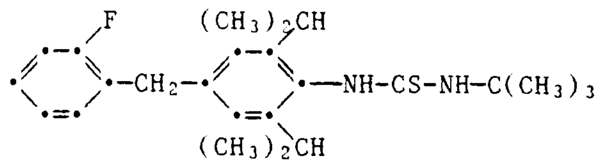 .

**8.** Verbindungen gemäss Anspruch 1 oder 5 der Formeln

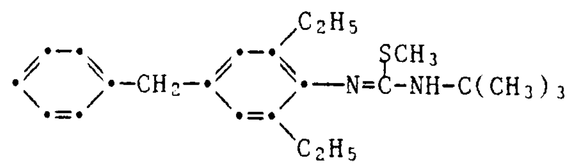 ,

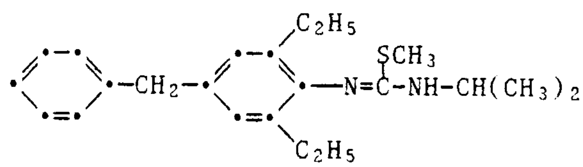 ,

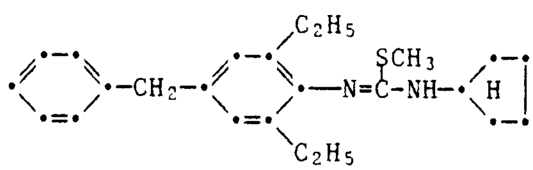 ,

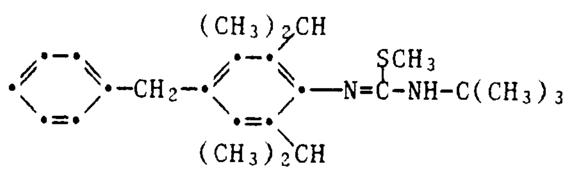 ,

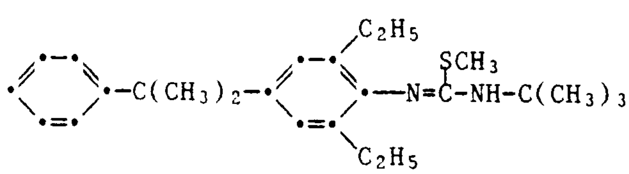 ,

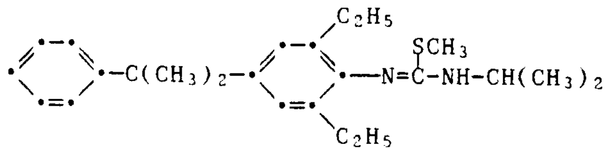,

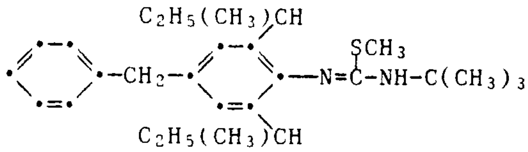,

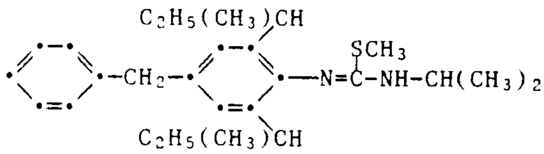,

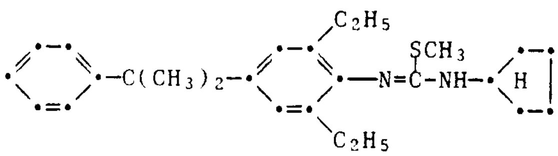,

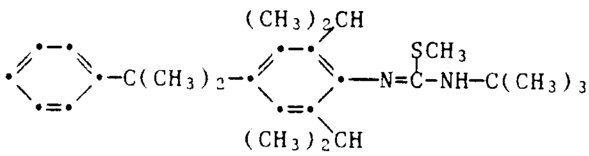,

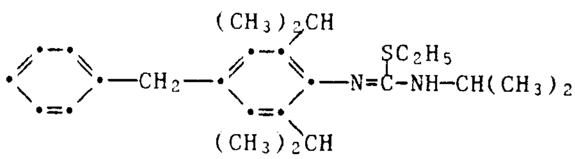,

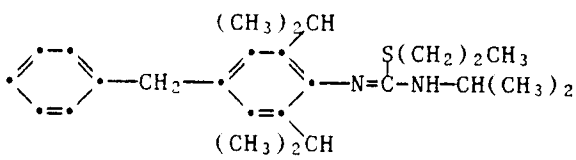,

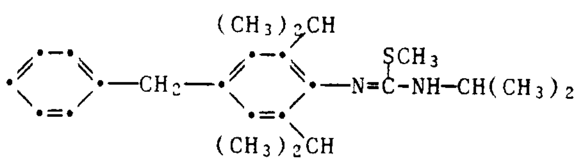,

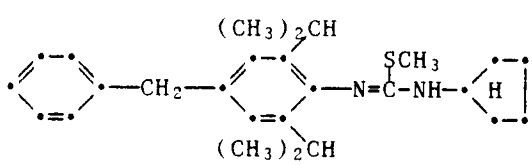,

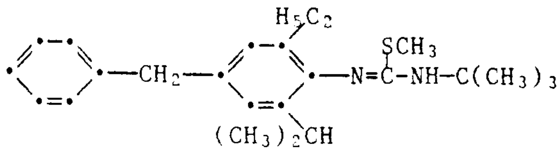
,

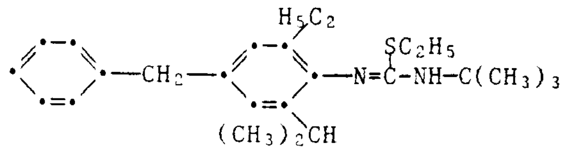
,

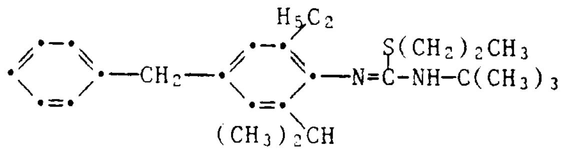
,

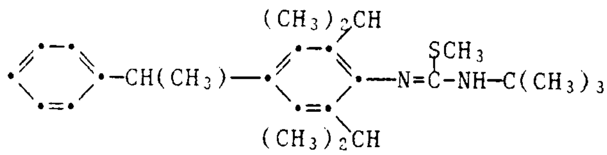
,

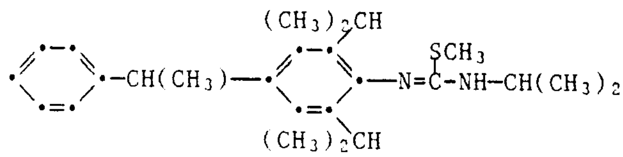
,

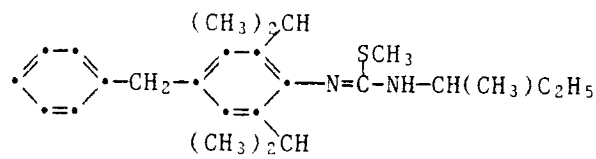
,

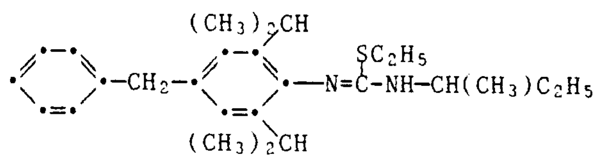
,

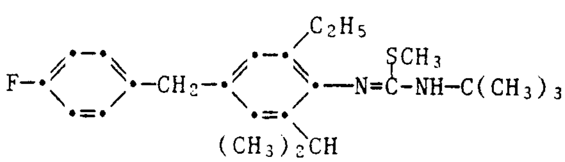
,

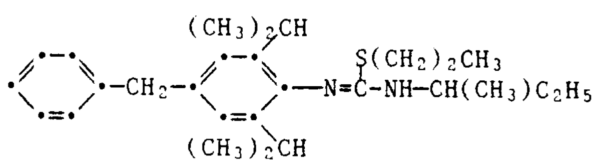
,

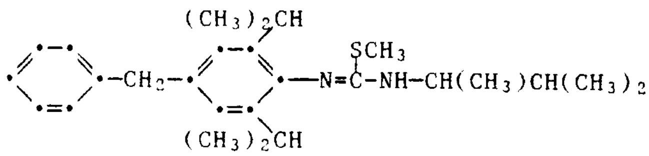,

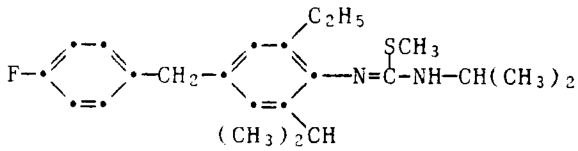,

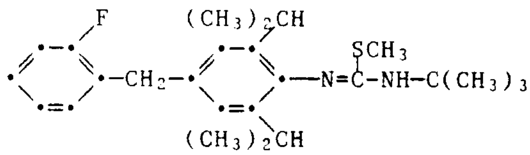,

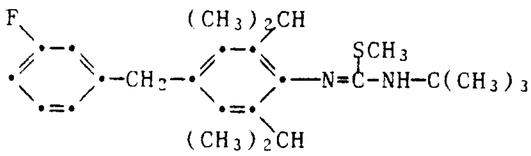,

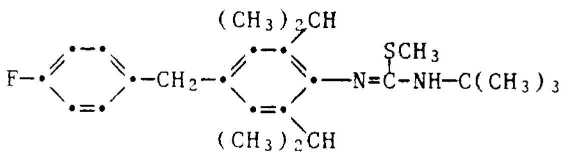,

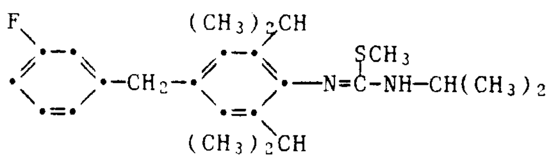,

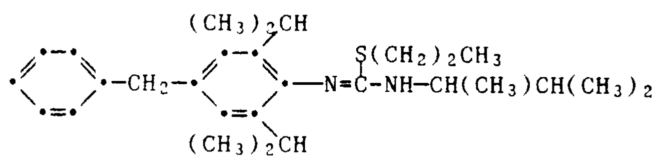,

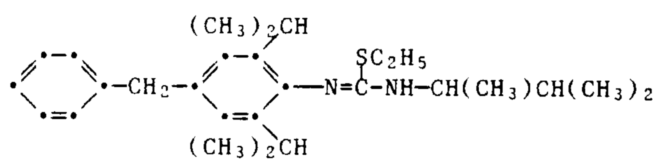,

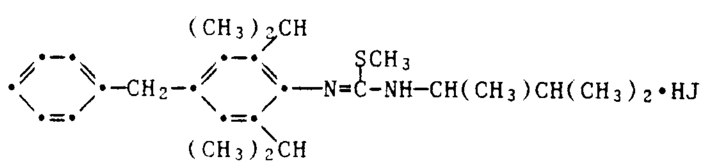,

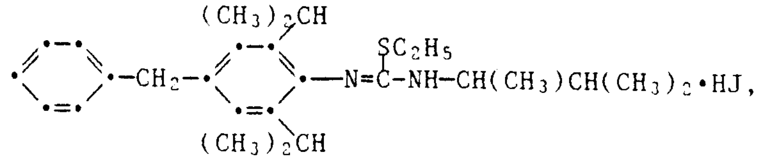

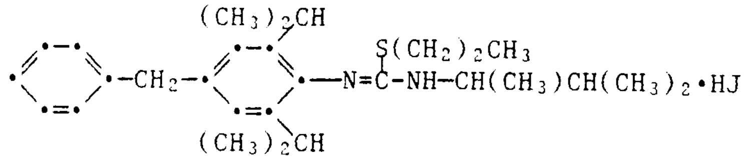

und

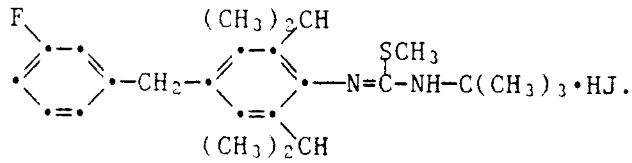

**9.** Die Verbindungen gemäss Anspruch 1 oder 6 der Formeln

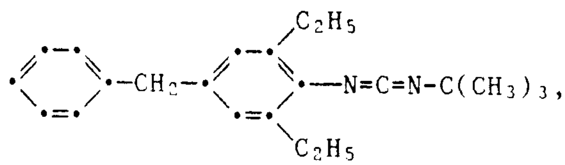

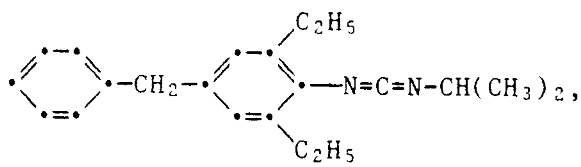

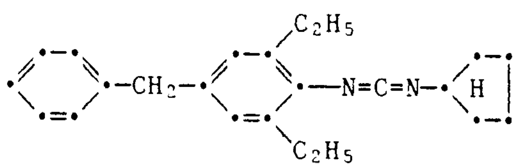

,

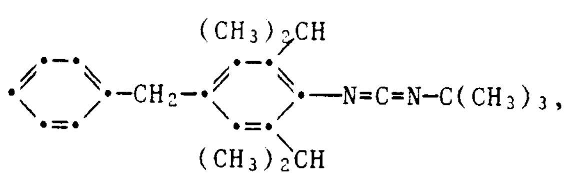

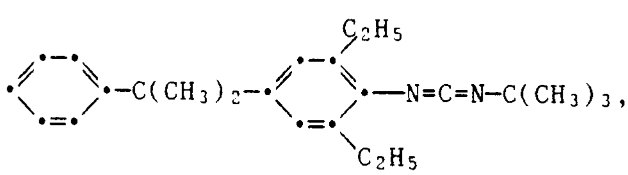

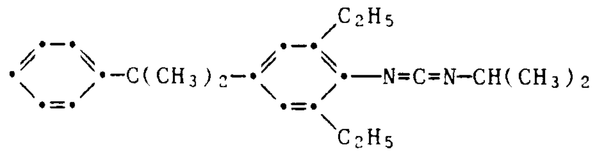
,

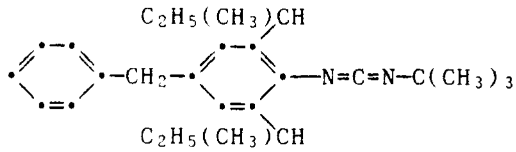
,

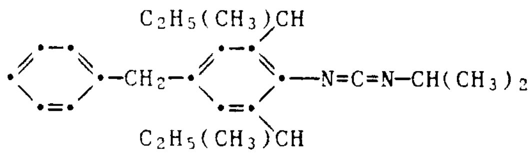
,

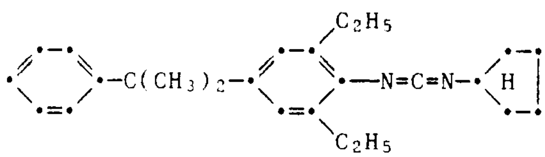
,

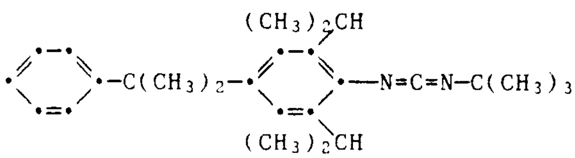
,

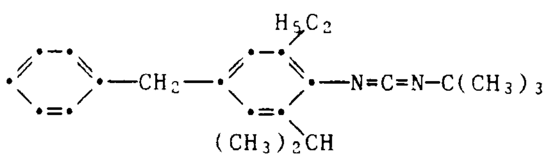
,

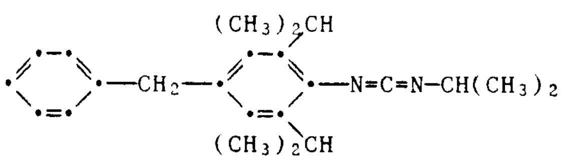
,

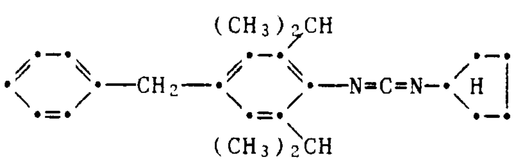
,

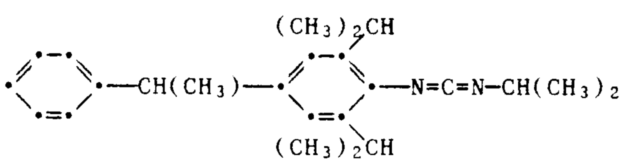
,

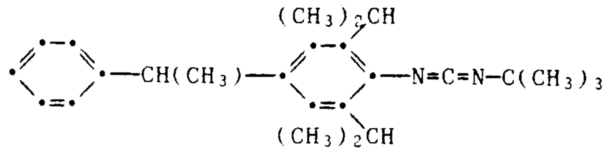,

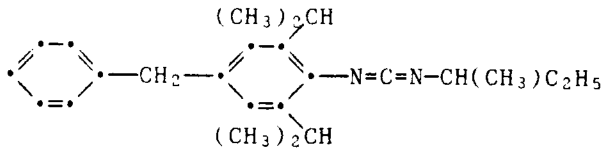,

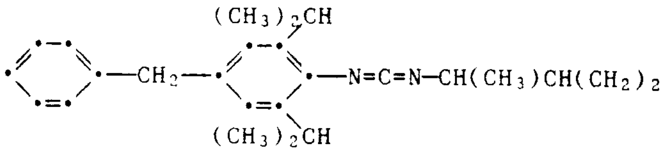,

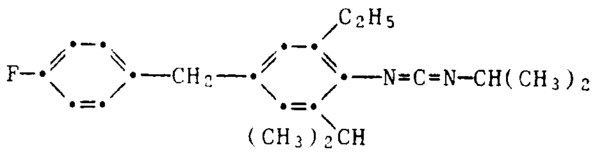,

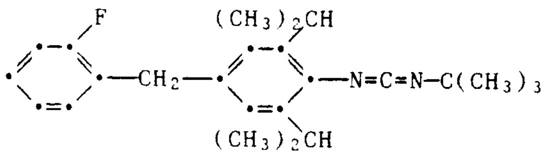,

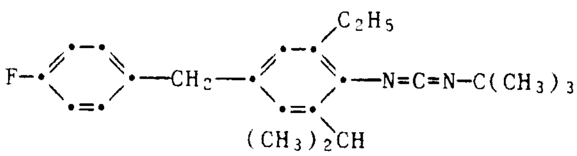,

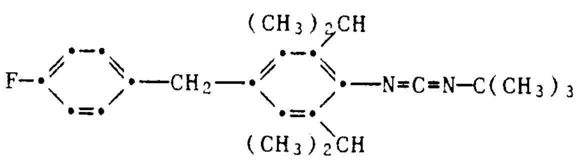,

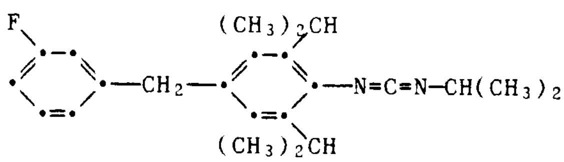 und

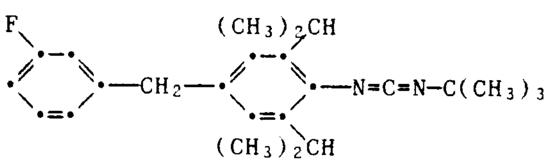.

**10.** Verfahren zur Herstellung einer Verbindung der Formel I

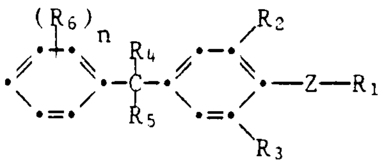 (I),

worin
$R_1$ für $C_1$-$C_8$-Alkyl, ein- oder mehrfach durch Halogen und/oder $C_1$-$C_6$-Alkoxy substituiertes $C_1$-$C_8$-Alkyl, $C_3$-$C_8$-Cycloalkyl, ein- oder mehrfach durch $C_1$-$C_3$-Alkyl substituiertes $C_3$-$C_8$-Cycloalkyl oder $C_3$-$C_8$-Cycloalkyl-$C_1$-$C_4$-alkyl;
$R_2$ und $R_3$ je für $C_1$-$C_6$-Alkyl, $C_5$-$C_6$-Cycloalkyl oder $C_5$-$C_6$-Cycloalkenyl;
$R_4$ und $R_5$ je für Wasserstoff oder $C_1$-$C_4$-Alkyl;
$R_6$ je für Halogen, $C_1$-$C_4$-Alkyl, $C_1$-$C_4$-Alkoxy, $C_1$-$C_4$-Halogenalkyl, $C_1$-$C_2$-Halogenalkoxy oder für eine

$-(-CH=CH-)_2-$, $-(-CH_2-)_3-$

oder $-(-CH_2-)_4-$

Brücke in 2,3- oder 3,4-Stellung;
n für 0, 1 oder 2;
Z für -NH-CS-NH-, -N=C($SR_7$)-NH- oder -N=C=N- und
$R_7$ für $C_1$-$C_{10}$-Alkyl oder $C_3$-$C_5$-Alkenyl
stehen, dadurch gekennzeichnet, dass man
A) ein Isothiocyanat der Formel II

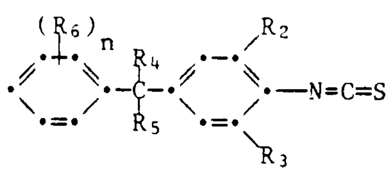 (II)

mit einem Amin der Formel III

$H_2N—R_1$ (III)

in einem organischen Lösungs- oder Verdünnungsmittel bei Normaldruck und einer Temperatur von 0 bis +150 °C zum Thioharnstoff umsetzt und gegebenenfalls
B) den erhaltenen Thioharnstoff mit einer Verbindung der Formel IV

$X—R_7$ (IV)

in einem inerten organischen Lösungsmittel unter leicht erhöhtem oder normalem Druck und bei einer Temperatur von +10 bis 250 °C zum Isothioharnstoff umsetzt oder
C) den erhaltenen Thioharnstoff durch Abspalten von Schwefelwasserstoff in einem aprotischen organischen Lösungs- oder Verdünnungsmittel unter Normaldruck und bei einer Temperatur von 0 bis +150 °C in das Carbodiimid überführt, wobei $R_1$, $R_2$, $R_3$, $R_4$, $R_5$, $R_6$, $R_7$ und n die angegebene Bedeutung haben und X für eine Abgangsgruppe steht.

**11.** Schädlingsbekämpfungsmittel, welches als aktive Komponente mindestens eine Verbindung der Formel I

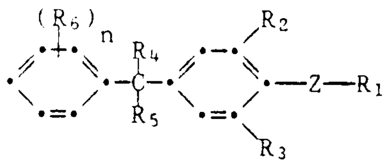

(I)

enthält, worin
$R_1$ für $C_1$-$C_8$-Alkyl, ein- oder mehrfach durch Halogen und/oder $C_1$-$C_6$-Alkoxy substituiertes $C_1$-$C_8$-Alkyl, $C_3$-$C_8$-Cycloalkyl oder $C_3$-$C_8$-Cycloalkyl-$C_1$-$C_4$-alkyl;
$R_2$ und $R_3$ je für $C_1$-$C_6$-Alkyl, $C_5$-$C_6$-Cycloalkyl oder $C_5$-$C_6$-Cycloalkenyl;
$R_4$ und $R_5$ je für Wasserstoff oder $C_1$-$C_4$-Alkyl;
$R_6$ je für Halogen, $C_1$-$C_4$-Alkyl, $C_1$-$C_4$-Alkoxy, $C_1$-$C_4$-Halogenalkyl, $C_1$-$C_4$-Halogenalkoxy oder für eine

$$-(-CH=CH-)_2-,\quad -(-CH_2-)_3-$$

$$\text{oder } -(-CH_2-)_4-$$

Brücke in 2,3- oder 3,4-Stellung;
n für 0, 1 oder 2;
Z für -NH-CS-NH-, -N=C($SR_7$)-NH- oder -N=C=N- und
$R_7$ für $C_1$-$C_{10}$-Alkyl oder $C_3$-$C_5$-Alkenyl
stehen, oder eines ihrer Salze mit einer organischen oder anorganischen Säure zusammen mit geeigneten Trägerstoffen und/oder Zuschlagstoffen.

**12.** Schädlingsbekämpfungsmittel gemäss Anspruch 11, welches als aktive Komponente mindestens eine Verbindung der Formel I enthält, worin $R_1$ für $C_1$-$C_6$-Alkyl, ein- oder mehrfach durch Halogen und/oder $C_1$-$C_3$-Alkoxy substituiertes $C_1$-$C_6$-Alkyl oder $C_5$-$C_6$-Cycloalkyl; $R_2$ und $R_3$ je für $C_1$-$C_5$-Alkyl oder $C_5$-$C_6$-Cycloalkyl; $R_4$ und $R_5$ je für Wasserstoff oder $C_1$-$C_3$-Alkyl, $R_6$ für Halogen, Methyl, Methoxy, Trifluormethyl oder Trifluormethoxy; n für 0 oder 1; Z für -NH-CS-NH-, -N=C($SR_7$)-NH-oder -N=C=N-; und $R_7$ für $C_1$-$C_6$-Alkyl oder $C_3$-$C_4$-Alkenyl stehen.

**13.** Schädlingsbekämpfungsmittel gemäss Anspruch 12, welches als aktive Komponente mindestens eine Verbindung der Formel I enthält, worin $R_1$ für $C_1$-$C_5$-Alkyl oder Cyclopentyl; $R_2$ und $R_3$ je für $C_3$-$C_5$-Alkyl oder $C_5$-$C_6$-Cycloalkyl; $R_4$ und $R_5$ je für Wasserstoff oder Methyl; $R_6$ für Fluor, Chlor oder Methyl; n für 0 oder 1; Z für -NH-CS-NH-, -N=C($SR_7$)-NH- oder -N=C=N-; und $R_7$ für $C_1$-$C_3$-Alkyl oder Allyl stehen.

**14.** Schädlingsbekämpfungsmittel gemäss Anspruch 13, welches als aktive Komponente mindestens eine Verbindung der Formel I enthält, worin $R_1$ für $C_3$-$C_5$-Alkyl oder Cyclopentyl; $R_2$ und $R_3$ je für $C_3$-$C_5$-Alkyl; $R_4$ und $R_5$ je für Wasserstoff oder Methyl; n für 0; und Z für -NH-CS-NH-stehen.

**15.** Schädlingsbekämpfungsmittel gemäss Anspruch 13, welches als aktive Komponente mindestens eine Verbindung der Formel I enthält, worin $R_1$ für $C_3$-$C_5$-Alkyl oder Cyclopentyl, $R_2$ und $R_3$ je für $C_3$-$C_5$-Alkyl; $R_4$ und $R_5$ je für Wasserstoff oder Methyl; n für 0; Z für -N=C($SR_7$)-NH; und $R_7$ für $C_1$-$C_2$-Alkyl stehen.

**16.** Schädlingsbekämpfungsmittel gemäss Anspruch 13, welches als aktive Komponente mindestens eine Verbindung der Formel I enthält, worin $R_1$ für $C_3$-$C_5$-Alkyl oder Cyclopentyl, $R_2$ und $R_3$ je für $C_3$-$C_5$-Alkyl; $R_4$ und $R_5$ je für Wasserstoff oder Methyl; n für 0; Z für -N=C=N- stehen.

**17.** Verwendung einer Verbindung der Formel I

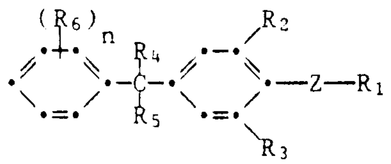 (I),

worin
$R_1$ für $C_1$-$C_8$-Alkyl, ein- oder mehrfach durch Halogen und/oder $C_1$-$C_6$-Alkoxy substituiertes $C_1$-$C_8$-Alkyl, $C_3$-$C_8$-Cycloalkyl, ein- oder mehrfach durch $C_1$-$C_3$-Alkyl substituiertes $C_3$-$C_8$-Cycloalkyl oder $C_3$-$C_8$-Cycloalkyl-$C_1$-$C_4$-alkyl;
$R_2$ und $R_3$ je für $C_1$-$C_6$-Alkyl, $C_5$-$C_6$-Cycloalkyl oder $C_5$-$C_6$-Cycloalkenyl;
$R_4$ und $R_5$ je für Wasserstoff oder $C_1$-$C_4$-Alkyl;
$R_6$ je für Halogen, $C_1$-$C_4$-Alkyl, $C_1$-$C_4$-Alkoxy, $C_1$-$C_4$-Halogenalkyl, $C_1$-$C_4$-Halogenalkoxy oder für eine

$$-(-CH=CH-)_2-,\quad -(-CH_2-)_3-$$

$$\text{oder } -(-CH_2-)_4-$$

Brücke in 2,3- oder 3,4-Stellung;
n für 0, 1 oder 2;
Z für -NH-CS-NH-, -N = C($SR_7$)-NH- oder -N = C = N- und
$R_7$ für $C_1$-$C_{10}$-Alkyl oder $C_3$-$C_5$-Alkenyl
stehen, oder eines ihrer Salze mit einer organischen oder anorganischen Säure zur Bekämpfung von Schädlingen an Tieren und Pflanzen.

**18.** Verwendung gemäss Anspruch 17 einer Verbindung der Formel I zur Bekämpfung von Insekten und Arachniden.

**19.** Verfahren zur Bekämpfung von Schädlingen an Tieren und Pflanzen, dadurch gekennzeichnet, dass man die Schädlinge in ihren verschiedenen Entwicklungsstadien mit einer Verbindung der Formel I

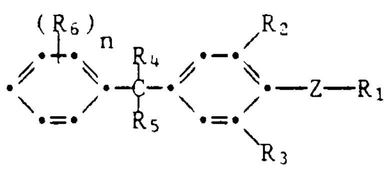 (I),

worin
$R_1$ für $C_1$-$C_8$-Alkyl, ein- oder mehrfach durch Halogen und/oder $C_1$-$C_6$-Alkoxy substituiertes $C_1$-$C_8$-Alkyl, $C_3$-$C_8$-Cycloalkyl, ein- oder mehrfach durch $C_1$-$C_3$-Alkyl substituiertes $C_3$-$C_8$-Cycloalkyl oder $C_3$-$C_8$-Cycloalkyl-$C_1$-$C_4$-alkyl;
$R_2$ und $R_3$ je für $C_1$-$C_6$-Alkyl, $C_5$-$C_6$-Cycloalkyl oder $C_5$-$C_6$-Cycloalkenyl;
$R_4$ und $R_5$ je für Wasserstoff oder $C_1$-$C_4$-Alkyl;
$R_6$ je für Halogen, $C_1$-$C_4$-Alkyl, $C_1$-$C_4$-Alkoxy, $C_1$-$C_4$-Halogenalkyl, $C_1$-$C_4$-Halogenalkoxy oder für eine

$$-(-CH=CH-)_2-,\quad -(-CH_2-)_3-$$

$$\text{oder } -(-CH_2-)_4-$$

Brücke in 2,3- oder 3,4-Stellung;
n für 0, 1 oder 2;
Z für -NH-CS-NH-, -N = C($SR_7$)-NH- oder -N = C = N- und
$R_7$ für $C_1$-$C_{10}$-Alkyl oder $C_3$-$C_5$-Alkenyl
stehen oder einem ihrer Salze mit einer organischen oder anorganischen Säure in Kontakt bringt.

**20.** Verbindungen der Formel II′

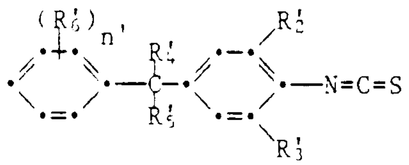

(II′),

worin $R_2^1$ und $R_3^1$ für $C_3$-$C_5$-Alkyl oder $C_5$-$C_6$-Cycloalkyl; $R_4^1$ und $R_5^1$ je für Wasserstoff oder Methyl; $R_6^1$ für Fluor, Chlor oder Methyl; und n′ für 0 oder 1 stehen.

**21.** Verbindungen der Formel II′ gemäss Anspruch 20, worin $R_2^1$ und $R_3^1$ je für $C_3$-$C_5$-Alkyl; $R_4^1$ und $R_5^1$ je für Wasserstoff oder Methyl; und n′ für 0 stehen.

**22.** Die Verbindungen gemäss Anspruch 20 oder 21 der Formeln

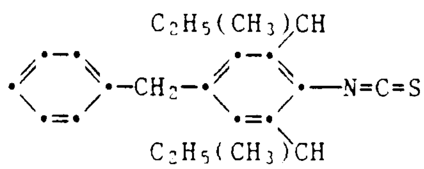

,

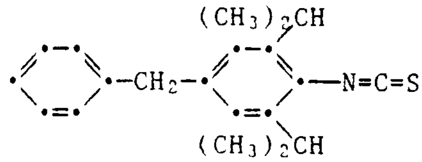
,

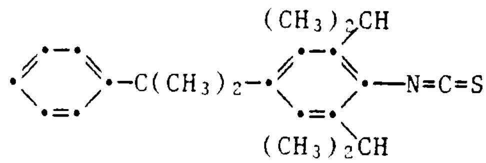
,

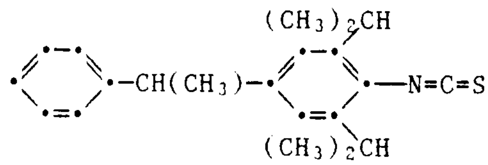
,

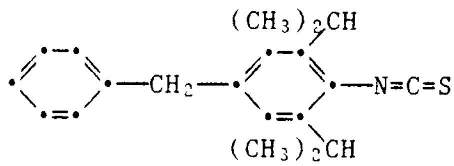
,

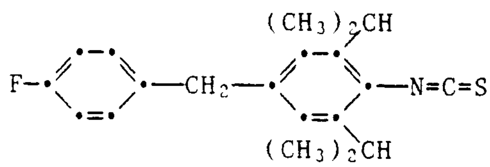
,

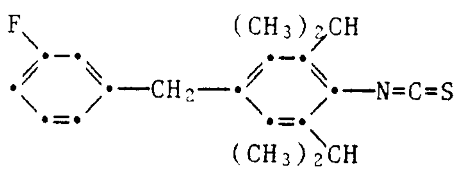
und

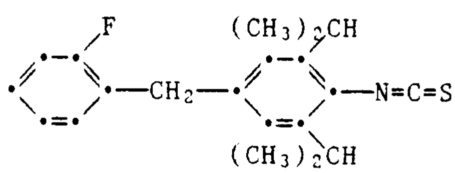
.

**23.** Verbindungen der Formel V′

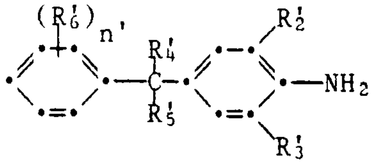
(V'),

worin $R_2^1$ und $R_3^1$ je für $C_3$-$C_5$-Alkyl oder $C_5$-$C_6$-Cycloalkyl; $R_4^1$ und $R_5^1$ je für Wasserstoff oder Methyl; $R_6^1$ für Fluor, Chlor oder Methyl; und n′ für 0 oder 1 stehen.

**24.** Verbindungen der Formel V′ gemäss Anspruch 23, worin $R_2^1$ und $R_3^1$ je für $C_3$-$C_5$-Alkyl; $R_4^1$ und $R_5^1$ je für

Wasserstoff oder Methyl; und n′ für 0 oder 1 stehen.

**25.** Die Verbindungen gemäss Anspruch 23 oder 24 der Formeln

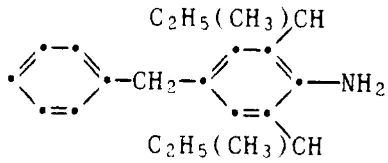
,

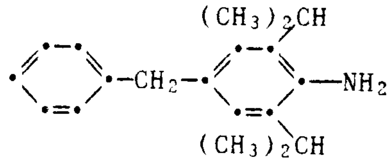
,

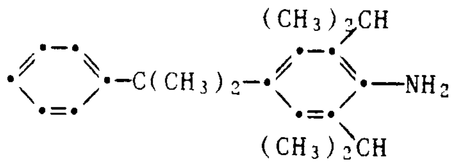
,

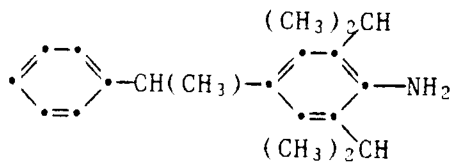
,

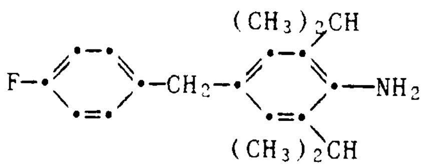
,

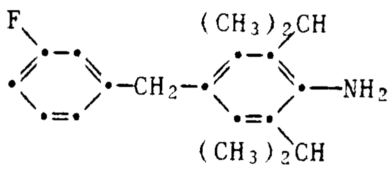
und

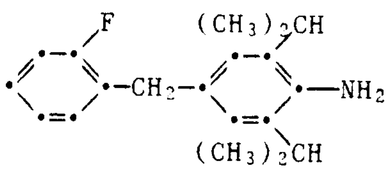
.

## Claims

**1.** A compound of formula I

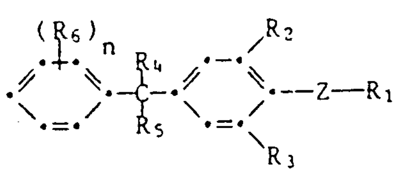
(I)

in which $R_1$ is $C_1$-$C_8$alkyl, $C_1$-$C_8$alkyl mono- or polysubstituted by halogen and/or by $C_1$-$C_6$alkoxy, $C_3$-$C_8$cycloalkyl, $C_3$-$C_8$cycloalkyl mono- or poly-substituted by $C_1$-$C_3$alkyl, or is $C_3$-$C_8$cycloalkyl-$C_1$-$C_4$alkyl; each of $R_2$ and $R_3$ is $C_1$-$C_6$alkyl, $C_5$-$C_6$cycloalkyl or $C_5$-$C_6$cycloalkenyl; each of $R_4$ and $R_5$ is hydrogen or $C_1$-$C_4$alkyl; each $R_6$ is halogen, $C_1$-$C_4$alkyl, $C_1$-$C_4$alkoxy, $C_1$-$C_4$haloalkyl, $C_1$-$C_4$haloalkoxy or a

$$-(CH=CH)_2-,\ -(CH_2)_3-\ \text{or}$$

$$-(CH_2)_4-$$

bridge in the 2,3- or 3,4-position; n is 0, 1 or 2; Z is -NH-CS-NH-, -N=C($SR_7$)-NH- or -N=C=N-, and $R_7$ is $C_1$-$C_{10}$alkyl or $C_3$-$C_5$alkenyl, or a salt thereof with an organic or inorganic acid.

**2.** A compound of formula I according to claim 1, in which $R_1$ is $C_1$-$C_6$alkyl, $C_1$-$C_6$alkyl mono- or poly-substituted by halogen and/or by $C_1$-$C_3$alkoxy, or $C_5$-$C_6$cycloalkyl; each of $R_2$ and $R_3$ is $C_1$-$C_5$alkyl or $C_5$-$C_6$cycloalkyl; each of $R_4$ and $R_5$ is hydrogen or $C_1$-$C_3$alkyl; $R_6$ is halogen, methyl, methoxy, trifluoromethyl or trifluoromethoxy; n is 0 or 1; Z is -NH-CS-NH-, -N=C($SR_7$)-NH- or -N=C=N-; and $R_7$ is $C_1$-$C_6$alkyl or $C_3$-$C_4$alkenyl.

**3.** A compound of formula I according to claim 2, in which $R_1$ is $C_1$-$C_5$alkyl or cyclopentyl; each of $R_2$ and $R_3$ is $C_3$-$C_5$alkyl or $C_5$-$C_6$cycloalkyl; each of $R_4$ and $R_5$ is hydrogen or methyl; $R_6$ is fluorine, chlorine or methyl; n is 0 or 1; Z is -NH-CS-NH-, -N=C($SR_7$)-NH- or -N=C=N-; and $R_7$ is $C_1$-$C_3$alkyl or allyl.

**4.** A compound of formula I according to claim 3, in which $R_1$ is $C_3$-$C_5$alkyl or cyclopentyl; each of $R_2$ and $R_3$ is $C_3$-$C_5$alkyl; each of $R_4$ and $R_5$ is hydrogen or methyl; n is 0; and Z is -NH-CS-NH-.

**5.** A compound of formula I according to claim 3, in which $R_1$ is $C_3$-$C_5$alkyl or cyclopentyl, each of $R_2$ and $R_3$ is $C_3$-$C_5$alkyl; each of $R_4$ and $R_5$ is hydrogen or methyl; n is 0; Z is -N=C($SR_7$)-NH; and $R_7$ is $C_1$-$C_2$alkyl.

**6.** A compound of formula I according to claim 3, in which $R_1$ is $C_3$-$C_5$alkyl or cyclopentyl, each of $R_2$ and $R_3$ is $C_3$-$C_5$alkyl; each of $R_4$ and $R_5$ is hydrogen or methyl; n is 0; and Z is -N=C=N-.

**7.** A compound according to claim 1 or claim 4 of formula

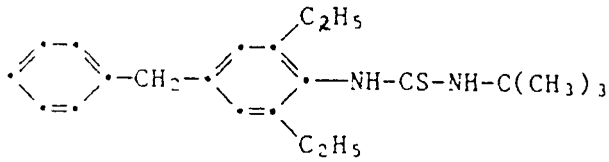,

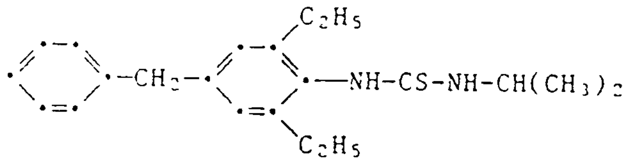,

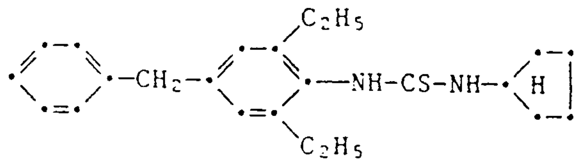,

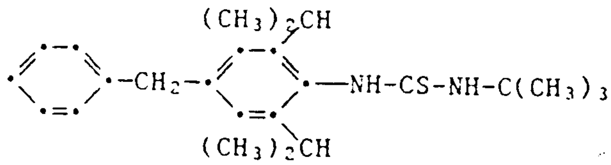,

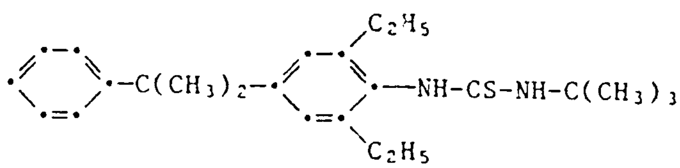,

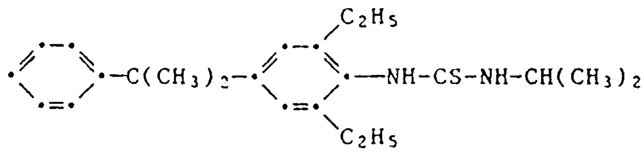

,

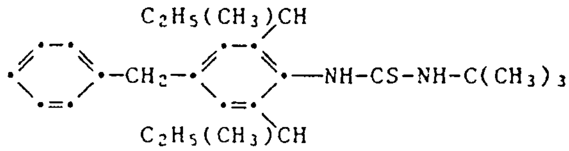

,

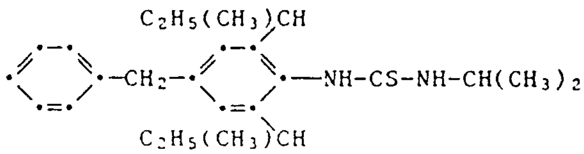

,

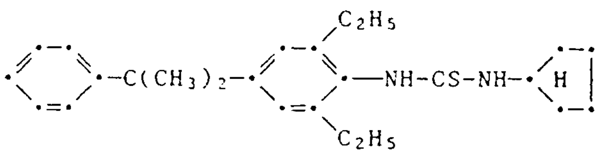

,

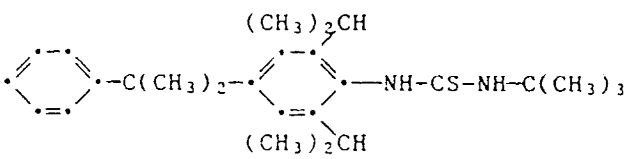

,

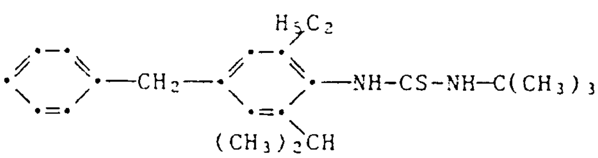

,

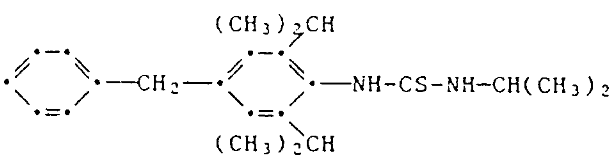

,

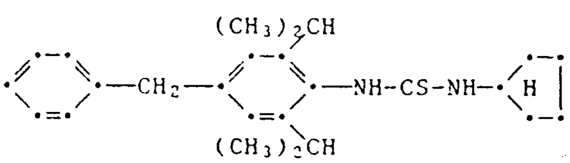

,

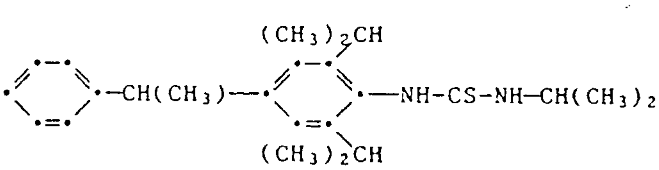

,

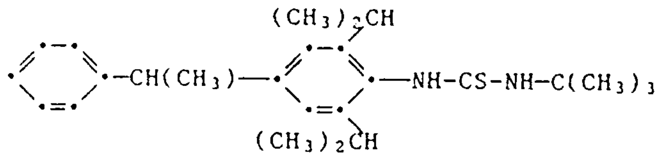
,

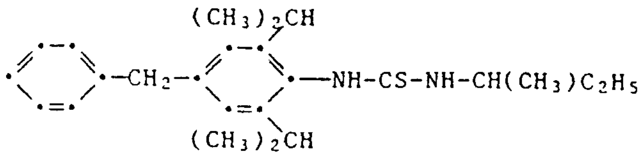
,

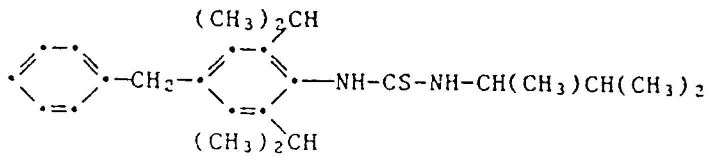
,

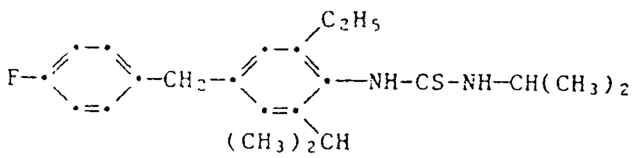
,

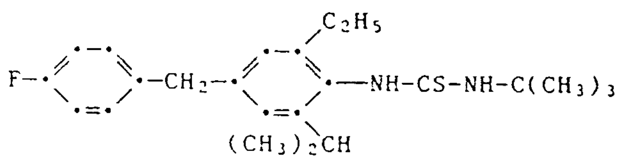
,

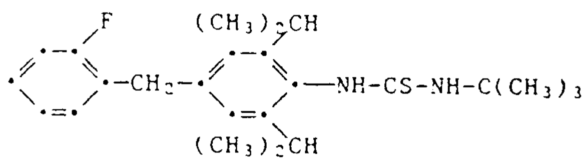
,

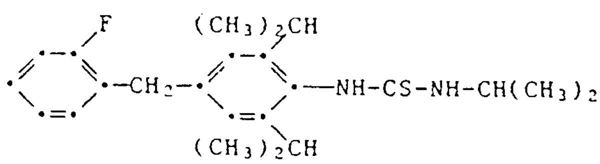
,

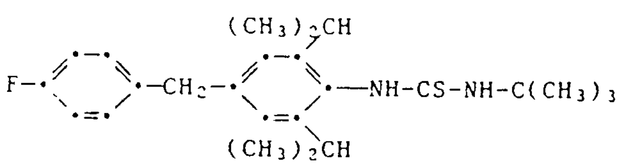
,

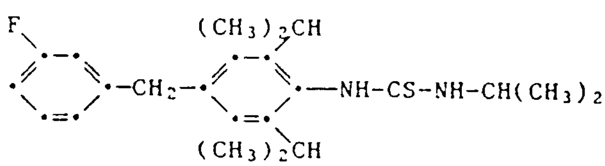
,

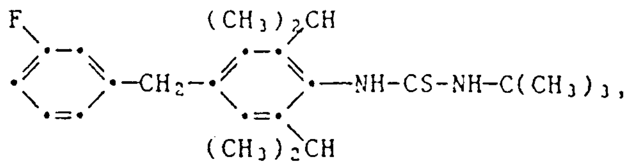

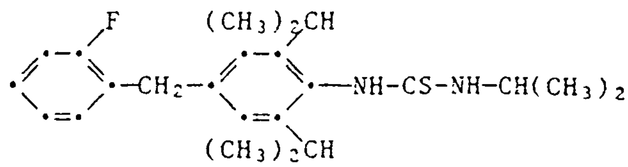

or

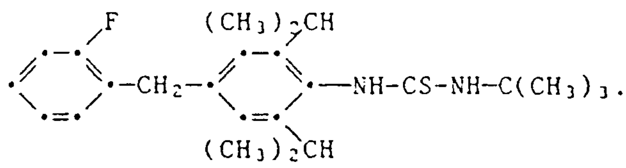

8. A compound according to claim 1 or claim 5 of formula

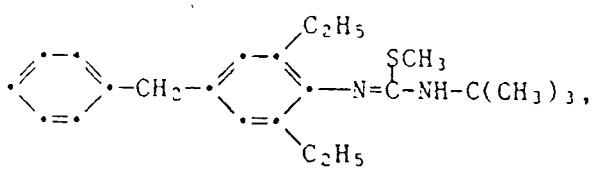

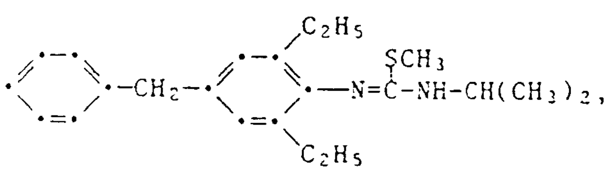

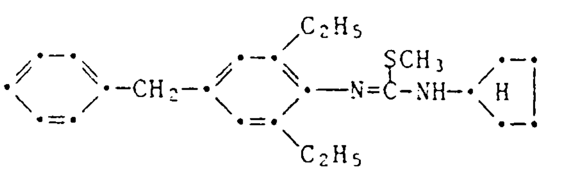

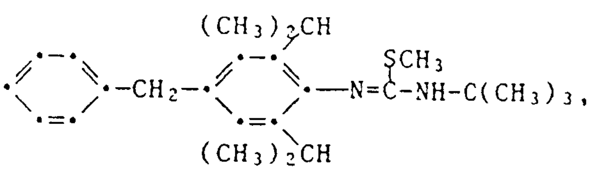

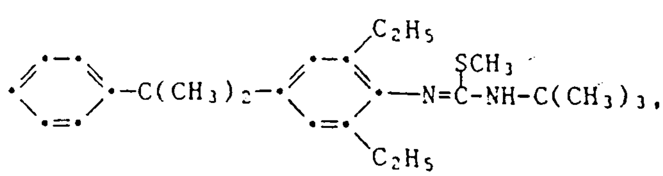

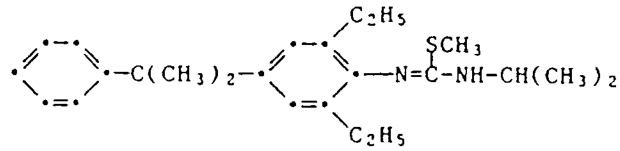
,

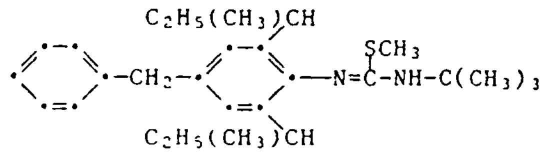
,

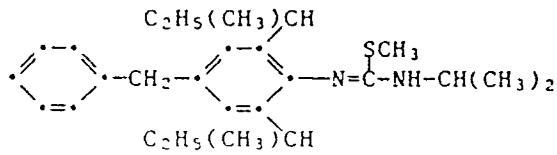
,

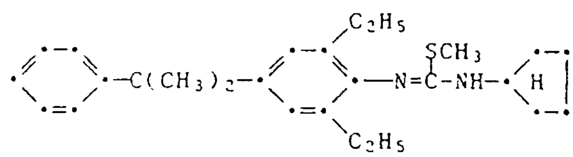
,

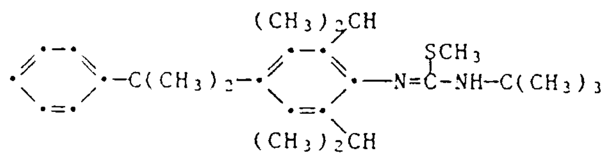
,

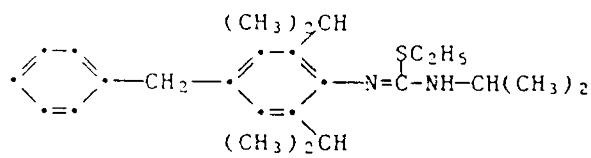
,

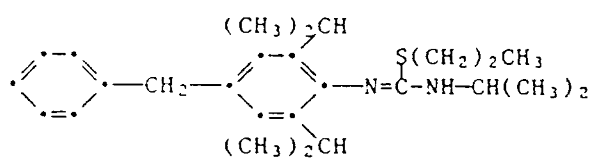
,

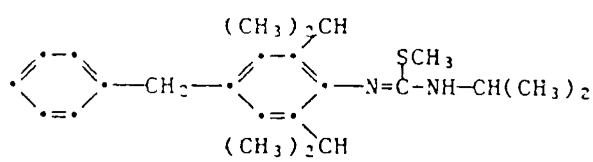
,

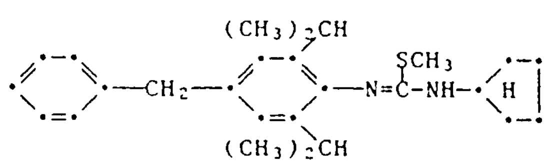
,

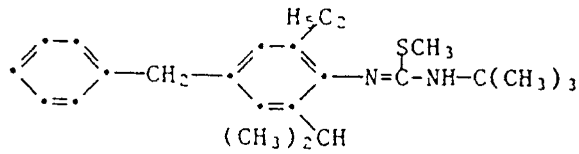,

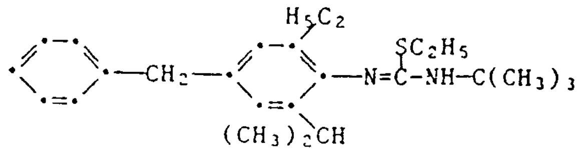,

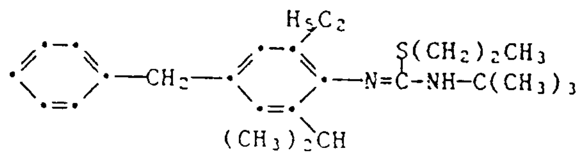,

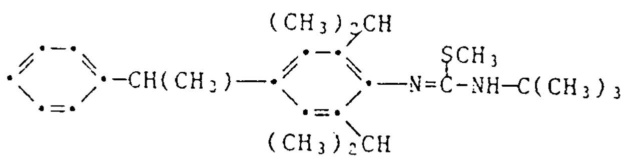,

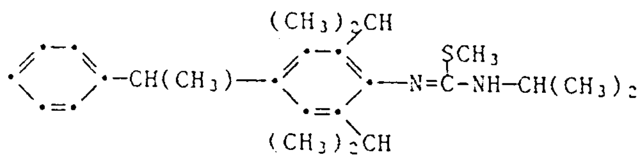,

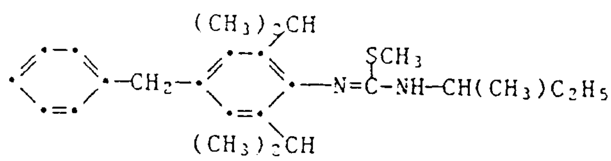,

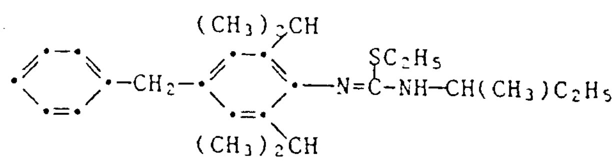,

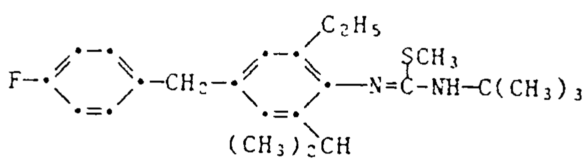,

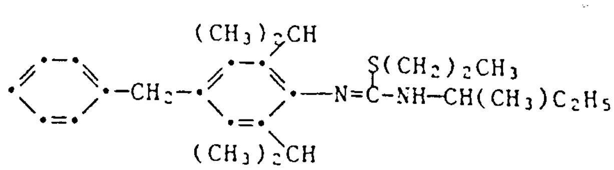,

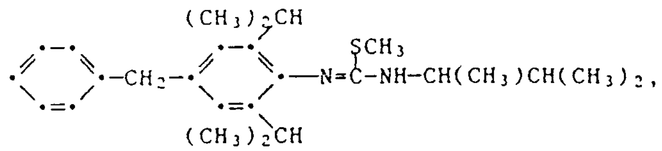

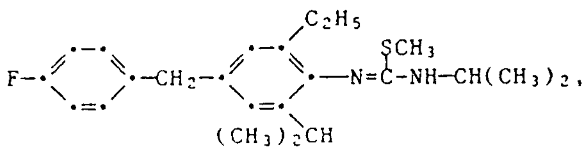

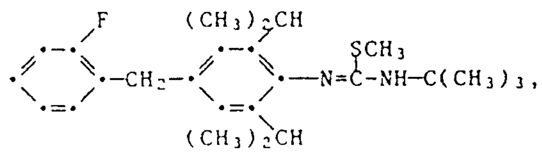

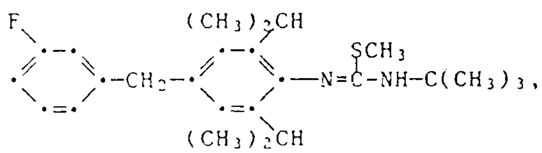

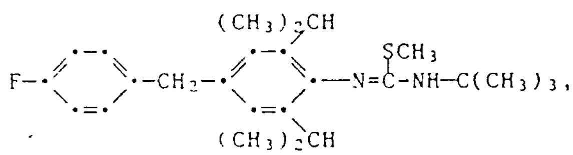

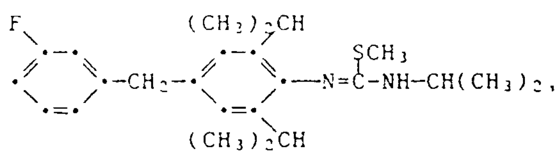

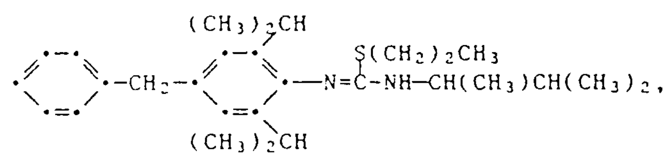

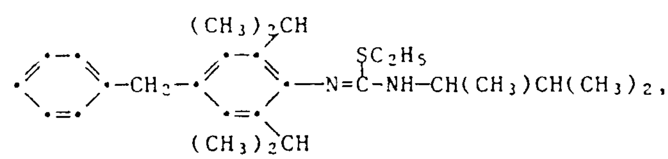

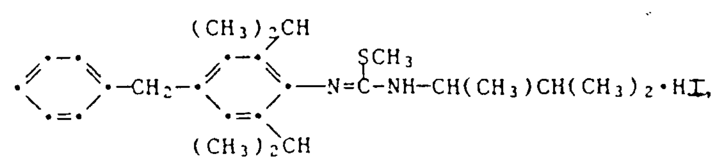

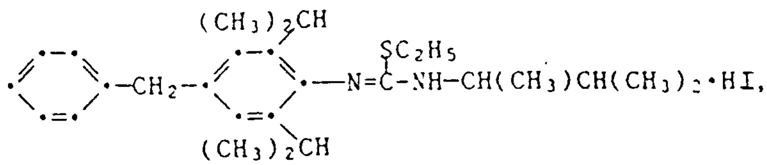

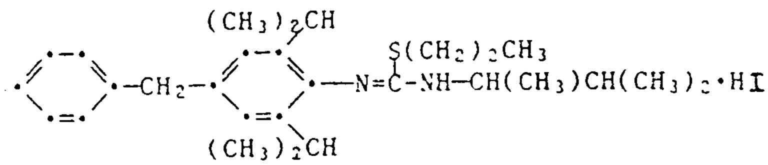

or

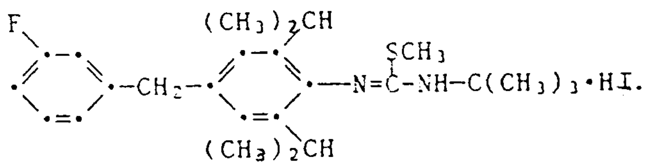

9. A compound according to claim 1 or claim 6 of formula

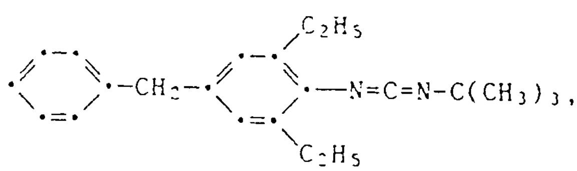

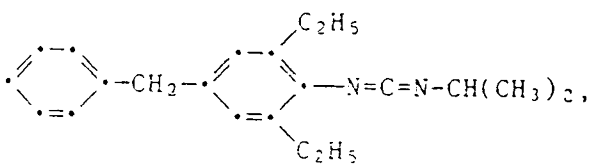

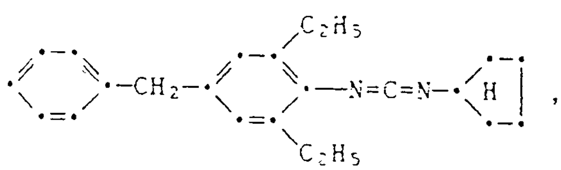

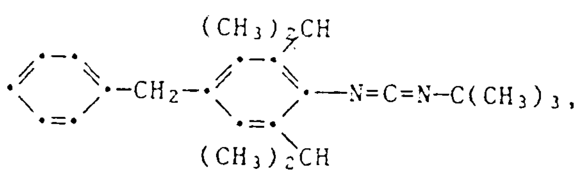

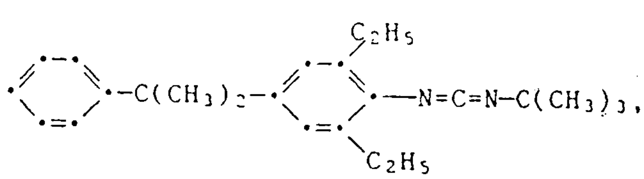

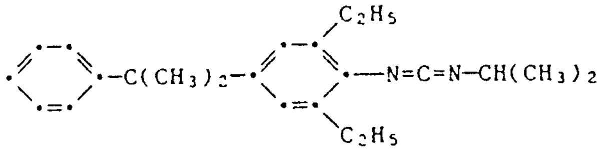,

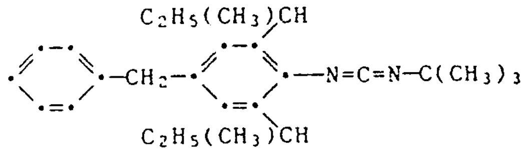,

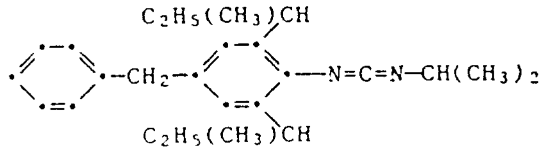,

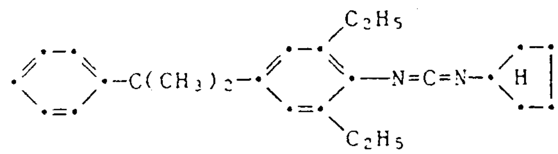,

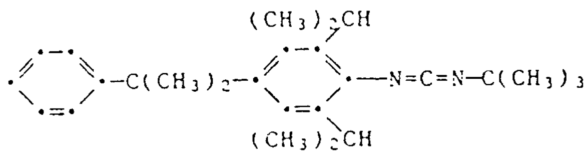,

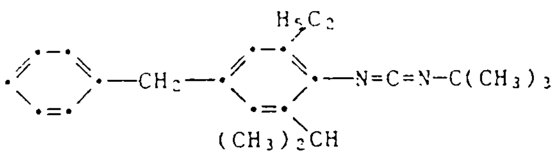,

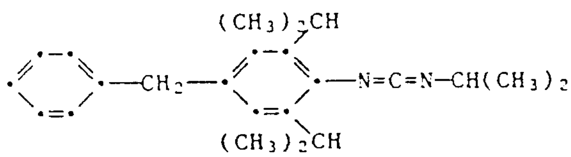,

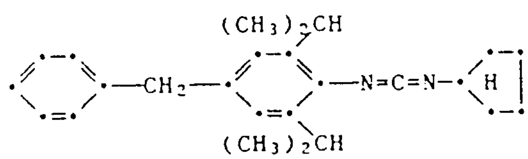,

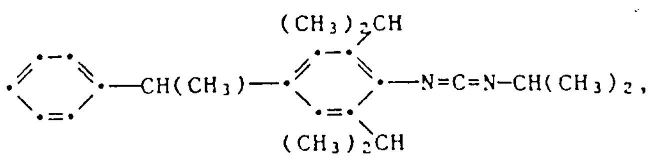,

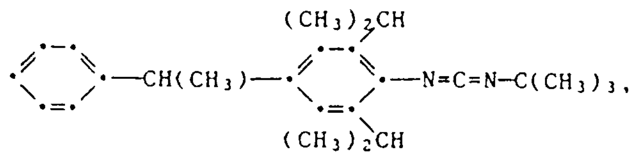

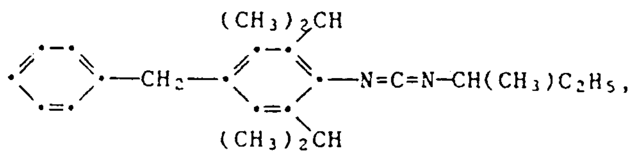

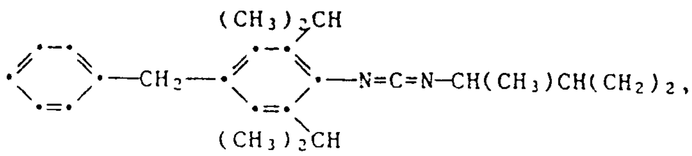

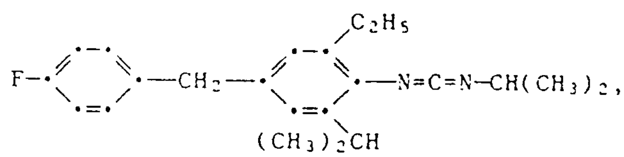

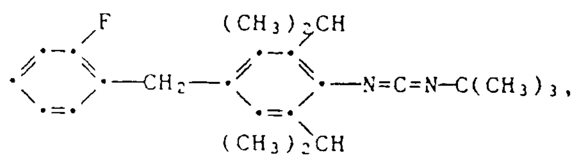

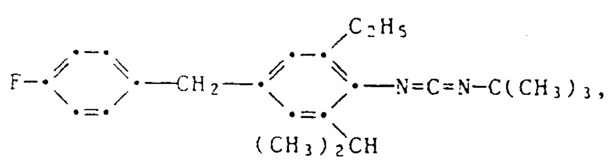

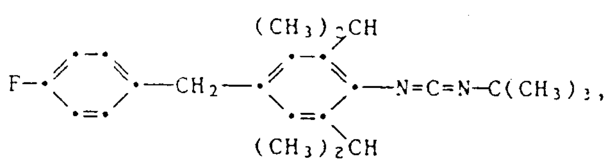

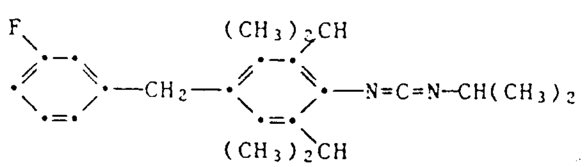

or

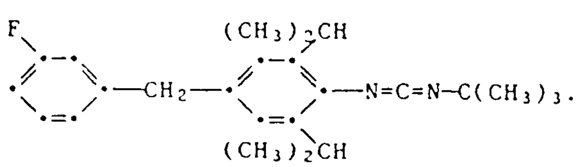

**10.** A process for the preparation of a compound of formula I

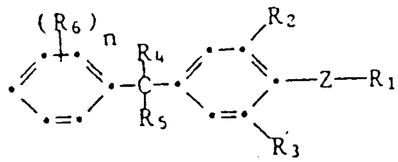

(I)

in which $R_1$ is $C_1$-$C_8$alkyl, $C_1$-$C_8$alkyl mono- or polysubstituted by halogen and/or by $C_1$-$C_6$alkoxy, $C_3$-$C_8$cycloalkyl, $C_3$-$C_8$cycloalkyl mono- or poly-substituted by $C_1$-$C_3$alkyl, or is $C_3$-$C_8$cycloalkyl-$C_1$-$C_4$alkyl; each of $R_2$ and $R_3$ is $C_1$-$C_6$alkyl, $C_5$-$C_6$cycloalkyl or $C_5$-$C_6$cycloalkenyl; each of $R_4$ and $R_5$ is hydrogen or $C_1$-$C_4$alkyl; each $R_6$ is halogen, $C_1$-$C_4$alkyl, $C_1$-$C_4$alkoxy, $C_1$-$C_4$haloalkyl, $C_1$-$C_4$haloalkoxy or a

$$-(CH=CH)_2-,\ -(CH_2)_3-\ \text{or}$$

$$-(CH_2)_4-$$

bridge in the 2,3- or 3,4-position; n is 0, 1 or 2; Z is -NH-CS-NH-, -N=C($SR_7$)-NH- or -N=C=N-, and $R_7$ is $C_1$-$C_{10}$alkyl or $C_3$-$C_5$alkenyl, which process comprises

A) reacting an isothiocyanate of formula II

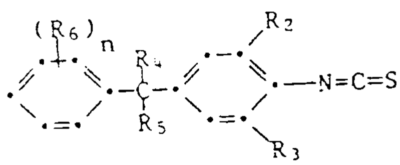

(II)

with an amine of formula III

$H_2N—R_1$ (III)

in an organic solvent or diluent under normal pressure and at a temperature of from 0 to +150 °C to form the thiourea and optionally

B) reacting the resulting thiourea with a compound of formula IV

$X—R_7$ (IV)

in an inert organic solvent under slightly elevated or normal pressure and at a temperature of from +10 to 250 °C to form the isothiourea, or

C) converting the resulting thiourea into the carbodiimide by removal of hydrogen sulfide in an aprotic organic solvent or diluent under normal pressure and at a temperature of from 0 to +150 °C, $R_1$, $R_2$, $R_3$, $R_4$, $R_5$, $R_6$, $R_7$ and n having the meanings given and X being a leaving group.

**11.** A pesticidal composition that comprises as active component at least one compound of formula I

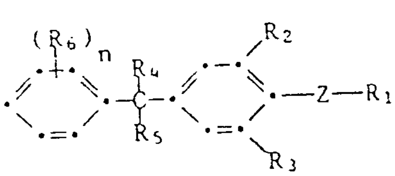

(I)

in which $R_1$ is $C_1$-$C_8$alkyl, $C_1$-$C_8$alkyl mono- or polysubstituted by halogen and/or by $C_1$-$C_6$alkoxy, $C_3$-

$C_8$cycloalkyl, $C_3$-$C_8$cycloalkyl mono- or poly-substituted by $C_1$-$C_3$alkyl, or is $C_3$-$C_8$cycloalkyl-$C_1$-$C_4$alkyl; each of $R_2$ and $R_3$ is $C_1$-$C_6$alkyl, $C_5$-$C_6$cycloalkyl or $C_5$-$C_6$cycloalkenyl; each of $R_4$ and $R_5$ is hydrogen or $C_1$-$C_4$alkyl; each $R_6$ is halogen, $C_1$-$C_4$alkyl, $C_1$-$C_4$alkoxy, $C_1$-$C_4$haloalkyl, $C_1$-$C_4$haloalkoxy or a

$$-(CH=CH)_2-,\quad -(CH_2)_3- \text{ or}$$

$$-(CH_2)_4-$$

bridge in the 2,3- or 3,4-position; n is 0, 1 or 2; Z is -NH-CS-NH-, -N = C($SR_7$)-NH- or -N = C = N-, and $R_7$ is $C_1$-$C_{10}$alkyl or $C_3$-$C_5$alkenyl, or a salt thereof with an organic or inorganic acid, together with suitable carriers and/or adjuvants.

**12.** A pesticidal composition according to claim 11, which comprises as active component at least one compound of formula I in which $R_1$ is $C_1$-$C_6$alkyl, $C_1$-$C_6$alkyl mono- or poly-substituted by halogen and/or by $C_1$-$C_3$alkoxy, or $C_5$-$C_6$cycloalkyl; each of $R_2$ and $R_3$ is $C_1$-$C_5$alkyl or $C_5$-$C_6$cycloalkyl; each of $R_4$ and $R_5$ is hydrogen or $C_1$-$C_3$alkyl; $R_6$ is halogen, methyl, methoxy, trifluoromethyl or trifluoromethoxy; n is 0 or 1; Z is -NH-CS-NH-, -N = C($SR_7$)-NH- or -N = C = N-; and $R_7$ is $C_1$-$C_6$alkyl or $C_3$-$C_4$alkenyl.

**13.** A pesticidal composition according to claim 12, which comprises as active component at least one compound of formula I in which $R_1$ is $C_1$-$C_5$alkyl or cyclopentyl; each of $R_2$ and $R_3$ is $C_3$-$C_5$alkyl or $C_5$-$C_6$cycloalkyl; each of $R_4$ and $R_5$ is hydrogen or methyl; $R_6$ is fluorine, chlorine or methyl; n is 0 or 1; Z is -NH-CS-NH-, -N = C($SR_7$)-NH- or -N = C = N-; and $R_7$ is $C_1$-$C_3$alkyl or allyl.

**14.** A pesticidal composition according to claim 13, which comprises as active component at least one compound of formula I in which $R_1$ is $C_3$-$C_5$alkyl or cyclopentyl; each of $R_2$ and $R_3$ is $C_3$-$C_5$alkyl; each of $R_4$ and $R_5$ is hydrogen or methyl; n is 0; and Z is -NH-CS-NH-.

**15.** A pesticidal composition according to claim 13, which comprises as active component at least one compound of formula I in which $R_1$ is $C_3$-$C_5$alkyl or cyclopentyl, each of $R_2$ and $R_3$ is $C_3$-$C_5$alkyl; each of $R_4$ and $R_5$ is hydrogen or methyl; n is 0; Z is -N = C($SR_7$)-NH; and $R_7$ is $C_1$-$C_2$alkyl.

**16.** A pesticidal composition according to claim 13, which comprises as active component at least one compound of formula I in which $R_1$ is $C_3$-$C_5$alkyl or cyclopentyl, each of $R_2$ and $R_3$ is $C_3$-$C_5$alkyl; each of $R_4$ and $R_5$ is hydrogen or methyl; n is 0; and Z is -N = C = N-.

**17.** Use of a compound of formula I

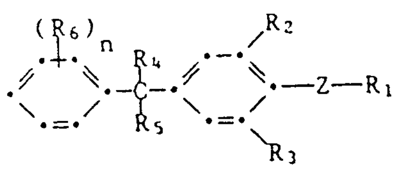

(I)

in which $R_1$ is $C_1$-$C_8$alkyl, $C_1$-$C_8$alkyl mono- or polysubstituted by halogen and/or by $C_1$-$C_6$alkoxy, $C_3$-$C_8$cycloalkyl, $C_3$-$C_8$cycloalkyl mono- or poly-substituted by $C_1$-$C_3$alkyl, or is $C_3$-$C_8$cycloalkyl-$C_1$-$C_4$alkyl; each of $R_2$ and $R_3$ is $C_1$-$C_6$alkyl, $C_5$-$C_6$cycloalkyl or $C_5$-$C_6$cycloalkenyl; each of $R_4$ and $R_5$ is hydrogen or $C_1$-$C_4$alkyl; each $R_6$ is halogen, $C_1$-$C_4$alkyl, $C_1$-$C_4$alkoxy, $C_1$-$C_4$haloalkyl, $C_1$-$C_4$haloalkoxy or a

$$-(CH=CH)_2-,\quad -(CH_2)_3- \text{ or}$$

$-(CH_2)_4-$

bridge in the 2,3- or 3,4-position; n is 0, 1 or 2; Z is -NH-CS-NH-, -N=C($SR_7$)-NH- or -N=C=N-, and $R_7$ is $C_1$-$C_{10}$alkyl or $C_3$-$C_5$alkenyl, or a salt thereof with an organic or inorganic acid, for controlling pests of animals and plants.

**18.** Use according to claim 17 of a compound of formula I for controlling insects and arachnids.

**19.** A method of controlling pests of animals and plants, which comprises bringing the pests in their various stages of development into contact with a compound of formula I

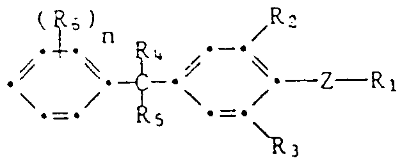

(I)

in which $R_1$ is $C_1$-$C_8$alkyl, $C_1$-$C_8$alkyl mono- or polysubstituted by halogen and/or by $C_1$-$C_6$alkoxy, $C_3$-$C_8$cycloalkyl, $C_3$-$C_8$cycloalkyl mono- or poly-substituted by $C_1$-$C_3$alkyl, or is $C_3$-$C_8$cycloalkyl-$C_1$-$C_4$alkyl; each of $R_2$ and $R_3$ is $C_1$-$C_6$alkyl, $C_5$-$C_6$cycloalkyl or $C_5$-$C_6$cycloalkenyl; each of $R_4$ and $R_5$ is hydrogen or $C_1$-$C_4$alkyl; each $R_6$ is halogen, $C_1$-$C_4$alkyl, $C_1$-$C_4$alkoxy, $C_1$-$C_4$haloalkyl, $C_1$-$C_4$haloalkoxy or a

$-(CH=CH)_2-$, $-(CH_2)_3-$ or

$-(CH_2)_4-$

bridge in the 2,3- or 3,4-position; n is 0, 1 or 2; Z is -NH-CS-NH-, -N=C($SR_7$)-NH- or -N=C=N-, and $R_7$ is $C_1$-$C_{10}$alkyl or $C_3$-$C_5$alkenyl, or with one of the salts thereof with an organic or inorganic acid.

**20.** A compound of formula II'

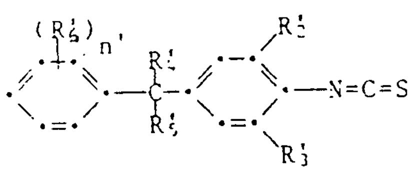

(II')

in which each of $R_2^1$ and $R_3^1$ is $C_3$-$C_5$alkyl or $C_5$-$C_6$cycloalkyl; each of $R_4^1$ and $R_5^1$ is hydrogen or methyl; $R_6^1$ is fluorine, chlorine or methyl; and n' is 0 or 1.

**21.** A compound of formula II' according to claim 20, in which each of $R_2^1$ and $R_3^1$ is $C_3$-$C_5$alkyl; each of $R_4^1$ and $R_5^1$ is hydrogen or methyl; and n' is 0.

**22.** A compound according to claim 20 or claim 21 of formula

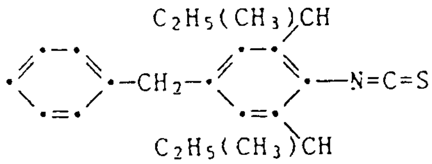,

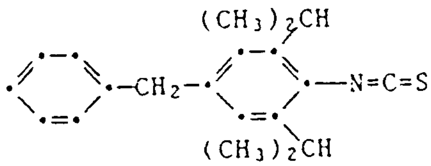,

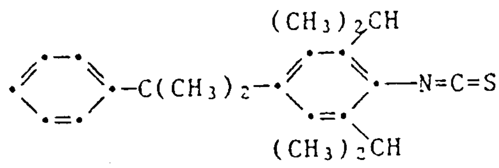,

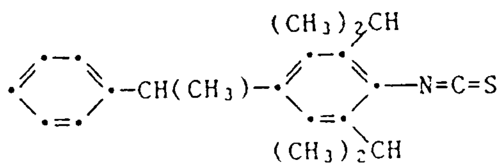,

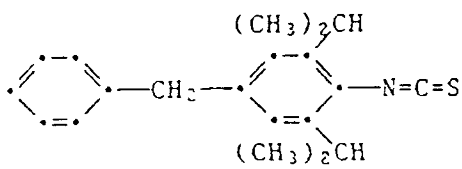,

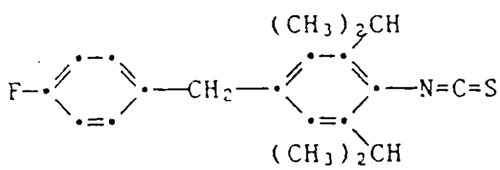,

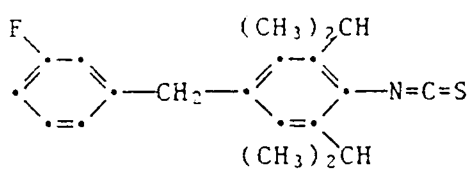

or

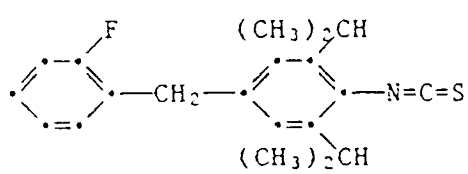.

**23.** A compound of formula V'

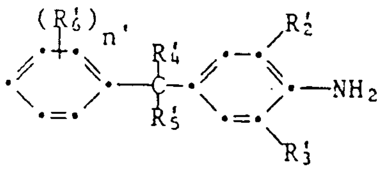

(V')

in which each of $R^1_2$ and $R^1_3$ is $C_3$-$C_5$alkyl or $C_5$-$C_6$cycloalkyl; each of $R^1_4$ and $R^1_5$ is hydrogen or methyl; $R^1_6$ is fluorine, chlorine or methyl; and n' is 0 or 1.

**24.** A compound of formula V' according to claim 23 in which each of $R^1_2$ and $R^1_3$ is $C_3$-$C_5$alkyl; each of $R^1_4$ and $R^1_5$ is hydrogen or methyl; and n' is 0 or 1.

**25.** A compound according to claim 23 or claim 24 of formula

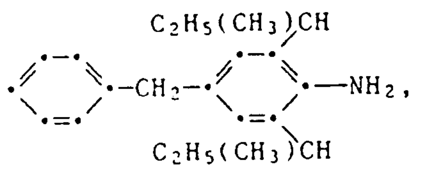

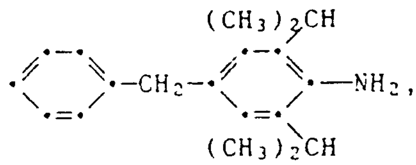

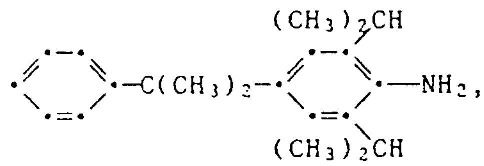

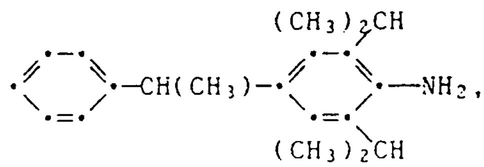

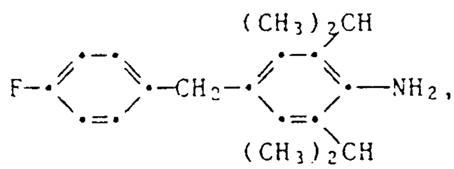

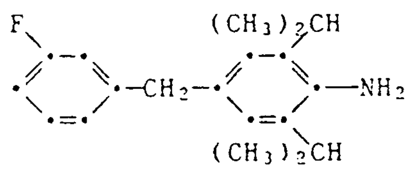

or

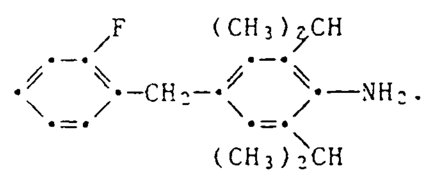

## Revendications

**1.** Composés de formule I

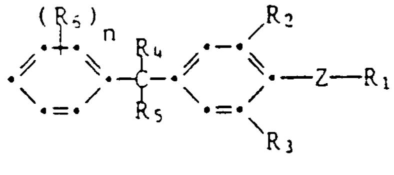

(I),

dans laquelle

$R_1$ représente un groupe alkyle en C 1-C 8, un groupe alkyle en C 1-C 8 portant un ou plusieurs substituants halogéno et/ou alcoxy en C 1-C 6, un groupe cycloalkyle en C 3-C 8, un groupe cycloalkyle en C 3-C 8 portant un ou plusieurs substituants alkyle en C 1-C 3, ou un groupe (cycloalkyle en C 3-C 8)-alkyle en C 1-C 4;

$R_2$ et $R_3$ représentent chacun un groupe alkyle en C 1-C 6, cycloalkyle en C 5-C 6 ou cycloalcényle en C 5-C 6;

$R_4$ et $R_5$ représentent chacun l'hydrogène ou un groupe alkyle en C 1-C 4; chacun des symboles $R_6$ représente un halogène, un groupe alkyle en C 1-C 4, alcoxy en C 1-C 4, halogénoalkyle en C 1-C 4, halogénoalcoxy en C 1-C 4 ou un pont

$$-(-CH=CH-)_2 , \quad -(-CH_2-)_3 \quad \text{ou} \quad -(-CH_2-)_4$$

en position 2,3 ou 3,4;

n est égal à 0, 1 ou 2;

Z représente -NH-CS-NH-, -N=C($SR_7$)-NH- ou -N=C=N-, et

$R_7$ représente un groupe alkyle en C 1-C 10 ou alcényle en C 3-C 5,

et leurs sels d'acides organiques ou minéraux.

**2.** Composés de formule I de la revendication 1, dans laquelle $R_1$ représente un groupe alkyle en C 1-C 6, un groupe alkyle en C 1-C 6 portant un ou plusieurs substituants halogéno et/ou alcoxy en C 1-C 3, ou un groupe cycloalkyle en C 5-C 6; $R_2$ et $R_3$ représentent chacun un groupe alkyle en C 1-C 5 ou cycloalkyle en C 5-C 6; $R_4$ et $R_5$ représentent chacun l'hydrogène ou un groupe alkyle en C 1-C 3; $R_6$ représente un halogène, un groupe méthyle, méthoxy, trifluorométhyle ou trifluorométhoxy, n est égal à 0 ou 1; Z représente -NH-CS-NH-, -N=C($SR_7$)-NH- ou -N=C=N-; et $R_7$ représente un groupe alkyle en C 1-C 6 ou alcényle en C 3-C 4.

**3.** Composés de formule I de la revendication 2, dans laquelle $R_1$ représente un groupe alkyle en C 1-C 5 ou cyclopentyle; $R_2$ et $R_3$ représentent chacun un groupe alkyle en C 3-C 5 ou cycloalkyle en C 5-C 6; $R_4$ et $R_5$ représentent chacun l'hydrogène ou un groupe méthyle; $R_6$ représente le fluor, le chlore ou un groupe méthyle; n est égal à 0 ou 1; Z représente -NH-CS-NH-, -N=C($SR_7$)-NH- ou -N=C=N-; et $R_7$ représente un groupe alkyle en C 1-C 3 ou allyle.

**4.** Composés de formule I de la revendication 3, dans laquelle $R_1$ représente un groupe alkyle en C 3-C 5 ou cyclopentyle; $R_2$ et $R_3$ représentent chacun un groupe alkyle en C 3-C 5; $R_4$ et $R_5$ représentent chacun l'hydrogène ou un groupe méthyle; n est égal à 0; et Z représente -NH-CS-NH.

**5.** Composés de formule I de la revendication 3, dans laquelle $R_1$ représente un groupe alkyle en C 3-C 5 ou cyclopentyle; $R_2$ et $R_3$ représentent chacun un groupe alkyle en C 3-C 5; $R_4$ et $R_5$ représentent chacun l'hydrogène ou un groupe méthyle; n est égal à 0; et Z représente -N=C($SR_7$)-NH et $R_7$ représente un groupe alkyle en C 1-C 2.

**6.** Composés de formule I de la revendication 3, dans laquelle $R_1$ représente un groupe alkyle en C 3-C 5 ou cyclopentyle; $R_2$ et $R_3$ représentent chacun un groupe alkyle en C 3-C 5; $R_4$ et $R_5$ représentent chacun l'hydrogène ou un groupe méthyle; n est égal à 0; et Z représente -N=C=N-.

**7.** Les composés selon revendication 1 ou 4, de formules

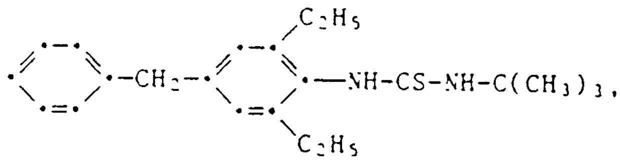
,

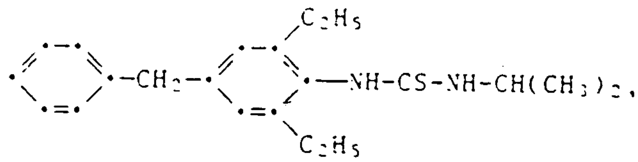
,

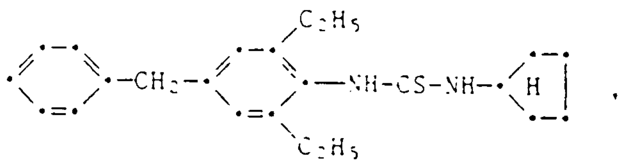
,

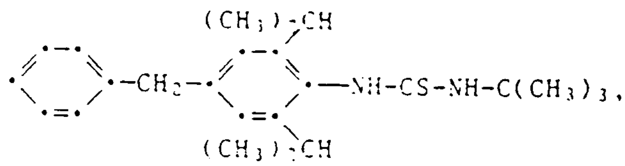
,

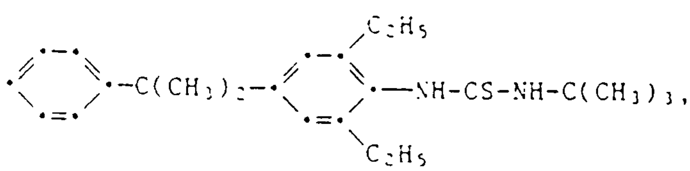
,

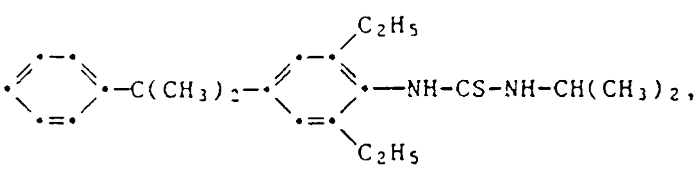
,

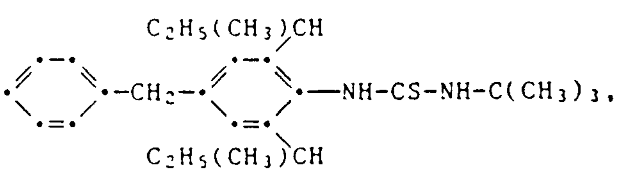
,

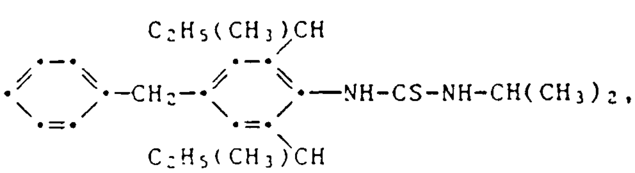
,

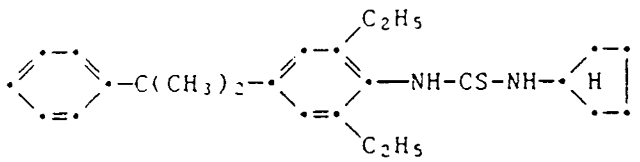
,

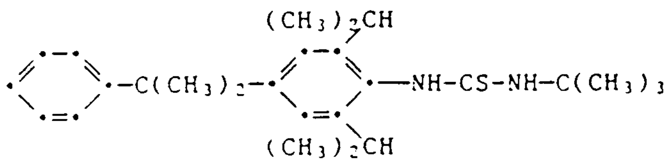
,

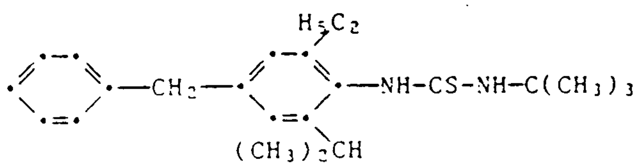
,

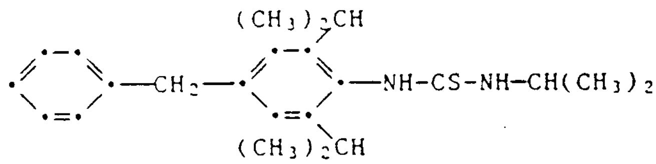
,

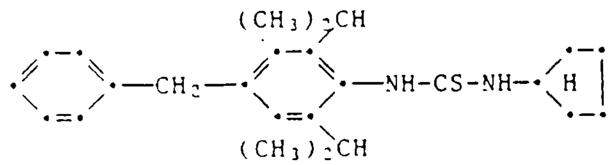
,

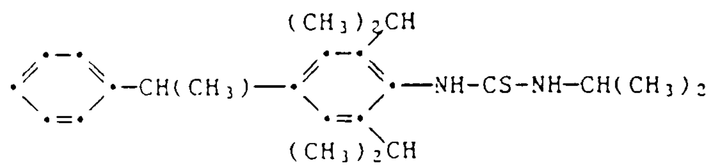
,

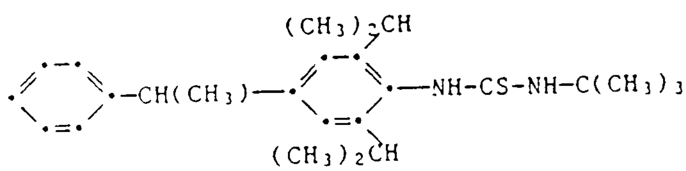
,

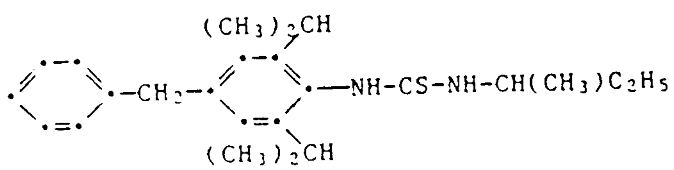
,

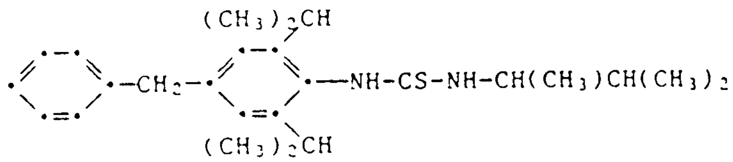,

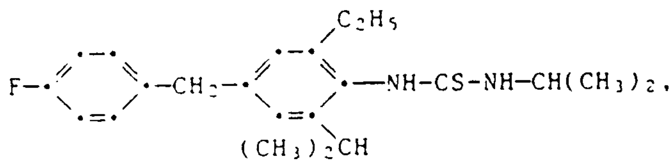,

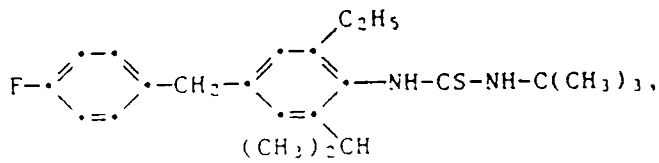,

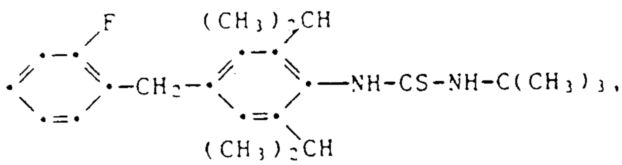,

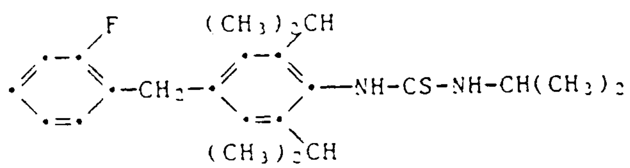,

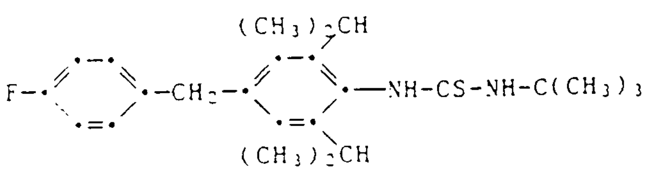,

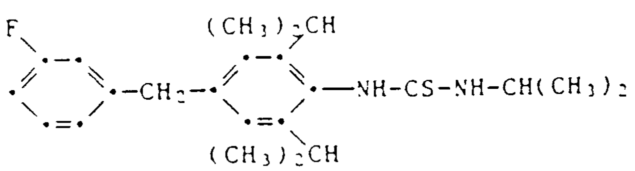,

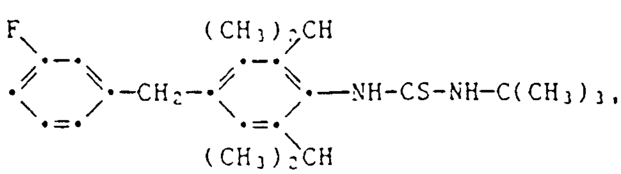,

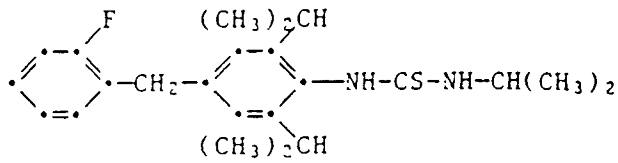 et

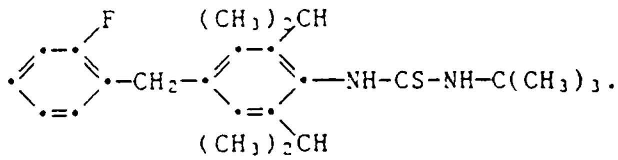

8. Composés selon revendication 1 ou 5, de formules

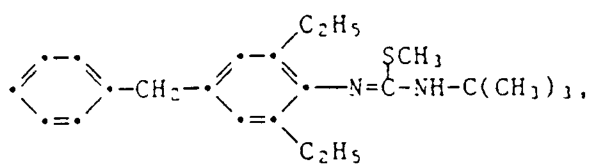

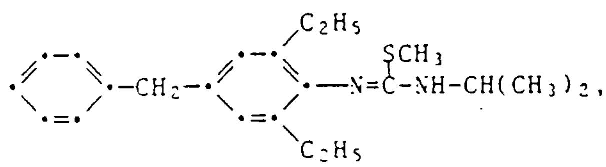

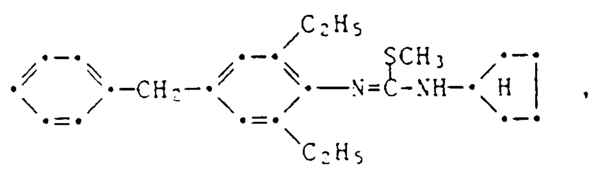

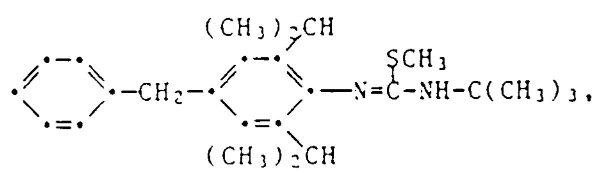

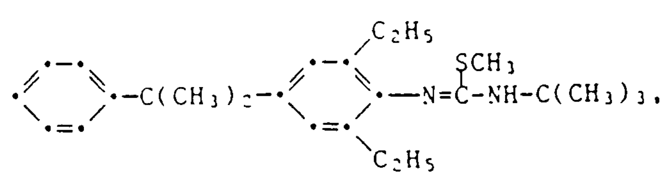

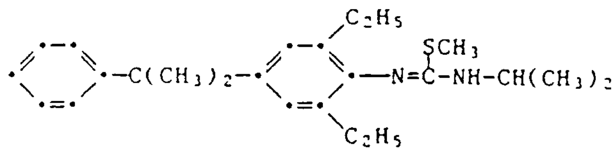

,

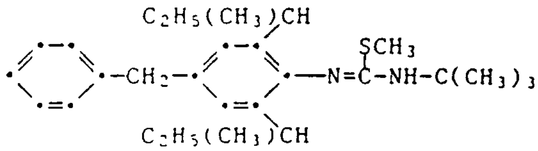

,

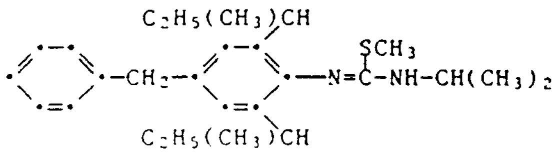

,

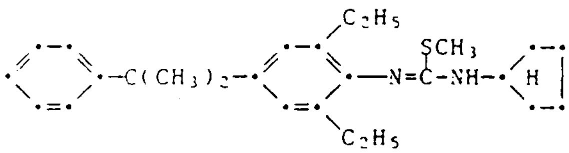

,

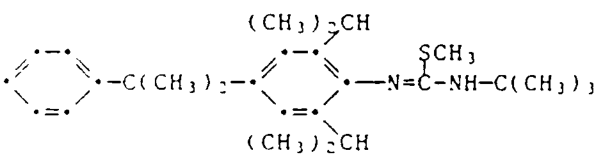

,

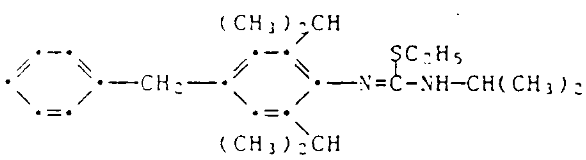

,

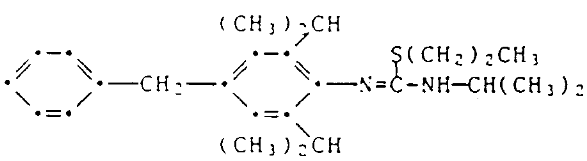

,

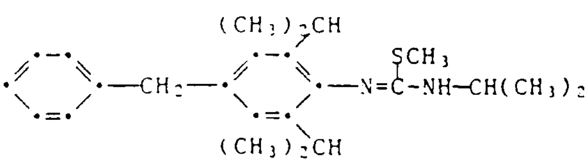

,

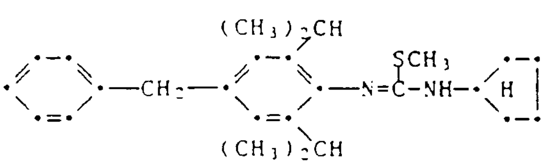

,

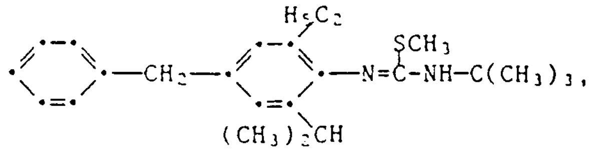,

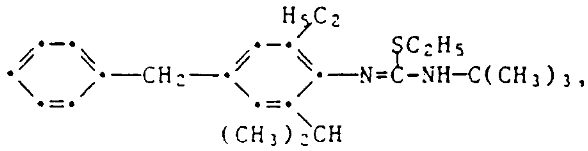,

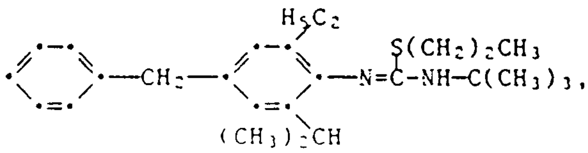,

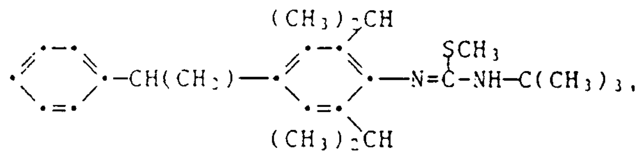,

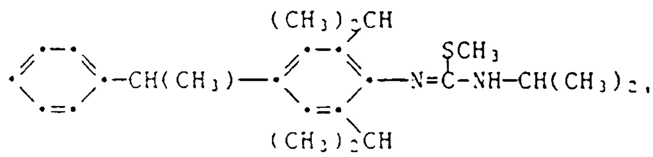,

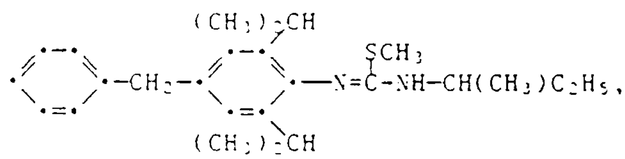,

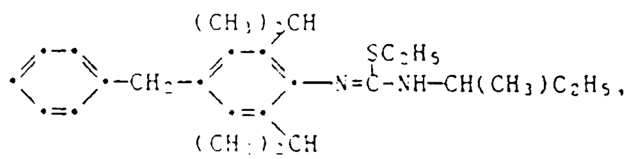,

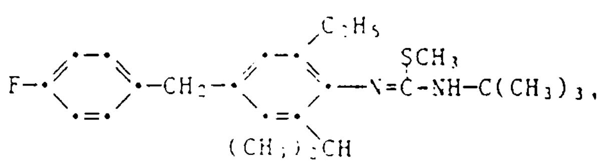,

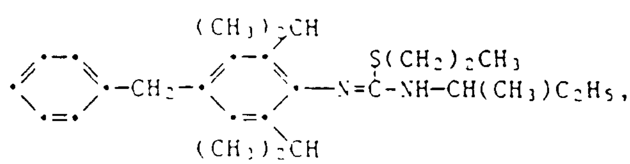,

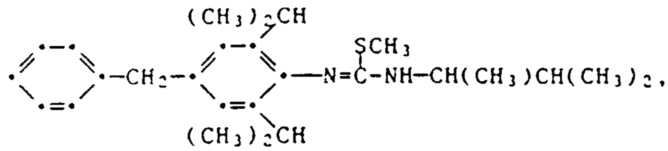

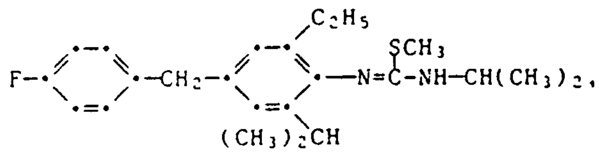

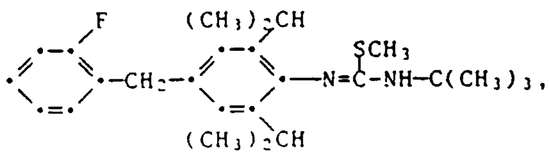

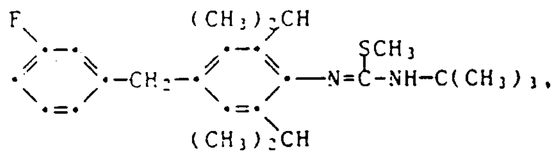

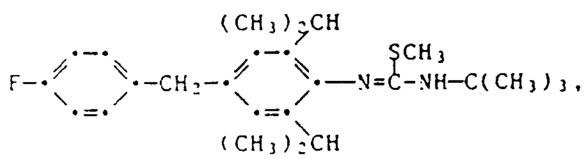

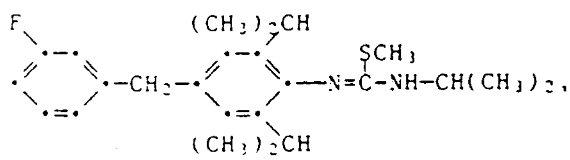

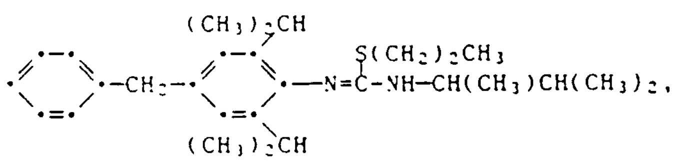

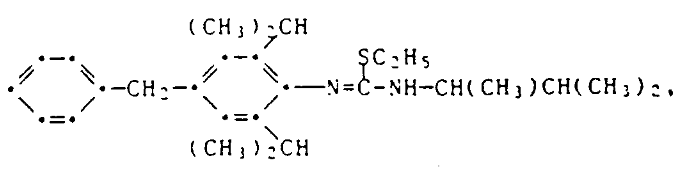

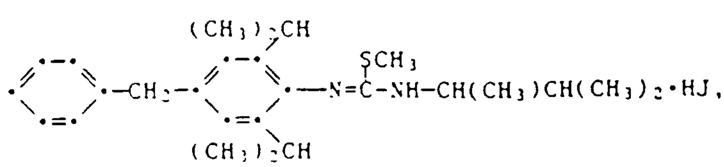

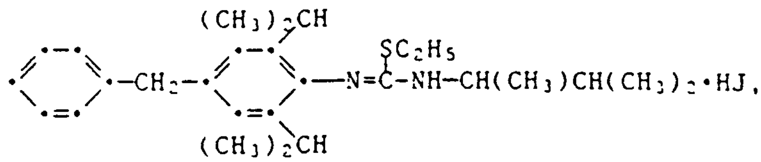

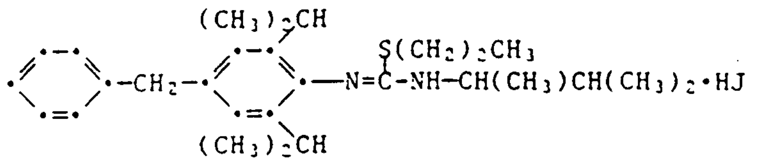

et

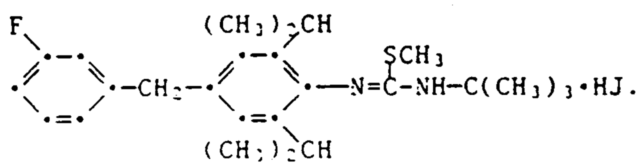

**9.** Les composés selon revendication 1 ou 6, de formules

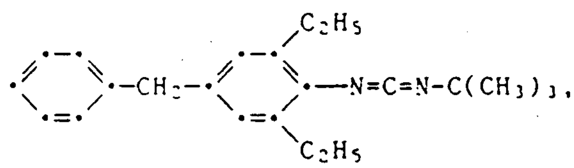

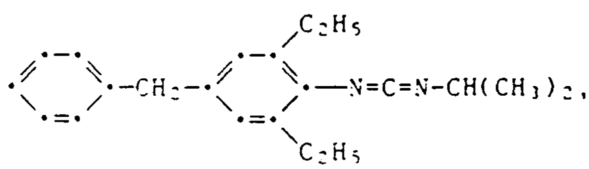

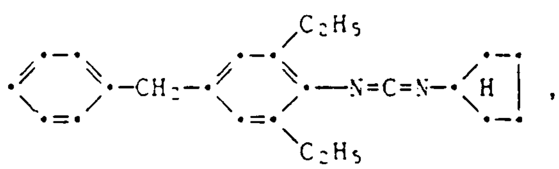

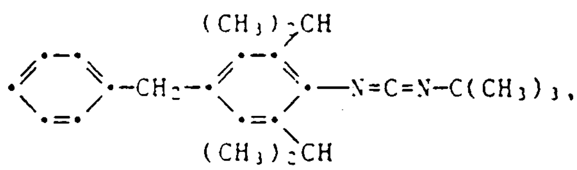

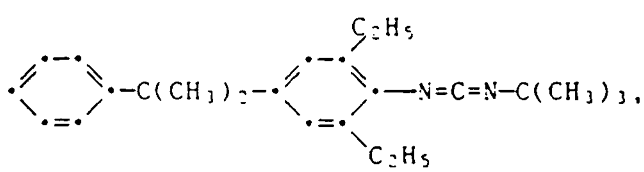

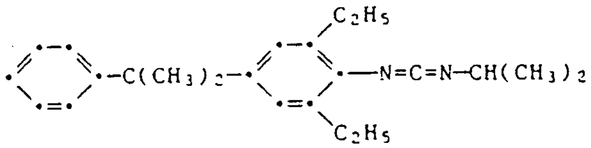 ,

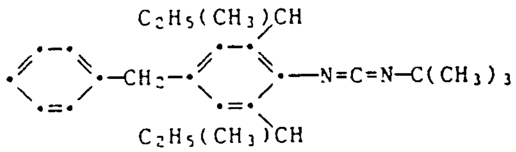 ,

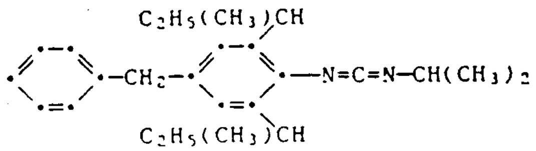 ,

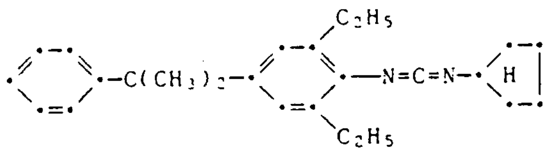 ,

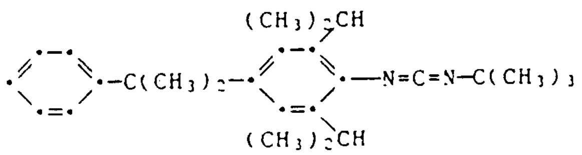 ,

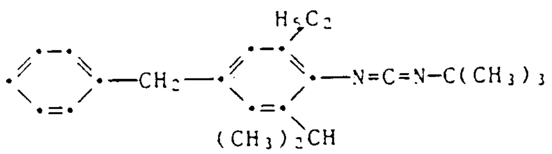 ,

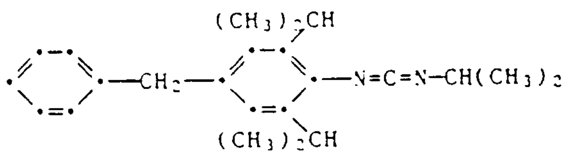 ,

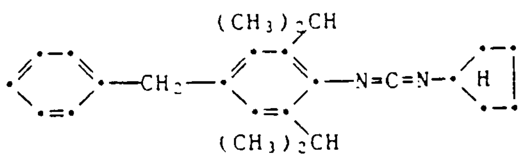 ,

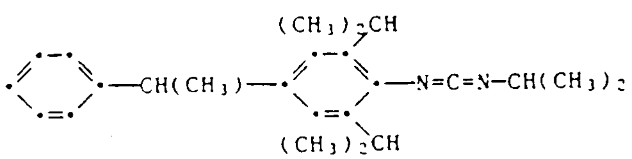 ,

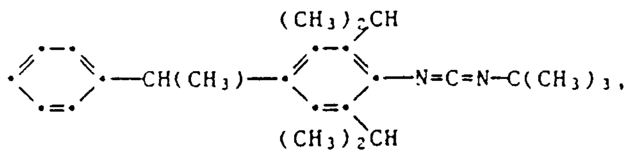

,

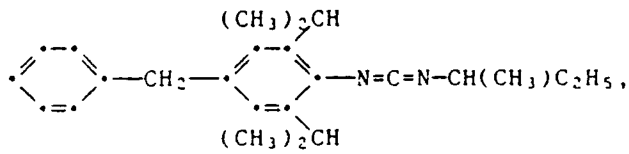

,

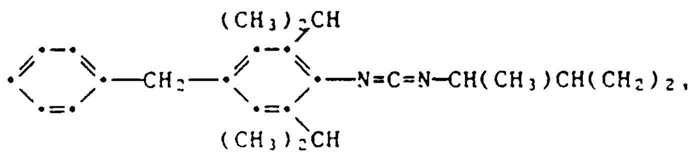

,

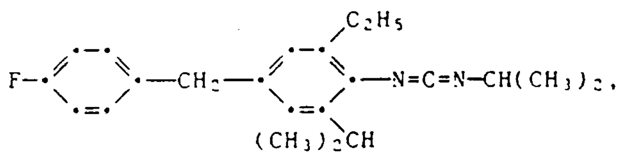

,

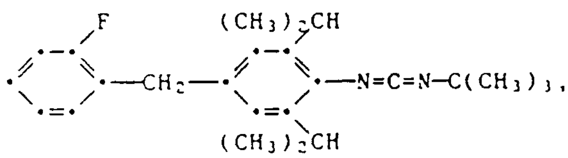

,

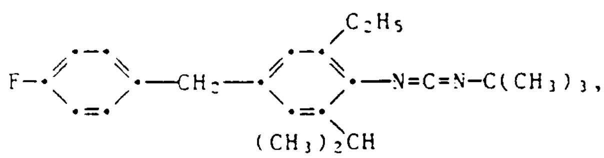

,

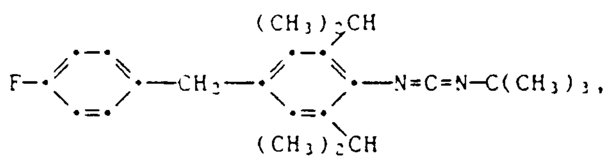

,

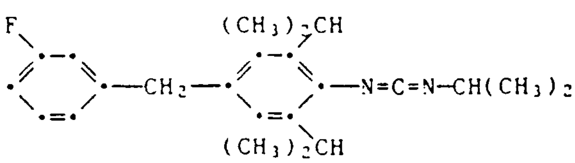

et

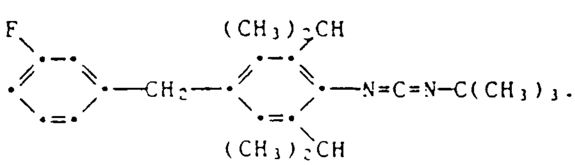

.

**10.** Procédé de préparation d'un composé de formule I

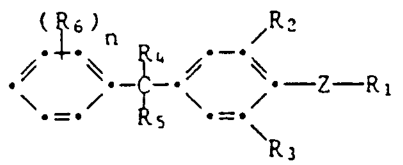 (I),

dans laquelle

$R_1$ représente Un groupe alkyle en C 1-C 8, un groupe alkyle en C 1-C 8 portant un ou plusieurs substituants halogéno et/ou alcoxy en C 1-C 6, un groupe cycloalkyle en C 3-C 8, un groupe cycloalkyle en C 3-C 8 portant un ou plusieurs substituants alkyle en C 1-C 3, ou un groupe (cycloalkyle en C 3-C 8)-alkyle en C 1-C 4;

$R_2$ et $R_3$ représentent chacun un groupe alkyle en C 1-C 6, cycloalkyle en C 5-C 6 ou cycloalcényle en C 5-C 6;

$R_4$ et $R_5$ représentent chacun l'hydrogène ou un groupe alkyle en C 1-C 4;
chacun des symboles $R_6$ représente un halogène, un groupe alkyle en C 1-C 4, alcoxy en C 1-C 4, halogénoalkyle en C 1-C 4, halogénoalcoxy en C 1-C 4 ou un pont

$$-\!(-CH=CH-)_2-,$$

$$-\!(-CH_2-)_3 \text{ ou } -\!(-CH_2-)_4$$

en position 2,3 ou 3,4;

n est égal à 0, 1 ou 2;

Z représente -NH-CS-NH-, -N = C($SR_7$)-NH- ou -N = C = N-, et

$R_7$ représente un groupe alkyle en C 1-C 10 ou alcényle en C 3-C 5,

caractérisé en ce que :

A) on fait réagir un isothiocyanate de formule II

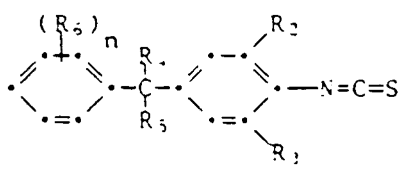 (II)

avec une amine de formule III

$H_2N—R_1$ (III)

dans un solvant ou diluant organique, à pression normale et à une température de 0 à +150 °C, la réaction donnant une thiourée, et le cas échéant

B) on fait réagir cette thiourée avec un composé de formule IV

$X—R_7$ (IV)

dans un solvant organique inerte, à pression normale ou légèrement supérieure à la normale et à une température de +10 à +250 °C, la réaction donnant l'isothiourée, ou bien

C) on convertit la thiourée par scission d'hydrogène sulfuré dans un solvant ou diluant organique aprotonique, à pression normale et à une température de 0 à +150 °C, en le carbodiimide, les symboles $R_1$, $R_2$, $R_3$, $R_4$, $R_5$, $R_6$, $R_7$ et n ayant les significations indiquées ci-dessus, et X représente un substituant éliminable.

**11.** Produit pesticide contenant au moins un composant actif consistant en un composé de formule I

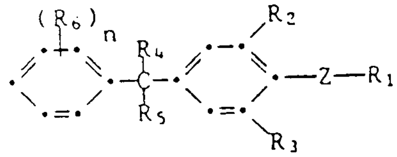

(I)

dans laquelle

$R_1$ représente un groupe alkyle en C 1-C 8, un groupe alkyle en C 1-C 8 portant un ou plusieurs substituants halogéno et/ou alcoxy en C 1-C 6, un groupe cycloalkyle en C 3-C 8, un groupe cycloalkyle en C 3-C 8 portant un ou plusieurs substituants alkyle en C 1-C 3, ou un groupe (cycloalkyle en C 3-C 8)-alkyle en C 1-C 4;

$R_2$ et $R_3$ représentent chacun un groupe alkyle en C 1-C 6, cycloalkyle en C 5-C 6 ou cycloalcényle en C 5-C 6;

$R_4$ et $R_5$ représentent chacun l'hydrogène ou un groupe alkyle en C 1-C 4;

chacun des symboles $R_6$ représente un halogène, un groupe alkyle en C 1-C 4, alcoxy en C 1-C 4, halogénoalkyle en C 1-C 4, halogénoalcoxy en C 1-C 4 ou un pont

$$-(-CH=CH-)_2-,\quad -(-CH_2-)_3-\quad \text{ou}\quad -(-CH_2-)_4-$$

en position 2,3 ou 3,4;

n est égal à 0, 1 ou 2;

Z représente -NH-CS-NH-, -N = C($SR_7$)-NH- ou -N = C = N-, et

$R_7$ représente un groupe alkyle en C 1-C 10 ou alcényle en C 3-C 5,

ou un sel d'un tel composé et d'un acide organique ou minéral, avec des véhicules et/ou additifs appropriés.

**12.** Produit pesticide selon revendication 11, contenant au moins un composant actif consistant en un composé de formule I dans laquelle $R_1$ représente un groupe alkyle en C 1-C 6, un groupe alkyle en C 1-C 6 portant un ou plusieurs substituants halogéno et/ou alcoxy en C 1-C 3, ou un groupe cycloalkyle en C 5-C 6; $R_2$ et $R_3$ représentent chacun un groupe alkyle en C 1-C 5 ou cycloalkyle en C 5-C 6; $R_4$ et $R_5$ représentent chacun l'hydrogène ou un groupe alkyle en C 1-C 3; $R_6$ représente un halogène, un groupe méthyle, méthoxy, trifluorométhyle ou trifluorométhoxy, n est égal à 0 ou 1; Z représente -NH-CS-NH-, -N = C($SR_7$)-NH- ou -N = C = N-; et $R_7$ représente un groupe alkyle en C 1-C 6 ou alcényle en C 3-C 4.

**13.** Produit pesticide selon revendication 12, contenant au moins un composant actif consistant en un composé de formule I dans laquelle $R_1$ représente un groupe alkyle en C 1-C 5 ou cyclopentyle; $R_2$ et $R_3$ représentent chacun un groupe alkyle en C 3-C 5 ou cycloalkyle en C 5-C 6; $R_4$ et $R_5$ représentent chacun l'hydrogène ou un groupe méthyle; $R_6$ représente le fluor, le chlore ou un groupe méthyle; n est égal à 0 ou 1; Z représente -NH-CS-NH-, -N = C($SR_7$)-NH- ou -N = C = N-; et $R_7$ représente un groupe alkyle en C 1-C 3 ou allyle.

**14.** Produit pesticide selon revendication 13, contenant au moins un composant actif consistant en un composé de formule I dans laquelle $R_1$ représente un groupe alkyle en C 3-C 5 ou cyclopentyle; $R_2$ et $R_3$ représentent chacun un groupe alkyle en C 3-C 5; $R_4$ et $R_5$ représentent chacun l'hydrogène ou un groupe méthyle; n est égal à 0; et Z représente -NH-CS-NH.

**15.** Produit pesticide selon revendication 13, contenant au moins un composant actif consistant en un composé de formule I dans laquelle $R_1$ représente un groupe alkyle en C 3-C 5 ou cyclopentyle; $R_2$ et $R_3$ représentent chacun un groupe alkyle en C 3-C 5; $R_4$ et $R_5$ représentent chacun l'hydrogène ou un groupe méthyle; n est égal à 0; et Z représente -N = C($SR_7$)-NH et $R_7$ représente un groupe alkyle en C 1-C 2.

**16.** Produit pesticide selon revendication 13, contenant au moins un composant actif consistant en un composé de formule I dans laquelle $R_1$ représente un groupe alkyle en C 3-C 5 ou cyclopentyle; $R_2$ et $R_3$ représentent chacun un groupe alkyle en C 3-C 5; $R_4$ et $R_5$ représentent chacun l'hydrogène ou un groupe méthyle; n est égal à 0; et Z représente -N = C = N-.

**17.** Utilisation d'un composé de formule I

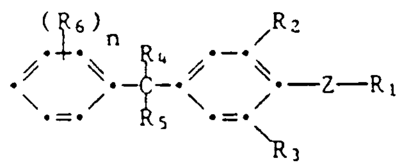

(I),

dans laquelle

- $R_1$ représente un groupe alkyle en C 1-C 8, un groupe alkyle en C 1-C 8 portant un ou plusieurs substituants halogéno et/ou alcoxy en C 1-C 6, un groupe cycloalkyle en C 3-C 8, un groupe cycloalkyle en C 3-C 8 portant un ou plusieurs substituants alkyle en C 1-C 3, ou un groupe (cycloalkyle en C 3-C 8)-alkyle en C 1-C 4;
- $R_2$ et $R_3$ représentent chacun un groupe alkyle en C 1-C 6, cycloalkyle en C 5-C 6 ou cycloalcényle en C 5-C 6;
- $R_4$ et $R_5$ représentent chacun l'hydrogène ou un groupe alkyle en C 1-C 4;
  chacun des symboles $R_6$ représente un halogène, un groupe alkyle en C 1-C 4, alcoxy en C 1-C 4, halogénoalkyle en C 1-C 4, halogénoalcoxy en C 1-C 4 ou un pont

$$-(-CH=CH-)_2, \quad -(-CH_2-)_3 \text{ ou } -(-CH_2-)_4$$

  en position 2,3 ou 3,4;
- n est égal à 0, 1 ou 2;
- Z représente -NH-CS-NH-, -N = C($SR_7$)-NH- ou -N = C = N-, et
- $R_7$ représente un groupe alkyle en C 1-C 10 ou alcényle en C 3-C 5,

ou de l'un de ses sels d'acides organiques ou minéraux pour la lutte contre les parasites des animaux et des végétaux.

**18.** Utilisation selon la revendication 17 d'un composé de formule I pour la lutte contre les insectes et les arachnides.

**19.** Procédé pour combattre les parasites des animaux et des végétaux, caractérisé en ce que l'on met les parasites, dans leurs divers stades de développement, au contact avec un composé de formule I

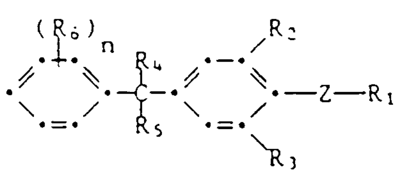

(I),

dans laquelle

- $R_1$ représente un groupe alkyle en C 1-C 8, un groupe alkyle en C 1-C 8 portant un ou plusieurs substituants halogéno et/ou alcoxy en C 1-C 6, un groupe cycloalkyle en C 3-C 8, un groupe cycloalkyle en C 3-C 8 portant un ou plusieurs substituants alkyle en C 1-C 3, ou un groupe (cycloalkyle en C 3-C 8)-alkyle en C 1-C 4;
- $R_2$ et $R_3$ représentent chacun un groupe alkyle en C 1-C 6, cycloalkyle en C 5-C 6 ou cycloalcényle en C 5-C 6;
- $R_4$ et $R_5$ représentent chacun l'hydrogène ou un groupe alkyle en C 1-C 4;

chacun des symboles $R_6$ représente un halogène, un groupe alkyle en C 1-C 4, alcoxy en C 1-C 4, halogénoalkyle en C 1-C 4, halogénoalcoxy en C 1-C 4 ou un pont

$$-(-CH=CH-)_2-, \quad -(-CH_2-)_3- \text{ ou } -(-CH_2-)_4-$$

en position 2,3 ou 3,4;

n est égal à 0, 1 ou 2;

Z représente -NH-CS-NH-, -N = C($SR_7$)-NH- ou -N = C = N-, et

$R_7$ représente un groupe alkyle en C 1-C 10 ou alcényle en C 3-C 5,

ou avec l'un de ses sels d'acides organiques ou minéraux.

**20.** Composés de formule II'

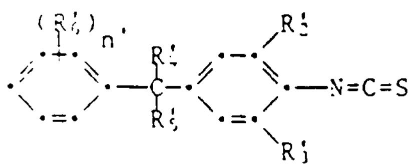

(II'),

dans laquelle $R'_2$ et $R'_3$ représentent chacun un groupe alkyle en C 3-C 5 ou cycloalkyle en C 5-C 6; $R'_4$ et $R'_5$ représentent chacun l'hydrogène ou un groupe méthyle; $R'_6$ représente le fluor, le chlore ou un groupe méthyle; et n' est égal à 0 ou 1.

**21.** Composés de formule II' de la revendication 20, dans laquelle $R'_2$ et $R'_3$ représentent chacun un groupe alkyle en C 3-C 5; $R'_4$ et $R'_5$ représentent chacun l'hydrogène ou un groupe méthyle; et n' est égal à 0.

**22.** Les composés selon revendication 20 ou 21, de formules

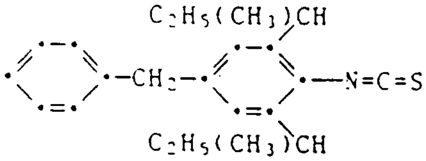 ,

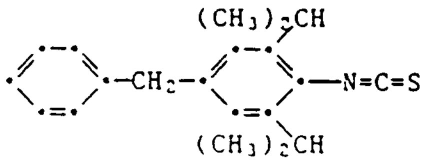 ,

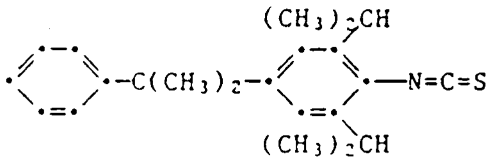 ,

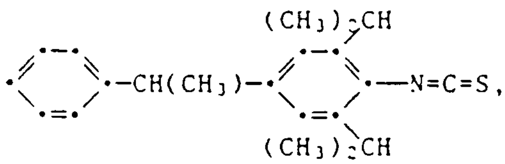 ,

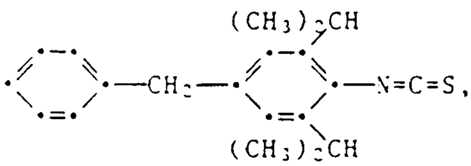 ,

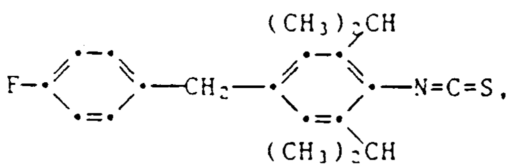 ,

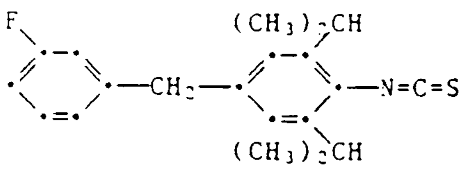 et

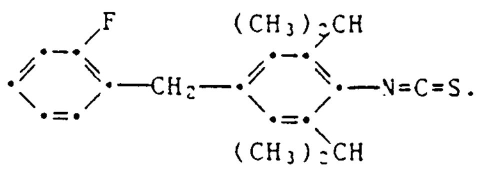 .

**23.** Composés de formule V'

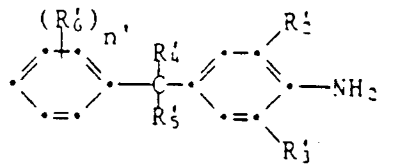 (V'),

dans laquelle R'$_2$ et R'$_3$ représentent chacun un groupe alkyle en C 3-C 5 ou cycloalkyle en C 5-C 6; R'$_4$ et R'$_5$ représentent chacun l'hydrogène ou un groupe méthyle; R'$_6$ représente le fluor, le chlore ou un groupe méthyle; et n' est égal à 0 ou 1.

**24.** Composés de formule V' de la revendication 23, dans laquelle R'$_2$ et R'$_3$ représentent chacun un groupe alkyle en C 3-C 5; R'$_4$ et R'$_5$ représentent chacun l'hydrogène ou un groupe méthyle; et n' est égal à 0 ou 1.

**25.** Les composés selon revendication 23 ou 24 de formules

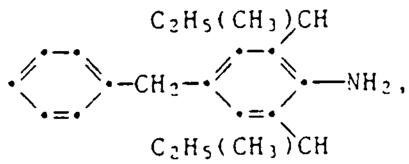

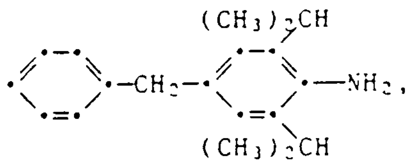

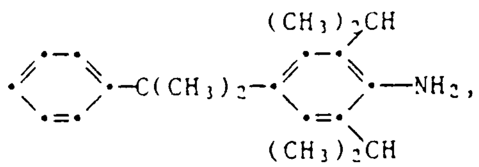

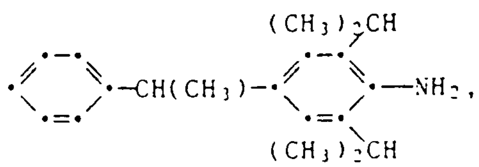

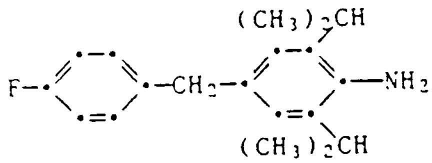

,

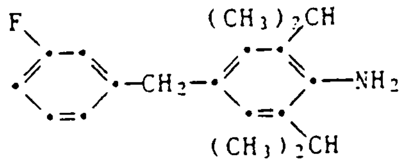

et

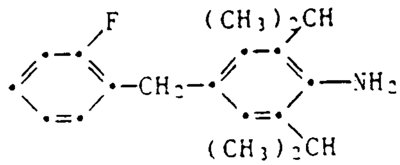

.